# EUROPEAN PATENT APPLICATION

(11) **EP 4 265 610 A1**
(43) Date of publication of application: **25.10.2023**
(21) Application number: 21908774.9
(22) Date of filing: 18.10.2021
(51) Int. Cl.: C07D 403/14, C07D 487/04, C07D 413/14, C07D 498/04, A61K 31/519, A61K 31/522, A61P 35/00

(54) **MACROCYCLIC K-RAS G12C INHIBITOR, PREPARATION METHOD THEREFOR AND USE THEREOF**

(30) Priority: 21.12.2020 CN 202011518497
(71) Applicant: Abbisko Therapeutics Co., Ltd., Shanghai 201203 (CN)
(72) Inventor: XUN, Guoliang, Shanghai 201203 (CN); YU, Hongping, Shanghai 201203 (CN); CHEN, Zhui, Shanghai 201203 (CN); XU, Yaochang, Shanghai 201203 (CN)
(74) Representative: dompatent von Kreisler Selting Werner - Partnerschaft von Patent- und Rechtsanwälten mbB
(86) International application number: PCT/CN2021/124469
(87) International publication number: WO 2022/134773

(57) **Abstract**

A macrocyclic K-RAS G12C inhibitor, a preparation method therefor and use thereof. The inhibitor can be widely used in preparation of a drug for treating cancer or tumor which is at least partially mediated by a K-RAS G12C mutation, particularly a drug for treating lung, liver, gastrointestinal tract, blood system, skin, bone, genitourinary tract, nervous system, gynecological, and adrenal related malignant tumor or cancer.

## Description

### TECHNICAL FIELD

The present invention belongs to the field of medicament synthesis, and in particular, relates to a macrocyclic K-RAS G12C inhibitor, a preparation method therefor and use thereof.

### BACKGROUND

The RAS gene family includes HRAS, KRAS and NRAS, which often mutate as oncogenes in cancers. 20-30% of human tumors have a mutated RAS protein. The activation of RAS protein gives rise to malignant phenotypes of cancer cells, including dysfunction of cell growth and programmed cell death and increase of aggressiveness and neoangiogenesis. Due to its high affinity to GTP/GDP and the lack of clear binding pockets, the development of medicaments targeting the RAS protein has been slower to take off.

Under normal conditions, the RAS protein acts as a molecular switch, alternating between a GDP-bound inactive state and a GTP-bound activated state. After the stimulation by exogenous growth factors and the promotion by guanine nucleotide exchanger factors (GEFs), the RAS protein changes from an inactive GDP-bound form to an activated GTP-bound form, and can thereby bind to and activate downstream signaling pathways. Subsequently, with the aid of its intrinsic GTPase activity and GTPase activator/accelerator proteins (GAPs), the RAS restores to its inactive GDP-bound form.

The missense mutations of codon 12, 13, or 61 lead to abnormal activation of the RAS. These mutations prolong the time that the RAS protein stays in the GTP-bound state, resulting in persistent activation of the downstream signaling pathways. K-RAS is the most common subtype of mutant in the RAS family of human cancers, including pancreatic cancer (71%), small intestine cancer (35%), colon cancer (35%), biliary tract cancer (26%), endometrial cancer (17%), and lung cancer (19%). In terms of mutation site, G12D/G12V/G12C/G13D is the most common type of mutation for K-RAS in pancreatic, lung, and colorectal cancers.

Since this protein lacks obvious pockets, the development of inhibitors against K-RAS is challenging. Recent studies have found a pocket that is previously undetected in the GDP-bound state of K-RAS. Based on these new findings, covalent binding inhibitors targeting the mutant cysteine of the codon 12 have become a research and development hotspot for K-RAS inhibitors. In the past, the mutated K-RAS was considered locked in the GTP-bound activated state. However, it was later discovered that G12C mutation still showed relatively high levels of GTP hydrolytic activity, and were therefore more susceptible to the covalent inhibitors against the GDP-bound K-RAS. Recently, several covalent inhibitors targeting K-RAS G12C mutation have successively entered early clinical trials.

### SUMMARY

After extensive and in-depth studies, the inventor of the present application has developed for the first time a macrocyclic K-RAS G12C inhibitor, a preparation method therefor and use thereof. A series of compounds of the present invention shows a strong inhibitory effect on the enzymatic and cellular activity of K-RAS, and can be widely used in the preparation of medicaments for treating cancer or tumor at least partially mediated by K-RAS G12C mutation, in particular medicaments for treating malignant tumor or cancer associated with the lung, liver and gallbladder, gastrointestinal tract, blood system, skin, bone, genitourinary tract, nervous system, gynecological and adrenal gland. They are expected to be developed into a new generation of K-RAS G12C inhibitor medicaments.

The first aspect of the present invention provides a compound of formula (I), a stereoisomer or pharmaceutically acceptable salt thereof: 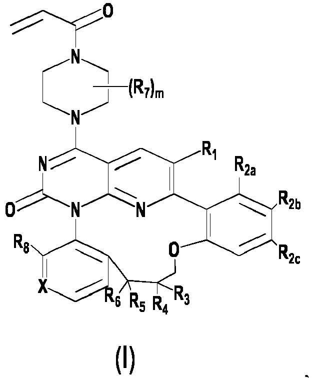 wherein, X is C(R₉) or N;
R₁ is selected from the group consisting of hydrogen, deuterium, halogen, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl, acetamido and -SF₅;
R₂ₐ is selected from the group consisting of hydrogen, deuterium, halogen, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl, acetamido and -SF₅;
R_{2b} is selected from the group consisting of hydrogen, deuterium, halogen, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl, acetamido and -SF₅;
R_{2c} is selected from the group consisting of hydrogen, deuterium, halogen, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl, acetamido and -SF₅;
R₃ and R₄ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, -SF₅, -S(O)ᵣR₁₀, -O-R₁₁, -C(O)OR₁₁, -C(O)R₁₂, - O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, or, R₃ and R₄, together with the carbon atom directly attached thereto, form C(O), 3-12 membered cycloalkyl or 3-12 membered heterocyclyl, above groups are optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, =O, -SF₅, -S(O)ᵣR₁₀, -O-R₁₁, - C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₅ and R₆ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, -SF₅, -S(O)ᵣR₁₀, -O-R₁₁, -C(O)OR₁₁, -C(O)R₁₂, - O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, or, R₅ and R₆, together with the carbon atom directly attached thereto, form C(O), 3-12 membered cycloalkyl or 3-12 membered heterocyclyl, above groups are optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, =O, -SF₅, -S(O)ᵣR₁₀, -O-R₁₁, - C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
provided that R₃, R₄, R₅ and R₆ are not all hydrogen at a time;
or, R₄ and R₅, together with the moiety directly attached thereto, form 3-12 membered cycloalkyl or 3-12 membered heterocyclyl, the 3-12 membered cycloalkyl or 3-12 membered heterocyclyl is optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, =O, -C₀₋₈ alkyl-SFs, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂, R₃ and R₆ are defined as above;
m is 0, 1, 2, 3 or 4;
each R₇ is independently selected from the group consisting of hydrogen, deuterium, halogen, cyano, hydroxy, C₁₋₄ alkoxy, C₁₋₄ alkyl, C₃₋₆ cycloalkyl, 4-6 membered heterocyclyl, -SF₅, C₂₋₄ alkynyl, C₁₋₄ cyanoalkyl, C₁₋₄ hydroxyalkyl, C₁₋₄ haloalkyl and C₁₋₄ deuterioalkyl, or, when m≥2, two of R₇, together with the moiety directly attached thereto, form C₃₋₁₂ cycloalkyl or 3-12 membered heterocyclyl;
R₈ and R₉ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, -C₀₋₈ alkyl-SFs, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂, above groups are optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, =O, -C₀₋₈ alkyl-SFs, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂;
each R₁₀ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₅₋₁₀ aryl, 5-10 membered heteroaryl and -NR₁₃R₁₄, above groups are optionally further substituted by one or more substituents selected from the group consisting of deuterium, halogen, hydroxy, oxo, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy and -NR₁₃R₁₄;
each R₁₁ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₅₋₁₀ aryl and 5-10 membered heteroaryl, above groups are optionally further substituted by one or more substituents selected from the group consisting of deuterium, halogen, hydroxy, oxo, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy and -NR₁₃R₁₄;
each R₁₂ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy and -NR₁₃R₁₄, above groups are optionally further substituted by one or more substituents selected from the group consisting of deuterium, halogen, hydroxy, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy and -NR₁₃R₁₄;
each R₁₃ and each R₁₄ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, sulfonyl, methanesulfonyl, isopropylsulfonyl, cyclopropylsulfonyl, p-toluenesulfonyl, amino, mono-C₁₋₈ alkyl amino, di-C₁₋₈ alkyl amino and C₁₋₁₀ alkanoyl, above groups are optionally further substituted by one or more substituents selected from the group consisting of deuterium, halogen, hydroxy, C₁₋₈ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy, amino, mono-C₁₋₈ alkyl amino, di-C₁₋₈ alkyl amino and C₁₋₁₀ alkanoyl;
or, R₁₃ and R₁₄, together with the nitrogen atom directly attached thereto, form 4-12 membered heterocyclyl, the 4-12 membered heterocyclyl is optionally further substituted by one or more substituents selected from the group consisting of deuterium, halogen, hydroxy, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy, amino, mono-C₁₋₈ alkyl amino, di-C₁₋₈ alkyl amino and C₁₋₁₀ alkanoyl;
each r is independently 0, 1 or 2.

As a preferred embodiment, in the compound of formula (I), the stereoisomer or pharmaceutically acceptable salt thereof, R₃ and R₄ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -SF₅, -S(O)ᵣR₁₀, -O-R₁₁, -C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, or, R₃ and R₄, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, -SF₅, -S(O)ᵣR₁₀, -O-R₁₁, -C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₅ and R₆ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -SF₅, -S(O)ᵣR₁₀, -O-R₁₁, -C(O)OR₁₁, -C(O)R₁₂, -O-C(O)R₁₂, - NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, or, R₅ and R₆, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, -SF₅, -S(O)ᵣR₁₀, -O-R₁₁, -C(O)OR₁₁, -C(O)R₁₂, -O-C(O)R₁₂, -NR₁₃R₁₄, - C(=NR₁₃)R₁₂, -N(R₁₃)-C(=NR₁₄)R₁₂, -C(O)NR₁₃R₁₄ and -N(R₁₃)-C(O)R₁₂;
provided that R₃, R₄, R₅ and R₆ are not all hydrogen at a time;
or, R₄ and R₅, together with the moiety directly attached thereto, form 3-6 membered cycloalkyl or 3-6 membered heterocyclyl, the 3-6 membered cycloalkyl or 3-6 membered heterocyclyl is optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, - C₀₋₄ alkyl-SFs, -C₀₋₄ alkyl-S(O)ᵣR₁₀, -C₀₋₄ alkyl-O-R₁₁, -C₀₋₄ alkyl-C(O)OR₁₁, -C₀₋₄ alkyl-C(O)R₁₂, - C₀₋₄ alkyl-O-C(O)R₁₂, -C₀₋₄ alkyl-NR₁₃R₁₄, -C₀₋₄ alkyl-C(=NR₁₃)R₁₂, -C₀₋₄ alkyl-N(R₁₃)-C(=NR₁₄)R₁₂, -C₀₋₄ alkyl-C(O)NR₁₃R₁₄ and -C₀₋₄ alkyl-N(R₁₃)-C(O)R₁₂, R₃ and R₆ are defined as above;
wherein, R₁₀, R₁₁, R₁₂, R₁₃, R₁₄ and r are defined as those in the compound of formula (I).

As a preferred embodiment, in the compound of formula (I), the stereoisomer or pharmaceutically acceptable salt thereof, R₁ is selected from the group consisting of hydrogen, deuterium, fluorine, chlorine, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl and -SF₅;
R₂ₐ is selected from the group consisting of hydrogen, deuterium, fluorine, chlorine, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl and -SF₅;
R_{2b} is selected from the group consisting of hydrogen, deuterium, fluorine, chlorine, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl and -SF₅;
R_{2c} is selected from the group consisting of hydrogen, deuterium, fluorine, chlorine, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl and -SFs.

As a preferred embodiment, in the compound of formula (I), the stereoisomer or pharmaceutically acceptable salt thereof, each R₇ is independently selected from the group consisting of hydrogen, deuterium, halogen, cyano, hydroxy, C₁₋₄ alkoxy, C₁₋₄ alkyl, C₂₋₄ alkynyl, C₃₋₆ cycloalkyl, 4-6 membered heterocyclyl, -SFs and C₁₋₄ cyanoalkyl, or, when m≥2, two of R₇, together with the moiety directly attached thereto, form C₃₋₆ cycloalkyl or 3-6 membered heterocyclyl.

As a further preferred embodiment, in the compound of formula (I), the stereoisomer or pharmaceutically acceptable salt thereof, each R₇ is independently selected from the group consisting of hydrogen, deuterium, fluorine, chlorine, cyano, hydroxy, methyl, ethyl, isopropoxy, cyclopropyl, cyclobutyl, cyclobutoxy, cyanomethyl and -SFs.

As a preferred embodiment, in the compound of formula (I), the stereoisomer or pharmaceutically acceptable salt thereof, R₈ and R₉ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -C₀₋₄ alkyl-SFs, - C₀₋₄ alkyl-S(O)ᵣR₁₀, -C₀₋₄ alkyl-O-R₁₁, -C₀₋₄ alkyl-C(O)OR₁₁, -C₀₋₄ alkyl-C(O)R₁₂, -C₀₋₄ alkyl-O-C(O)R₁₂, -C₀₋₄ alkyl-NR₁₃R₁₄, -C₀₋₄ alkyl-C(=NR₁₃)R₁₂, -C₀₋₄ alkyl-N(R₁₃)-C(=NR₁₄)R₁₂, -C₀₋₄ alkyl-C(O)NR₁₃R₁₄ and -C₀₋₄ alkyl-N(R₁₃)-C(O)R₁₂, above groups are optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, -C₀₋₄ alkyl-SFs, -C₀₋₄ alkyl-S(O)ᵣR₁₀, -C₀₋₄ alkyl-O-R₁₁, -C₀₋₄ alkyl-C(O)OR₁₁, -C₀₋₄ alkyl-C(O)R₁₂, -C₀₋₄ alkyl-O-C(O)R₁₂, -C₀₋₄ alkyl-NR₁₃R₁₄, -C₀₋₄ alkyl-C(=NR₁₃)R₁₂, -C₀₋₄ alkyl-N(R₁₃)-C(=NR₁₄)R₁₂, -C₀₋₄ alkyl-C(O)NR₁₃R₁₄ and - C₀₋₄ alkyl-N(R₁₃)-C(O)R₁₂; wherein, R₁₀, R₁₁, R₁₂, R₁₃, R₁₄ and r are defined as those in the compound of formula (I).

As a further preferred embodiment, in the compound of formula (I), the stereoisomer or pharmaceutically acceptable salt thereof, R₈ and R₉ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -SF₅, -S(O)ᵣR₁₀, -O-R₁₁, -C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, -SF₅, -S(O)ᵣR₁₀, -O-R₁₁, -C(O)OR₁₁, -C(O)R₁₂, -O-C(O)R₁₂, -NR₁₃R₁₄, - C(=NR₁₃)R₁₂, -N(R₁₃)-C(=NR₁₄)R₁₂, -C(O)NR₁₃R₁₄ and -N(R₁₃)-C(O)R₁₂;
wherein, R₁₀, R₁₁, R₁₂, R₁₃, R₁₄ and r are defined as those in the compound of formula (I).

As a preferred embodiment, in the compound of formula (I), the stereoisomer or pharmaceutically acceptable salt thereof, the compound of formula (I) is a compound having formula (II): 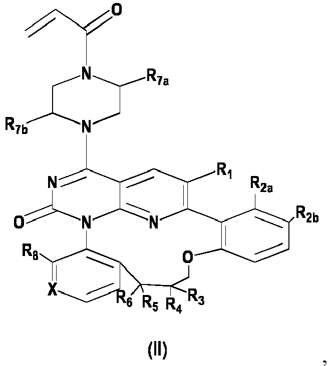
wherein, X is CH or N;
R₁ is selected from the group consisting of hydrogen, deuterium, fluorine, chlorine, cyano, hydroxy, methyl, ethyl, isopropyl, trifluoromethyl, difluoromethyl, trideuteriomethyl, dideuteriomethyl, methoxy, ethoxy, isopropoxy, trifluoromethoxy, difluoromethoxy, trideuteriomethoxy, dideuteriomethoxy, trifluoroisopropoxy, trideuterisoisopropoxy, cyclopropyl, cyclobutyl and cyclobutoxy;
R₂ₐ is selected from the group consisting of hydrogen, deuterium, fluorine, chlorine, cyano, hydroxy, methyl, ethyl, isopropyl, trifluoromethyl, difluoromethyl, trideuteriomethyl, dideuteriomethyl, methoxy, ethoxy, isopropoxy, trifluoromethoxy, difluoromethoxy, trideuteriomethoxy, dideuteriomethoxy, trifluoroisopropoxy, trideuterioisopropoxy, cyclopropyl, cyclobutyl and cyclobutoxy;
R_{2b} is selected from the group consisting of hydrogen, deuterium, fluorine, chlorine, cyano, hydroxy, methyl, ethyl, isopropyl, trifluoromethyl, difluoromethyl, trideuteriomethyl, dideuteriomethyl, methoxy, ethoxy, isopropoxy, trifluoromethoxy, difluoromethoxy, trideuteriomethoxy, dideuteriomethoxy, trifluoroisopropoxy, trideuterioisopropoxy, cyclopropyl, cyclobutyl and cyclobutoxy;
R₃ and R₄ are each independently selected from hydrogen, deuterium, -O-R₁₁ and -O-C(O)R₁₂, or, R₃ and R₄, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, - S(O)ᵣR₁₀, -O-R₁₁, -C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₅ and R₆ are each independently selected from hydrogen, deuterium, -O-R₁₁ and -O-C(O)R₁₂, or, R₅ and R₆, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, - S(O)ᵣR₁₀, -O-R₁₁, -C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
provided that R₃, R₄, R₅ and R₆ are not all hydrogen at a time;
or, R₄ and R₅, together with the moiety directly attached thereto, form 3-6 membered cycloalkyl or 3-6 membered heterocyclyl, the 3-6 membered cycloalkyl or 3-6 membered heterocyclyl is optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, -S(O)ᵣR₁₀, -O-R₁₁, -C(O)OR₁₁, -C(O)R₁₂, -O-C(O)R₁₂, -C₀₋₂ alkyl-NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, R₃ and R₆ are defined as above;
R₇ₐ is selected from the group consisting of hydrogen, deuterium, fluorine, chlorine, cyano, hydroxy, methyl, ethyl, isopropoxy, cyclopropyl, cyclobutyl and cyclobutoxy;
R_{7b} is selected from the group consisting of hydrogen, deuterium, fluorine, chlorine, cyano, hydroxy, methyl, ethyl, isopropoxy, cyclopropyl, cyclobutyl and cyclobutoxy;
R₈ is selected from the group consisting of hydrogen, deuterium, methyl, ethyl, propyl, isopropyl, trifluoromethyl, difluoromethyl, trideuteriomethyl, dideuteriomethyl, cyclopropyl, cyclobutyl, azacyclobutyl, oxacyclobutyl, methoxy, ethoxy and isopropoxy;
wherein, R₁₀, R₁₁, R₁₂, R₁₃, R₁₄ and r are defined as those in the compound of formula (I).

As a further preferred embodiment, in the compound of formula (I), the stereoisomer or pharmaceutically acceptable salt thereof, the compound of formula (I) is a compound having formula (III): 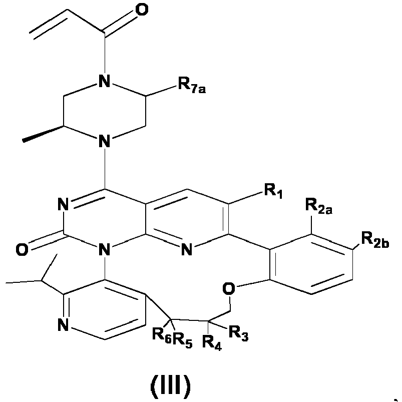
wherein, R₁ is hydrogen, deuterium, fluorine or chlorine;
R₂ₐ is hydrogen, deuterium, fluorine or chlorine;
R_{2b} is selected from the group consisting of hydrogen, deuterium, fluorine, chlorine, cyano, hydroxy, methyl, trifluoromethyl, methoxy, trifluoromethoxy and cyclopropyl;
R₇ₐ is selected from the group consisting of hydrogen, deuterium, fluorine, chlorine, methyl, ethyl, isopropoxy, cyclopropyl, cyclobutyl and cyclobutoxy;
R₃, R₄, R₅ and R₆ are defined as those in the compound of formula (II).

As a more further preferred embodiment, in the compound of formula (I), the stereoisomer or pharmaceutically acceptable salt thereof, R₅ is -O-R₁₁ or -O-C(O)R₁₂, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, -S(O)ᵣR₁₀, -O-R₁₁, -C(O)OR₁₁, -C(O)R₁₂, -O-C(O)R₁₂, -NR₁₃R₁₄, -C(O)NR₁₃R₁₄ and -N(R₁₃)-C(O)R₁₂; R₆ is hydrogen or deuterium;
R₃ is hydrogen, deuterium, -O-R₁₁ or -O-C(O)R₁₂, R₄ is hydrogen or deuterium, or, R₃ and R₄, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, -S(O)ᵣR₁₀, -O-R₁₁, -C(O)OR₁₁, -C(O)R₁₂, -O-C(O)R₁₂, -NR₁₃R₁₄, -C(O)NR₁₃R₁₄ and -N(R₁₃)-C(O)R₁₂;
wherein, R₁₀, R₁₁, R₁₂, R₁₃, R₁₄ and r are defined as those in the compound of formula (II). As a yet more further preferred embodiment, in the compound of formula (I), the stereoisomer or pharmaceutically acceptable salt thereof, R₅ is selected from the group consisting of hydroxy, 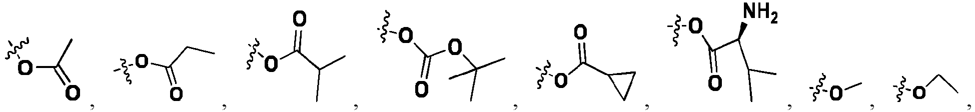 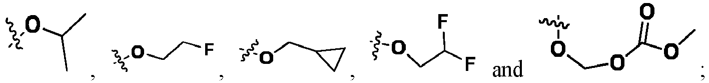 R₆ is hydrogen or deuterium;
R₃ is selected from the group consisting of hydrogen, deuterium, hydroxy, 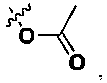 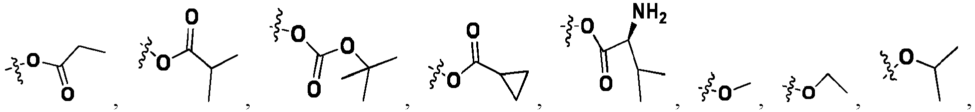 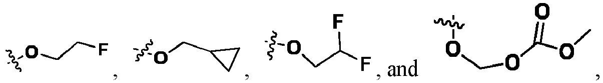 R₄ is hydrogen or deuterium, or, R₃ and R₄, together with the carbon atom directly attached thereto, form C(O), cyclopropyl, cyclobutyl, azacyclobutyl or oxacyclobutyl.

As a more further preferred embodiment, in the compound of formula (I), the stereoisomer or pharmaceutically acceptable salt thereof, R₅ and R₆, together with the carbon atom directly attached thereto, form C(O);
R₃ is hydrogen, deuterium, -O-R₁₁ or -O-C(O)R₁₂, R₄ is hydrogen or deuterium, or, R₃ and R₄, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, -S(O)ᵣR₁₀, -O-R₁₁, -C(O)OR₁₁, -C(O)R₁₂, -O-C(O)R₁₂, -NR₁₃R₁₄, -C(O)NR₁₃R₁₄ and -N(R₁₃)-C(O)R₁₂;
wherein, R₁₀, R₁₁, R₁₂, R₁₃, R₁₄ and r are defined as those in the compound of formula (II).

As a yet more further preferred embodiment, in the compound of formula (I), the stereoisomer or pharmaceutically acceptable salt thereof, R₅ and R₆, together with the carbon atom directly attached thereto, form C(O);
R₃ is selected from the group consisting of hydrogen, deuterium, hydroxy, 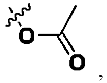 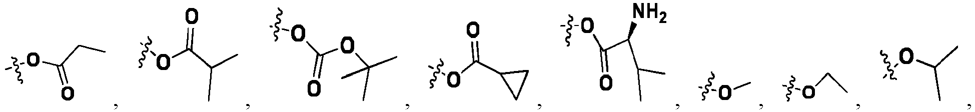 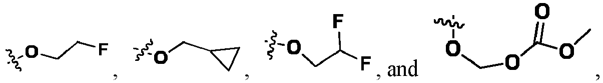 R₄ is hydrogen or deuterium, or, R₃ and R₄, together with the carbon atom directly attached thereto, form C(O), cyclopropyl, cyclobutyl, azacyclobutyl or oxacyclobutyl.

As a more further preferred embodiment, in the compound of formula (I), the stereoisomer or pharmaceutically acceptable salt thereof, R₄ and R₅, together with the moiety directly attached thereto, form 3-6 membered cycloalkyl or 3-6 membered heterocyclyl, the 3-6 membered cycloalkyl or 3-6 membered heterocyclyl is optionally further substituted by one or more substituents selected from the group consisting of deuterium, fluorine, chlorine, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, -S(O)ᵣR₁₀, -O-R₁₁, -C(O)OR₁₁, -C(O)R₁₂, -O-C(O)R₁₂, -CH₂-NR₁₃R₁₄, -C(O)NR₁₃R₁₄ and -N(R₁₃)-C(O)R₁₂; R₃ is s hydrogen or deuterium; R₆ is hydrogen or deuterium;
wherein, R₁₀, R₁₁, R₁₂, R₁₃, R₁₄ and r are defined as those in the compound of formula (II).

As a yet more further preferred embodiment, in the compound of formula (I), the stereoisomer or pharmaceutically acceptable salt thereof, R₄ and R₅, together with the moiety directly attached thereto, form 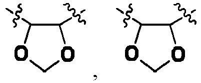 is optionally further substituted by one or more substituents selected from the group consisting of deuterium, fluorine, chlorine, cyano, methyl, ethyl, propyl, isopropyl, trifluoromethyl, difluoromethyl, trideuteriomethyl, dideuteriomethyl, cyclopropyl, cyclobutyl, azacyclobutyl, oxacyclobutyl, =O, methoxy, ethoxy, isopropoxy and 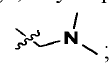 R₃ is hydrogen or deuterium; R₆ is hydrogen or deuterium.

As a preferred embodiment, in the compound of formula (I), the stereoisomer or pharmaceutically acceptable salt thereof, each R₁₀ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₅₋₈ aryl, 5-8 membered heteroaryl and -NR₁₃R₁₄, above groups are optionally further substituted by one or more substituents selected from the group consisting of deuterium, halogen, hydroxy, oxo, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy and -NR₁₃R₁₄;
each R₁₁ is independently selected from the group consisting of hydrogen, deuterium, C₁₋₄ alkyl, C₂₋₄ alkenyl, C₃₋₆ cycloalkyl, 3-6 membered heterocyclyl, C₅₋₈ aryl and 5-8 membered heteroaryl, above groups are optionally further substituted by one or more substituents selected from the group consisting of deuterium, halogen, hydroxy, oxo, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy and -NR₁₃R₁₄;
each R₁₂ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy and -NR₁₃R₁₄, above groups are optionally further substituted by one or more substituents selected from the group consisting of deuterium, halogen, hydroxy, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy and -NR₁₃R₁₄;
each R₁₃ and each R₁₄ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, sulfonyl, methanesulfonyl, isopropylsulfonyl, cyclopropylsulfonyl, p-toluenesulfonyl, amino, mono-C₁₋₄ alkyl amino, di-C₁₋₄ alkyl amino and C₁₋₄ alkanoyl, above groups are optionally further substituted by one or more substituents selected from the group consisting of deuterium, halogen, hydroxy, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy, amino, mono-C₁₋₄ alkyl amino, di-C₁₋₄ alkyl amino and C₁₋₄ alkanoyl;
or, R₁₃ and R₁₄, together with the nitrogen atom directly attached thereto, form 4-6 membered heterocyclyl, the 4-6 membered heterocyclyl is optionally further substituted by one or more substituents selected from the group consisting of deuterium, halogen, hydroxy, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy, amino, mono-C₁₋₄ alkyl amino, di-C₁₋₄ alkyl amino and C₁₋₄ alkanoyl.

As the most preferred embodiment, the compound of formula (I), the stereoisomer or pharmaceutically acceptable salt thereof comprises, but is not limited to, the following compounds: 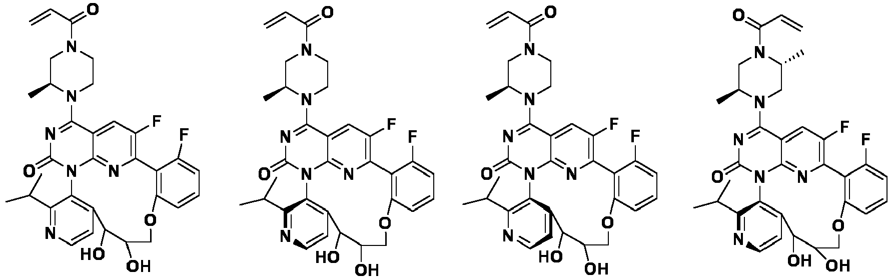 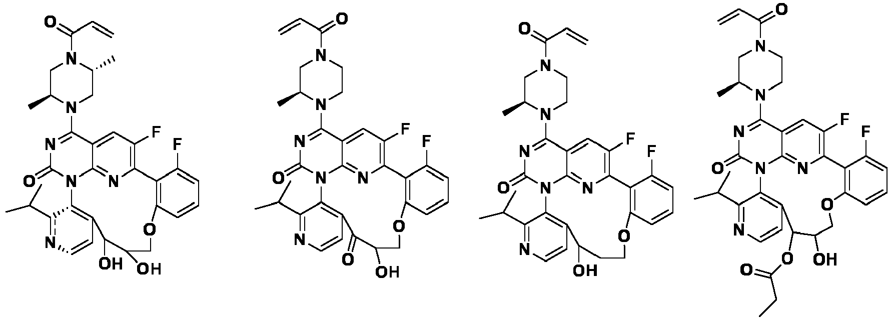 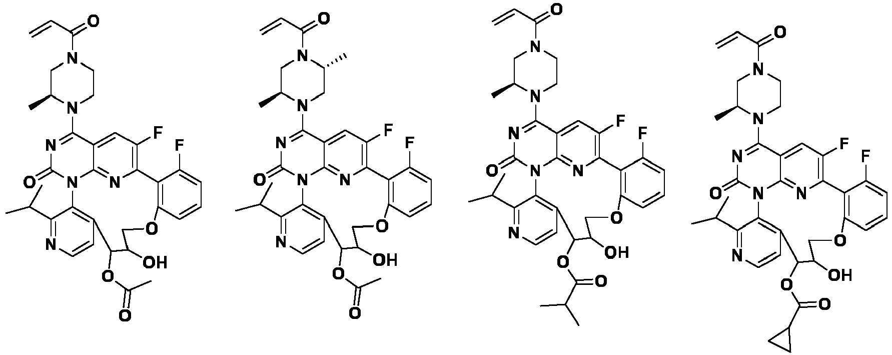 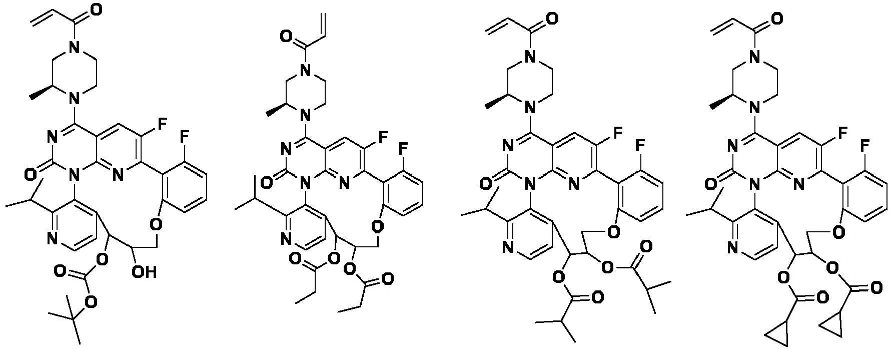 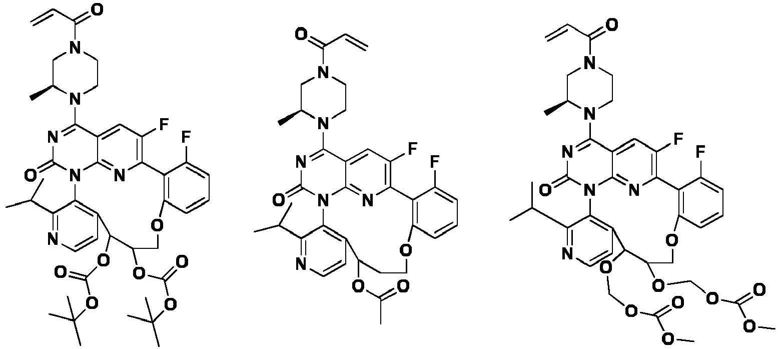 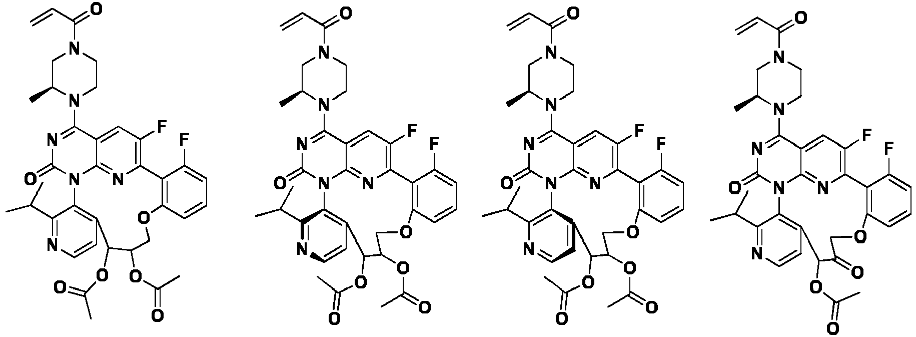 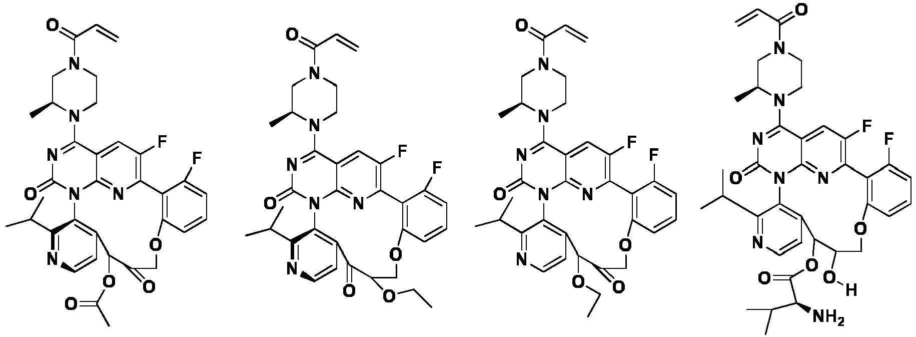 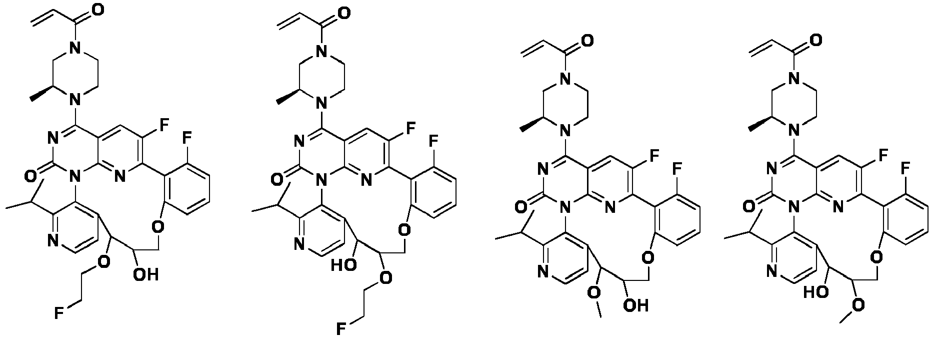 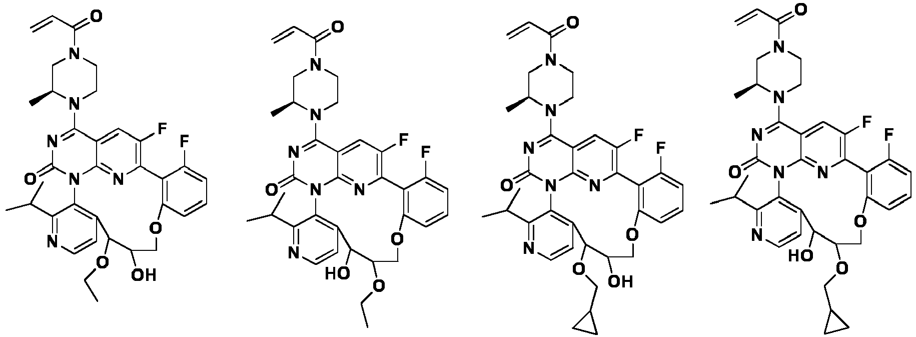 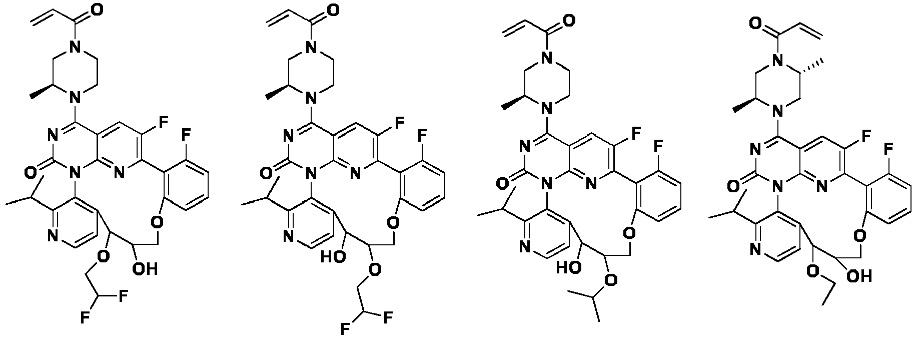 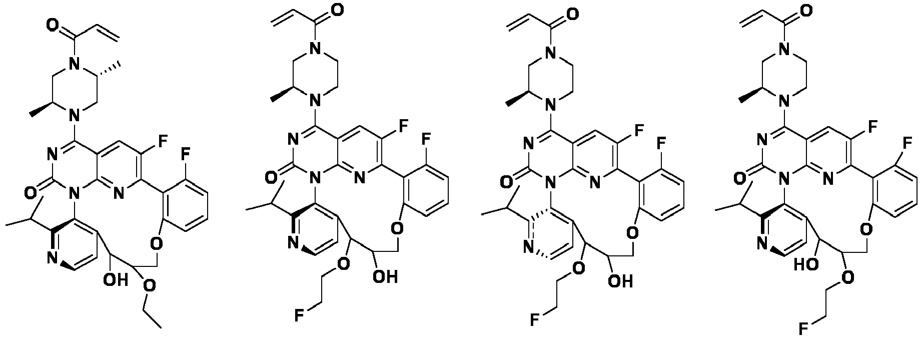 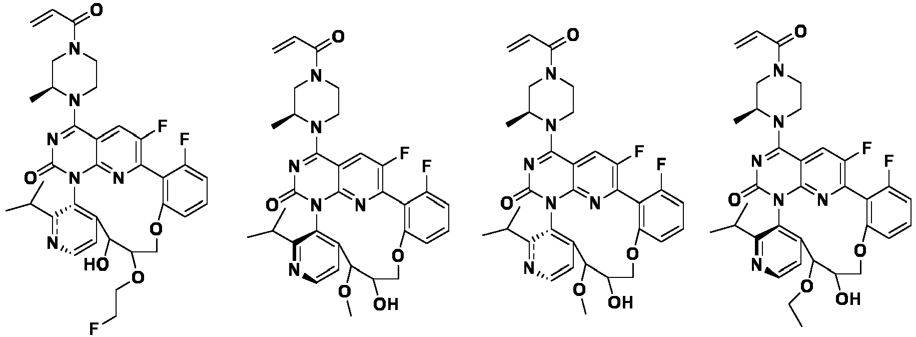 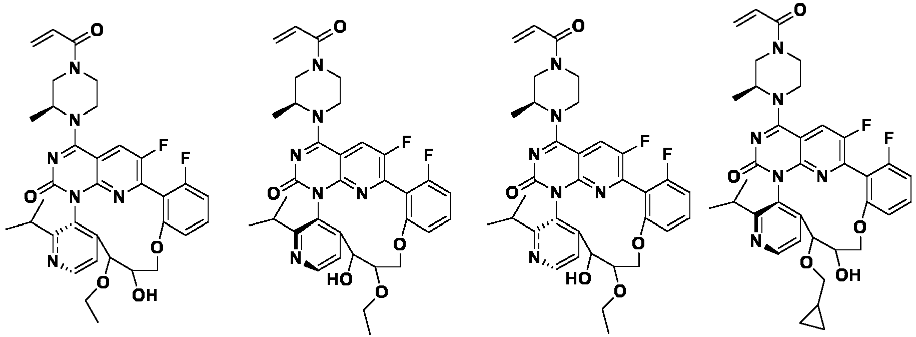 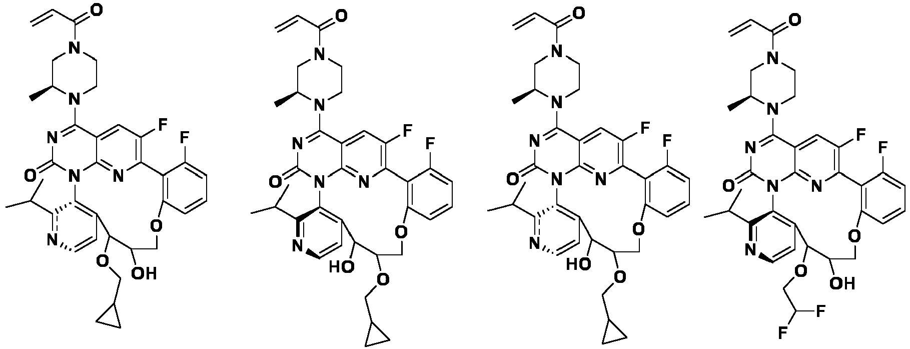 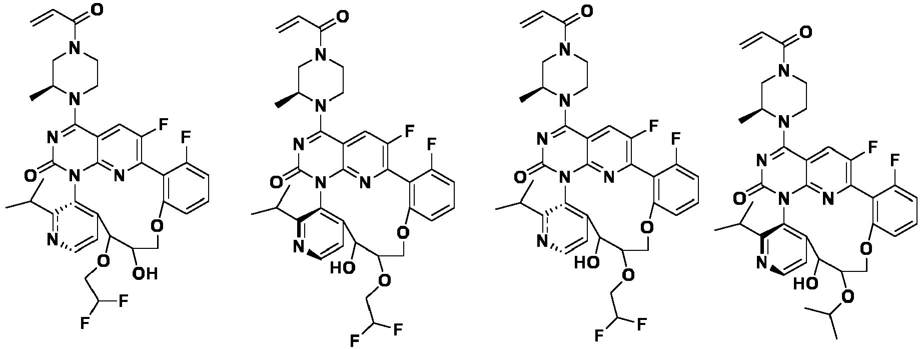 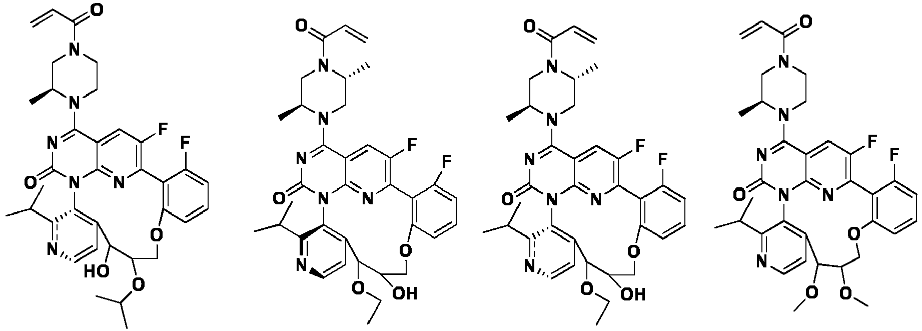 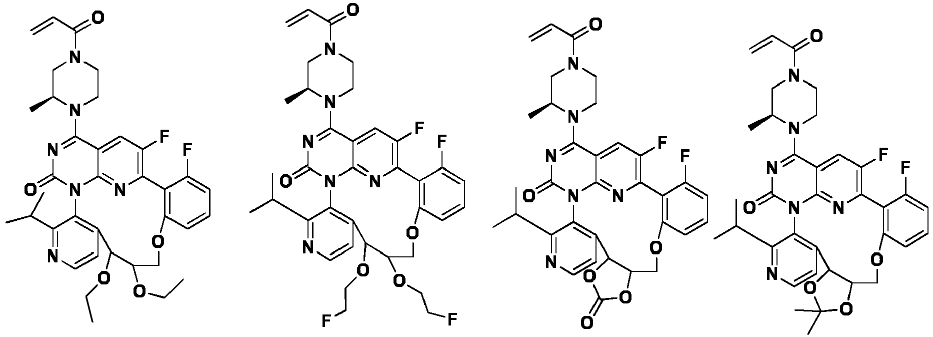 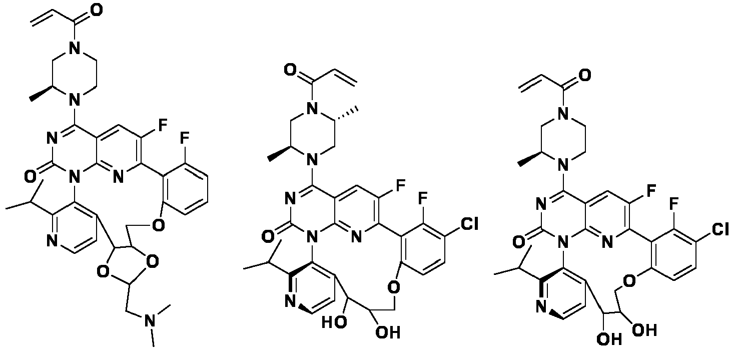 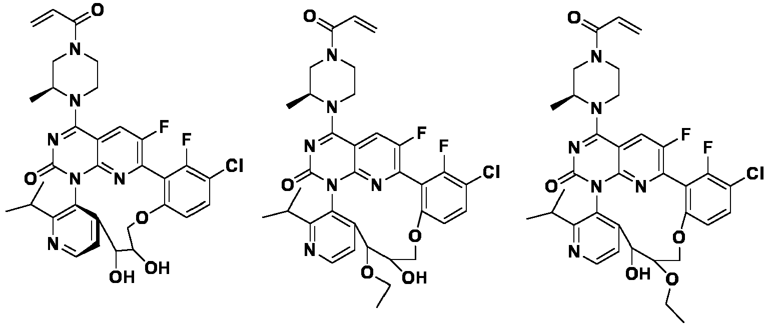 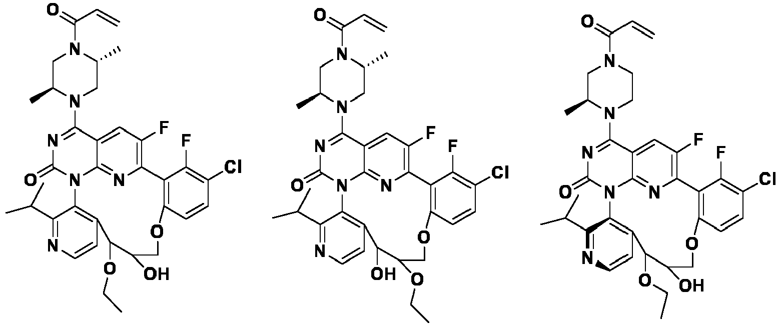 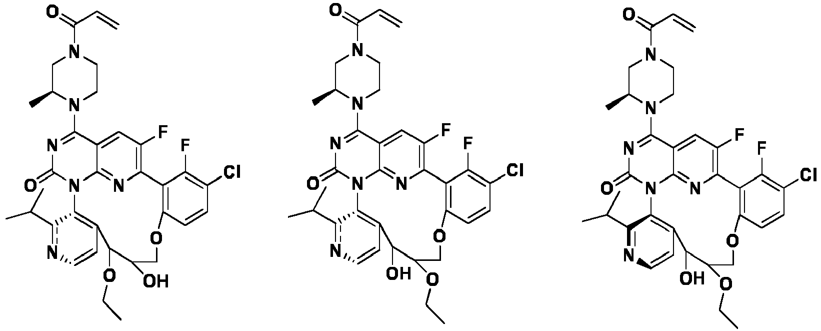 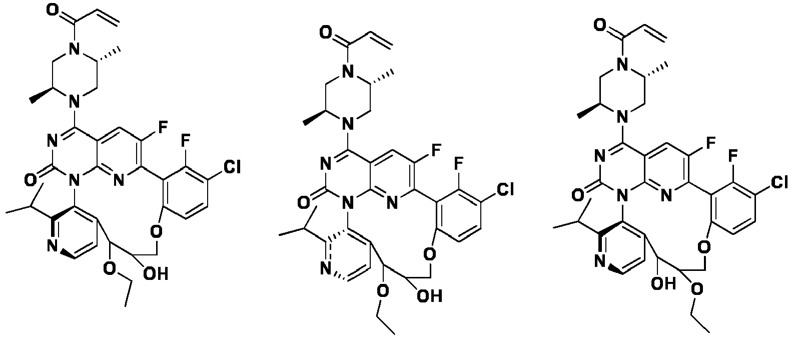 and

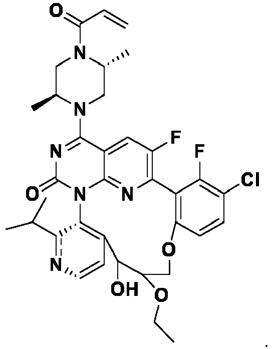

The second aspect of the present invention provides a method for preparing the compound of formula (I), the stereoisomer, or pharmaceutically acceptable salt thereof, comprising the following steps: 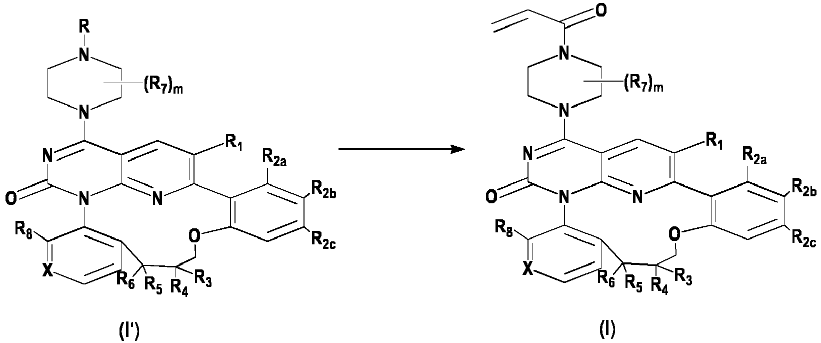 wherein, R is H or an amino-protecting group, preferably, the amino-protecting group is tert-butyloxycarbonyl; R₁, R₂ₐ, R_{2b}, R_{2c}, R₃, R₄, R₅, R₆, R₇, R₈, X and m are defined as those in the compound of formula (I).

The third aspect of the present invention provides a pharmaceutical composition, comprising the compound of formula (I), the stereoisomer or pharmaceutically acceptable salt thereof as previously defined, and a pharmaceutically acceptable carrier.

The fourth aspect of the present invention provides use of the compound of formula (I), the stereoisomer or pharmaceutically acceptable salt thereof or pharmaceutical composition as previously defined in the preparation of a medicament for treating tumor or cancer at least partially mediated by K-RAS G12C mutation.

As a preferred embodiment, the cancer or tumor is selected from the group consisting of a malignant tumor or cancer of the lung, a malignant tumor or cancer of the liver and gallbladder, a malignant tumor or cancer of the gastrointestinal tract, a malignant tumor or cancer of the blood system, a sarcoma, a malignant tumor or cancer of the skin, a malignant tumor or cancer of the bone, a malignant tumor or cancer of the genitourinary tract, a malignant tumor or cancer of the nervous system, a gynecological malignant tumor or cancer, and a malignant tumor or cancer of the adrenal gland.

As a further preferred embodiment, **the malignant tumor or cancer of the lung is selected from** the group consisting of bronchial carcinoma (squamous cell carcinoma, undifferentiated small cell, undifferentiated large cell or adenocarcinoma), non-small cell lung cancer, bronchial adenoma, sarcoma, lymphoma, cartilagenous hamartoma, and mesothelioma;
**the malignant tumor or cancer of the liver and gallbladder is selected from** the group consisting of liver cancer, hepatoblastoma, hemangiosarcoma, hepatocellular adenoma, hemangioma, gallbladder cancer, ampullary cancer and biliary duct cancer;
**the malignant tumor or cancer of the gastrointestinal tract is selected from** the group consisting of a malignant tumor or cancer of the esophagus (squamous cell carcinoma, adenocarcinoma, leiomyosarcoma or lymphoma), a malignant tumor or cancer of the stomach (cancer, lymphoma or leiomyosarcoma), a malignant tumor or cancer of the pancreas (ductal adenocarcinoma, insulinoma, glucagonoma, gastrinoma, carcinoid tumor, uveal tumor), a malignant tumor or cancer of the small intestine (adenocarcinoma, lymphoma, carcinoid tumor, Kaposi's sarcoma, leiomyoma, hemangioma, lipoma, neurofibroma, or fibroma), a malignant tumor or cancer of the large intestine (adenocarcinoma, adenoma, or tubular adenoma) and leiomyoma;
**the malignant tumor or cancer of the blood system is selected from** the group consisting of acute or chronic myeloid leukemia, acute lymphocytic leukemia, chronic lymphocytic leukemia, myeloproliferative disease, multiple myeloma, myelodysplastic syndrome, Hodgkin's disease and non-Hodgkin lymphoma;
**the sarcoma** is selected from the group consisting of angiosarcoma, fibrosarcoma, rhabdomyosarcoma, liposarcoma, myxoma, rhabdomyoma, fibroma, lipoma or teratoma;
**the malignant tumor or cancer of the skin is selected from** malignant melanoma, basal cell carcinoma, squamous cell carcinoma, Kaposi sarcoma, nevocarcinoma, hyperplastic nevus, lipoma, hemangioma, dermatofibroma, keloma, and psoriasis;
**the malignant tumor or cancer of the bone is selected from** the group consisting of osteosarcoma, fibrosarcoma, malignant fibrous histiotoma, chondrosarcoma, Ewing's sarcoma, malignant lymphoma, multiple myeloma, malignant giant cell tumor chordoma, osteochondroma, benign chondroma, chondroblastoma, cartilage mucosal fibroma, osteoid osteoma, and giant cell tumor;
**the malignant tumor or cancer of the genitourinary tract is selected from** the group consisting of a malignant tumor or cancer of the kidney (adenocarcinoma, Wilm's tumor, or nephroblastoma), lymphoma, leukemia, a malignant tumor or cancer of the bladder or urethra (squamous cell carcinoma, transitional cell carcinoma, or adenocarcinoma), a malignant tumor or cancer of the prostate gland (adenocarcinoma or sarcoma), a malignant tumor or cancer of the testicle (leukemia, teratoma, embryonal carcinoma, or teratoma), choriocarcinoma, sarcoma, mesenchymal cell cancer, fibroma, fibroadenoma, adenomatoid tumor and lipoma;
**the malignant tumor or cancer of the nervous system is selected from** the group consisting of osteoma, hemangioma, granuloma, xanthoma, scleromalacia, meningioma, meningosarcoma, glioma, astrocytoma, medulloblastoma, neuroglioma, ependymoma, genital tumor, glioblastoma multiforme, oligodendroglioma, schwannoma, retinoblastoma, congenital tumor, spinal neurofibroma, meningioma and sarcoma;
**the gynecological malignant tumor or cancer is selected from** the group consisting of endometrial cancer (serous cystadenocarcinoma, mucinous cystadenocarcinoma or unclassified cancer), granulosa-thecoma, leydig cell tumor, abnormal myometrial tumor, malignant teratoma, squamous epithelial carcinoma, Brenner's tumor, adenoepithelial carcinoma, melanoma, clear cell carcinoma, squamous cell carcinoma, botryoid sarcoma and fallopian tube carcinoma; and
**the malignant tumor or cancer of the adrenal gland is** neuroblastoma.

The fifth aspect of the present invention provides use of the compound of formula (I), the stereoisomer or pharmaceutically acceptable salt thereof as previously defined, or the pharmaceutical composition as previously defined, as a medicament for treating tumor or cancer at least partially mediated by K-RAS G12C mutation.

The sixth aspect of the present invention provides a method for preventing and/or treating tumor or cancer at least partially mediated by K-RAS G12C mutation, comprising: administrating the compound of formula (I), the stereoisomer or pharmaceutically acceptable salt thereof as previously defined, or the pharmaceutical composition as previously defined, to a patient.

### DETAILED DESCRIPTION OF THE INVENTION

After extensive and in-depth studies, the inventor of the present application has developed for the first time a macrocyclic K-RAS G12C inhibitor, a preparation method therefor and use thereof. A series of compounds of the present invention shows a strong inhibitory effect on the enzymatic and cellular activity of K-RAS, and can be widely used in the preparation of medicaments for treating cancer or tumor at least partially mediated by K-RAS G12C mutation, in particular medicaments for treating malignant tumor or cancer associated with the lung, liver and gallbladder, gastrointestinal tract, blood system, skin, bone, genitourinary tract, nervous system, gynecological and adrenal gland. They are expected to be developed into a new generation of K-RAS G12C inhibitor medicaments. On such basis, the present invention has been completed.

Detailed description: Unless otherwise stated to the contrary or specifically noted, the following terms used in the specification and claims have the following meanings.

"Alkyl" refers to linear or branched saturated aliphatic alkyl groups, preferably linear or branched alkyl groups containing 1 to 10 carbon atoms or 1 to 6 carbon atoms or 1 to 4 carbon atoms, which includes, but is not limited to methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, etc. "C₁₋₁₀ alkyl" refers to linear or branched alkyl containing 1 to 10 carbon atoms; "C₁₋₈ alkyl" refers to linear or branched alkyl containing 1 to 8 carbon atoms; "C₀₋₈ alkyl" refers to linear or branched alkyl containing 0 to 8 carbon atoms; "C₀₋₄ alkyl" refers to linear or branched alkyl containing 0 to 4 carbon atoms; "C₀₋₂ alkyl" refers to linear or branched alkyl containing 0 to 2 carbon atoms; "C₁₋₄ alkyl" refers to linear or branched alkyl containing 1 to 4 carbon atoms; and "C₀ alkyl" refers to that the number of carbon atom is 0.

Alkyl may be optionally substituted or unsubstituted, and when it is substituted, the substituent is preferably one or more (preferably, 1, 2, 3 or 4) of the groups below independently selected from the group consisting of deuterium, halogen, cyano, nitro, azido, C₁₋₁₀ alkyl, C₁₋₁₀ alkoxy, C₂₋₁₀ alkenyl, C₂₋₁₀ alkynyl, C₁₋₁₀ haloalkyl, C₁₋₁₀ haloalkoxy, C₁₋₁₀ deuterioalkyl, C₁₋₁₀ deuterioalkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Cycloalkyl" or "carbocycle" refers to a monocyclic or polycyclic cyclic hydrocarbon substituent that is saturated or partially unsaturated. The partially unsaturated cyclic hydrocarbon means a cyclic hydrocarbon that may contain one or more (preferably, 1, 2 or 3) double bonds, but none of rings has a fully conjugated π-electron system. The cycloalkyl includes monocyclic cycloalkyl and polycyclic cycloalkyl, preferably including a cycloalkyl containing 3 to 10 or 3 to 8 or 3 to 6 carbon atoms. For example, "C₃₋₁₀ cycloalkyl" means a cycloalkyl containing 3 to 10 carbon atoms, "C₃₋₈ cycloalkyl" means a cycloalkyl containing 3 to 8 carbon atoms, and "C₃₋₆ cycloalkyl" means a cycloalkyl containing 3 to 6 carbon atoms, wherein:
Monocyclic cycloalkyl includes, but is not limited to cyclopropyl, cyclobutyl, cyclopentyl, cyclopentenyl, cyclohexyl, cyclohexenyl, cyclohexadienyl, cycloheptyl, cycloheptatrienyl, cyclooctyl and the like;
and polycyclic cycloalkyl includes spiro, fused, and bridged cycloalkyls. "Spirocycloalkyl" refers to a polycyclic group in which a carbon atom (called spiro-atom) is shared among monocyclic rings, wherein those rings may contain one or more (preferably, 1, 2 or 3) double bonds, but none of them has a fully conjugated π-electron system. According to the number of the spiro-atoms shared among the rings, the spirocycloalkyl may be monospirocycloalkyl, bispirocycloalkyl or polyspirocycloalkyl, including but not limited to:

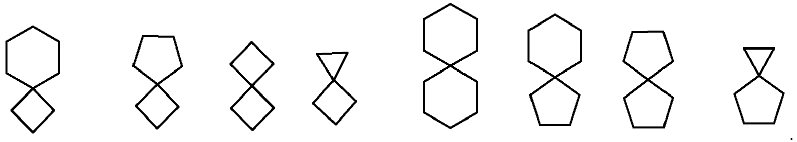

"Fused cycloalkyl" refers to an all-carbon polycyclic group in which each ring shares a pair of adjacent carbon atoms with the other rings in the system, wherein one or more of the rings may contain one or more (preferably, 1, 2 or 3) double bonds, but none of them has a fully conjugated π-electron system. According to the number of formed rings, the fused cycloalkyl may be bicyclic, tricyclic, tetracyclic or polycyclic, including but not limited to:

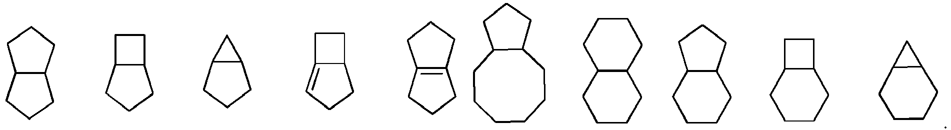

"Bridged cycloalkyl" refers to an all-carbon polycyclic group in which any two rings share two carbon atoms that are not directly connected to each other, wherein these rings may contain one or more (preferably, 1, 2 or 3) double bonds, but none of them has a fully conjugated π-electron system. According to the number of formed rings, the bridged cycloalkyl may be bicyclic, tricyclic, tetracyclic or polycyclic, including but not limited to:

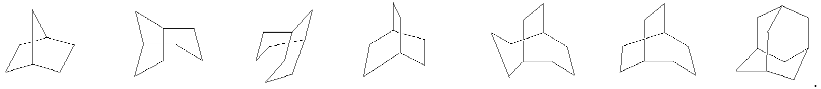

The cycloalkyl ring can be fused to an aryl, heteroaryl or heterocycloalkyl ring, wherein the ring attached to the parent structure is cycloalkyl, which includes, but is not limited to, indanyl, tetrahydronaphthyl, benzocycloheptyl, etc.

Cycloalkyl may be optionally substituted or unsubstituted, and when it is substituted, the substituent is preferably one or more (preferably, 1, 2, 3, or 4) of the groups below independently selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₅₋₁₀ aryl, 5-10 membered heteroaryl, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Heterocyclyl"or "heterocycle" refers to a monocyclic or polycyclic cyclic hydrocarbon substituent that is saturated or partially unsaturated. The partially unsaturated cyclic hydrocarbon means a cyclic hydrocarbon that may contain one or more (preferably 1, 2 or 3) double bonds, but none of rings has a fully conjugated π-electron system. One or more (preferably 1, 2, 3 or 4) ring atoms in the heterocyclyl are selected from the group consisting of heteroatoms of nitrogen, oxygen, S(O)(=NH) or S(O)ᵣ (wherein r is an integer of 0, 1, or 2), but excluding the ring moiety of -OO-, - OS- or -SS-, and the remaining ring atoms are carbon. The heterocyclyl preferably includes the one containing 3 to 10 or 3 to 8 or 3 to 6 ring atoms. For example, "3-6 membered heterocyclyl" refers to a ring group containing 3 to 6 ring atoms; "4-6 membered heterocyclyl" refers to a ring group containing 4 to 6 ring atoms; and "3-10 membered heterocyclyl" refers to a ring group containing 3 to 10 ring atoms.

Monocyclic heterocyclyl includes, but is not limited to pyrrolidinyl, piperidinyl, piperazinyl, morpholinyl, thiomorpholinyl, homopiperazinyl and the likes.

Polycyclic heterocyclyl includes spiro, fused, and bridged heterocyclyls. "Spiroheterocyclyl" refers to a polycyclic heterocyclyl group that shares a carbon atom (called a spiro atom) between the monocyclic rings, wherein one or more (preferably 1, 2, 3 or 4) of the ring atoms are heteroatoms selected from nitrogen, oxygen, S(O)(=NH) or S(O)ᵣ (wherein r is an integer of 0, 1, 2), and the remaining ring atoms are carbon atoms. These groups may contain one or more (preferably 1, 2, or 3) double bonds, but none of the rings have a fully conjugated π-electron system. The spiroheterocyclyl may be a monospiroheterocyclyl, a bispiroheterocyclyl or a polyspiroheterocyclyl according to the number of spiro atoms shared between the rings. Spiroheterocyclyl includes, but is not limited to:

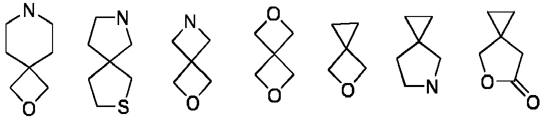

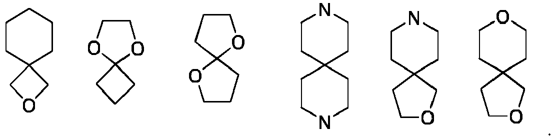

"Fused heterocyclyl" refers to a polycyclic heterocyclyl group in which each ring shares an adjacent pair of carbon atoms with other rings in the system, wherein one or more (preferably 1, 2, 3 or 4) of the rings may contain one or more (preferably 1, 2 or 3) double bonds, but none of the rings have a fully conjugated π-electron system, wherein one or more (preferably 1, 2, 3 or 4) of the ring atoms are heteroatoms selected from nitrogen, oxygen, S(O)(=NH) or S(O)ᵣ (wherein r is an integer of 0, 1, 2), and the remaining ring atoms are carbon atoms. Depending on the number of rings, it may be bicyclic, tricyclic, tetracyclic or polycyclic, fused heterocyclyl includes, but is not limited to:

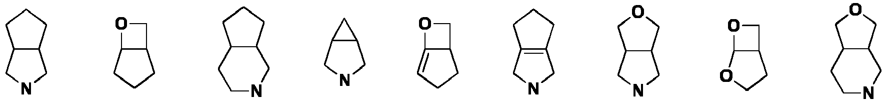

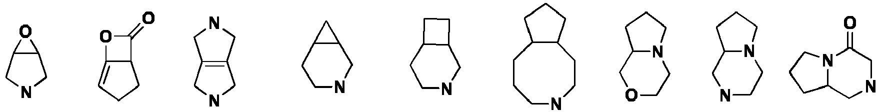

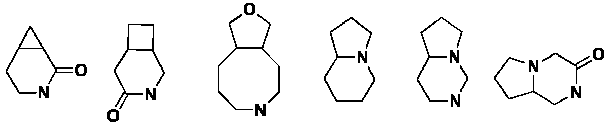

"Bridged heterocyclyl" refers to a polycyclic heterocyclyl group in which any two rings share two carbon atoms that are not directly bonded, which may contain one or more (preferably 1, 2 or 3) double bonds, but none of the rings have a fully conjugated π-electron system, wherein one or more (preferably 1, 2, 3 or 4) of the ring atoms are heteroatoms selected from nitrogen, oxygen, S(O)(=NH) or S(O)ᵣ (wherein r is an integer of 0, 1, 2), and the remaining ring atoms are carbon atoms. Depending on the number of rings, it may be bicyclic, tricyclic, tetracyclic or polycyclic, bridged heterocyclyl includes, but is not limited to:

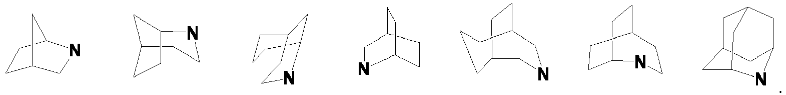

The ring of the heterocyclyl may be fused to a ring of aryl, heteroaryl or cycloalkyl wherein the ring attached to the parent structure is a heterocyclyl, which includes, but is not limited to:

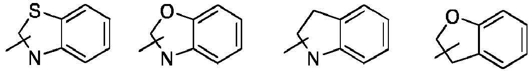

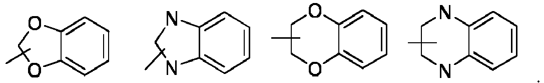

Heterocyclyl may be optionally substituted or unsubstituted, and when it is substituted, the substituent is preferably one or more (preferably 1, 2, 3 or 4) of the groups below independently selected from the group consisting of deuterium, halogen, cyano, nitro, azido, C₁₋₁₀ alkyl, C₁₋₁₀ alkoxy, C₂₋₁₀ alkenyl, C₂₋₁₀ alkynyl, C₁₋₁₀ haloalkyl, C₁₋₁₀ haloalkoxy, C₁₋₁₀ deuterioalkyl, C₁₋₁₀ deuterioalkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, - C₀₋₈ alkyl-C(O)NR₁₃R₁₄ and -C₀₋₈ alkyl-N(R₁₃)-C(O)R₁₂.

"Aryl" or "aromatic ring" refers to an all-carbon monocyclic or fused polycyclic (i.e., a ring that shares a pair of adjacent carbon atoms) group, and a polycyclic group having a conjugated π-electron system (i.e., a ring with adjacent pairs of carbon atoms). For example, an all-carbon aryl containing 5-10 or 5-8 carbons are preferred. For example, "C₅₋₁₀ aryl" refers to an all-carbon aryl containing 5-10 carbons, including but not limited to phenyl and naphthyl. The aryl ring may be fused to a ring of heteroaryl, heterocyclyl or cycloalkyl, wherein the ring attached to the parent structure is an aryl ring, which includes, but is not limited to:

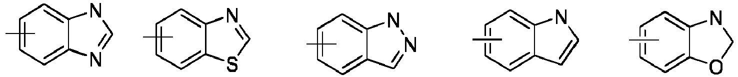

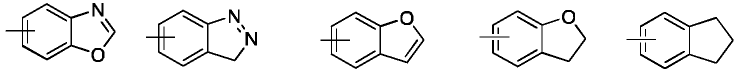

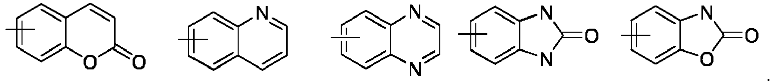

"Aryl" may be substituted or unsubstituted, and when it is substituted, the substituent is preferably one or more (preferably, 1, 2, 3 or 4) of the groups below independently selected from the group consisting of deuterium, halogen, cyano, nitro, azido, C₁₋₁₀ alkyl, C₁₋₁₀ alkoxy, C₂₋₁₀ alkenyl, C₂₋₁₀ alkynyl, C₁₋₁₀ haloalkyl, C₁₋₁₀ haloalkoxy, C₁₋₁₀ deuterioalkyl, C₁₋₁₀ deuterioalkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Heteroaryl" refers to a heteroaromatic system containing one or more (preferably 1, 2, 3 or 4) heteroatoms including a heteroatom selected from the group consisting of nitrogen, oxygen or S(O)r (wherein r is an integer of 0, 1, 2). The heteroaromatic system containing 5-10 or 5-8 ring atoms is preferred. For example, 5-10 membered heteroaryl refers to a heteroaromatic system containing 5 to 10 ring atoms, including but not limited to furyl, thiophenyl, pyridyl, pyrrolyl, N-alkylpyrrolyl, pyrimidinyl, pyrazinyl, imidazolyl, tetrazolyl group or the likes. The heteroaryl ring may be fused to a ring of aryl, heterocyclyl or cycloalkyl wherein the ring attached to the parent structure is a heteroaryl ring, which includes, but is not limited to:

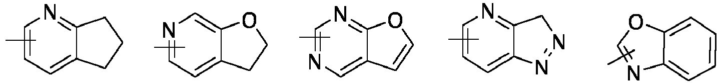

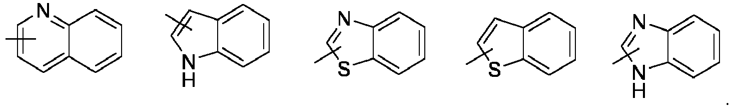

"Heteroaryl" may be optionally substituted or unsubstituted, and when it is substituted, the substituent is preferably one or more (preferably 1, 2, 3, or 4) of the groups independently selected from the group consisting of deuterium, halogen, cyano, nitro, azido, C₁₋₁₀ alkyl, C₁₋₁₀ alkoxy, C₂₋₁₀ alkenyl, C₂₋₁₀ alkynyl, C₁₋₁₀ haloalkyl, C₁₋₁₀ haloalkoxy, C₁₋₁₀ deuterioalkyl, C₁₋₁₀ deuterioalkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Alkenyl" refers to an alkyl group defined as above consisting of at least two carbon atoms and at least one carbon-carbon double bond, preferably a linear or branched alkenyl containing 2-10 or 2-4 carbons. For example, C₂₋₁₀ alkenyl refers to a linear or branched alkenyl containing 2 to 10 carbons, C₂₋₄ alkenyl refers to a linear or branched alkenyl containing 2 to 4 carbons. Alkenyl includes, but is not limited to, vinyl, 1-propenyl, 2-propenyl, 1-, 2- or 3-butenyl, and the likes.

"Alkenyl" may be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ alkoxy, C₂₋₁₀ alkenyl, C₂₋₁₀ alkynyl, C₁₋₁₀ haloalkyl, C₁₋₁₀ haloalkoxy, C₁₋₁₀ deuterioalkyl, C₁₋₁₀ deuterioalkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and - C₀₋₈ alkyl-N(R₁₃)-C(O)R₁₂.

"Alkynyl" refers to an alkyl group defined as above consisting of at least two carbon atoms and at least one carbon-carbon triple bond, preferably a linear or branched alkynyl containing 2-10 or 2-4 carbons. For example, C₂₋₁₀ alkynyl refers to a linear or branched alkynyl containing 2 to 10 carbons, and C₂₋₄ alkynyl refers to a linear or branched alkynyl containing 2 to 4 carbons. Alkynyl includes, but is not limited to, ethynyl, 1-propynyl, 2-propynyl, 1-, 2- or 3-butynyl, and the likes.

"Alkynyl" may be substituted or unsubstituted, and when it is substituted, the substituent is preferably one or more (preferably, 1, 2, 3 or 4) of the groups below independently selected from the group consisting of deuterium, halogen, cyano, nitro, azido, C₁₋₁₀ alkyl, C₁₋₁₀ alkoxy, C₂₋₁₀ alkenyl, C₂₋₁₀ alkynyl, C₁₋₁₀ haloalkyl, C₁₋₁₀ haloalkoxy, C₁₋₁₀ deuterioalkyl, C₁₋₁₀ deuterioalkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂.

"Alkoxy" refers to -O-alkyl, wherein alkyl is defined as above. For example, "C₁₋₁₀ alkoxy" refers to alkyloxy containing 1 to 10 carbons, and C₁₋₄ alkoxy refers to alkyloxy containing 1-4 carbons. Alkoxy includes, but is not limited to, methoxy, ethoxy, propoxy, butoxy, and the likes.

"Alkoxy" may be optionally substituted or unsubstituted, and when it is substituted, the substituent is preferably one or more (preferably, 1, 2, 3, or 4) of the groups below independently selected from the group consisting of deuterium, halogen, cyano, nitro, azido, C₁₋₁₀ alkyl, C₁₋₁₀ alkoxy, C₂₋₁₀ alkenyl, C₂₋₁₀ alkynyl, C₁₋₁₀ haloalkyl, C₁₋₁₀ haloalkoxy, C₁₋₁₀ deuterioalkyl, C₁₋₁₀ deuterioalkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, - C₀₋₈ alkyl-C(O)NR₁₃R₁₄ and -C₀₋₈ alkyl-N(R₁₃)-C(O)R₁₂.

"Cycloalkyloxy" refers to -O-cycloalkyl, wherein cycloalkyl is defined as above, for example, "C₃₋₁₀ cycloalkyloxy" refers to a cycloalkyloxy containing 3 to 10 carbons. Cycloalkyloxy includes, but is not limited to, cyclopropyloxy, cyclobutyloxy, cyclopentyloxy, cyclohexyloxy and the likes.

"Cycloalkyloxy" may be optionally substituted or unsubstituted, and when it is substituted, the substituent is preferably one or more (preferably 1, 2, 3, or 4) of the groups below independently selected from the group consisting of deuterium, halogen, cyano, nitro, azido, C₁₋₁₀ alkyl, C₁₋₁₀ alkoxy, C₂₋₁₀ alkenyl, C₂₋₁₀ alkynyl, C₁₋₁₀ haloalkyl, C₁₋₁₀ haloalkoxy, C₁₋₁₀ deuterioalkyl, C₁₋₁₀ deuterioalkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, - C₀₋₈ alkyl-C(O)NR₁₃R₁₄ and -C₀₋₈ alkyl-N(R₁₃)-C(O)R₁₂.

"Heterocyclyloxy" refers to -O-heterocyclyl, wherein the heterocyclyl is defined as above. The heterocyclyloxy includes, but is not limited to, azacyclobutyloxy, oxacyclobutyloxy, azacyclopentyloxy, nitrogen, oxacyclohexyloxy and the likes.

"Heterocyclyloxy" may be optionally substituted or unsubstituted, and when it is substituted, the substituent is preferably one or more (preferably 1, 2, 3, or 4) of the groups below independently selected from the group consisting of deuterium, halogen, cyano, nitro, azido, C₁₋₁₀ alkyl, C₁₋₁₀ alkoxy, C₂₋₁₀ alkenyl, C₂₋₁₀ alkynyl, C₁₋₁₀ haloalkyl, C₁₋₁₀ haloalkoxy, C₁₋₁₀ deuterioalkyl, C₁₋₁₀ deuterioalkoxy, C₃₋₁₀ cycloalkyl, C₃₋₁₀ cycloalkyloxy, 3-10 membered heterocyclyl, 3-10 membered heterocyclyloxy, C₅₋₁₀ aryl, C₅₋₁₀ aryloxy, 5-10 membered heteroaryl, 5-10 membered heteroaryloxy, =O, -SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, - C₀₋₈ alkyl-C(O)NR₁₃R₁₄ and -C₀₋₈ alkyl-N(R₁₃)-C(O)R₁₂.

"C₁₋₁₀ alkanoyl" refers to a monovalent atomic group obtained by removing hydroxy from C₁₋₁₀ alkyl acid, and it is also generally represented as "C₀₋₉ alkyl-C(O)-". For example, "C₁ alkyl-C(O)-" refers to acetyl; "C₂ alkyl-C(O)-" refers to propionyl; and "C₃ alkyl-C(O)-" refers to butyryl or isobutyryl.

"-C₀₋₈ alkyl-S(O)ᵣR₁₀" means that the sulfur atom in -S(O)ᵣR₁₀ is bonded to C₀₋₈ alkyl, wherein C₀₋₈ alkyl is defined as above.

"-C₀₋₈ alkyl-O-R₁₁" means that the oxygen atom in -O-R₁₁ is bonded to C₀₋₈ alkyl, wherein C₀₋₈ alkyl is defined as above.

"-C₀₋₈ alkyl-C(O)OR₁₁" means that the carbonyl group in -C(O)OR₁₁ is bonded to C₀₋₈ alkyl, wherein C₀₋₈ alkyl is defined as above.

"-C₀₋₈ alkyl-C(O)R₁₂" means that the carbonyl group in -C(O)R₁₂ is bonded to C₀₋₈ alkyl, wherein C₀₋₈ alkyl is defined as above.

"-C₀₋₈ alkyl-O-C(O)R₁₂" means that the oxygen atom in -O-C(O)R₁₂ is bonded to C₀₋₈ alkyl, wherein C₀₋₈ alkyl is defined as above.

"-C₀₋₈ alkyl-NR₁₃R₁₄" means that the nitrogen atom in -NR₁₃R₁₄ is bonded to C₀₋₈ alkyl, wherein C₀₋₈ alkyl is defined as above.

"-C₀₋₈ alkyl-C(=NR₁₃)R₁₂" means that the carbon atom in -C(=NR₁₃)R₁₂ is bonded to C₀₋₈ alkyl, wherein C₀₋₈ alkyl is defined as above.

"-C₀₋₈ alkyl-N(R₁₃)-C(=NR₁₄)R₁₂" means that the nitrogen atom in -N(R₁₃)-C(=NR₁₄)R₁₂ is bonded to C₀₋₈ alkyl, wherein C₀₋₈ alkyl is defined as above.

"-C₀₋₈ alkyl-C(O)NR₁₃R₁₄" means that the carbonyl in -C(O)NR₁₃R₁₄ is bonded to C₀₋₈ alkyl, wherein C₀₋₈ alkyl is defined as above.

"-C₀₋₈ alkyl-N(R₁₃)-C(O)R₁₂" means that the nitrogen atom in -N(R₁₃)-C(O)R₁₂ is bonded to C₀₋₈ alkyl, wherein C₀₋₈ alkyl is defined as above.

"C₁₋₄ haloalkyl" means an alkyl group having 1 to 4 carbon atoms, wherein any hydrogen atom on which is optionally substituted with F, Cl, Br or I, and includes, but is not limited to, difluoromethyl (-CHF₂), dichloromethyl (-CHCl₂), dibromomethyl (-CHBr₂), trifluoromethyl (-CF₃), trichloromethyl (-CCl₃), tribromomethyl (-CBr₃), and the likes.

"C₁₋₄ haloalkoxy" means an alkoxy group having 1 to 4 carbon atoms, wherein any hydrogen atom on which is optionally substituted with F, Cl, Br or I atom. It includes, but is not limited to, difluoromethoxy, dichloromethoxy, dibromomethoxy, trifluoromethoxy, trichloromethoxy, tribromomethoxy, and the likes.

"C₁₋₄ deuterioalkyl" means an alkyl group having 1 to 4 carbon atoms, wherein any hydrogen atom on which is optionally substituted with deuterium atom. It includes, but is not limited to, monodeuteriomethyl (-CH₂D), dideuteriomethyl (-CHD₂), trideuteriomethyl (-CD₃), and the likes.

"C₁₋₄ deuterioalkoxy" means an alkoxy group having 1 to 4 carbon atoms, wherein any hydrogen atom on which is optionally substituted with deuterium atom. It includes, but is not limited to, monodeuteriomethoxy, dideuteriomethoxy, trideuteriomethoxy, and the likes.

"Halogen" means F, Cl, Br or I. "MeOH" means methanol. "KF" means potassium fluoride. " KHMDS" means potassium bis(trimethylsilyl)amide. "NBS" means N-bromosuccinimide. "DMF" means N,N-dimethylformamide. "Grubbs G2 catalyst" means the second-generation catalyst from Grubbs. "IBX" means 2-iodoyl benzoic acid.

"Optional" or "optionally" means that the event or environment subsequently described may, but need not, occur, including where the event or environment occurs or does not occur, that is, including both substituted and unsubstituted situations. For example, "heterocyclyl optionally substituted by alkyl" means that an alkyl group may be, but is not necessarily, present, and the description includes the case where the heterocyclyl is substituted with an alkyl and the case where the heterocyclyl is not substituted with an alkyl.

The term "substituted" means that one or more "hydrogen atoms" in the group are each independently substituted by a corresponding number of substituents. It goes without saying that a substituent is only in its possible chemical position, which is consistent with the valence-bond theory of chemistry. Those skilled in the art will be able to determine possible or impossible substitution (by experiments or theories) without undue efforts. For example, it may be unstable when an amino group or a hydroxyl group having a free hydrogen is attached with a carbon atom having an unsaturated bond (such as an olefin).

"Stereoisomer" means an isomer produced due to a different spatial arrangement of atoms in the molecules, and can be classified into either cis-trans isomers and enantiomers, or enantiomers and diastereomers. Stereoisomers resulting from the rotation of a single bond are called conformational stereo-isomers, and sometimes also called rotamers. Stereoisomers induced by reasons such as bond lengths, bond angles, double bonds in molecules and rings are called configuration stereo-isomers, which are classified into two categories. Among them, isomers induced by the double bonds or single bonds of ring-forming carbon atoms that cannot rotate freely are called geometric isomers, also known as cis-trans isomers, which are divided into two configurations including Z and E. For example, cis-2-butene and trans-2-butene are a pair of geometric isomers. If the compound of the present invention contains a double bond, it can be understood as containing a E and/or Z form, unless otherwise specifically indicated. Stereoisomers with different optical activities due to the absence of anti-axial symmetry in the molecules are called optical isomers, which are classified into two configurations including R and S. Unless otherwise specified, the "stereoisomer" in the present invention can be understood as including one or several of the above-mentioned enantiomers, configurational isomers and conformational isomers, preferably the R configuration.

"Pharmaceutically acceptable salt" in the present invention means pharmaceutically acceptable acid addition salts or alkali addition salts, including inorganic acid salts and organic acid salts, and these salts can be prepared by methods known in the art.

"Pharmaceutical composition" refers to a mixture comprising one or more of the compounds described herein, or a physiologically/pharmaceutically acceptable salt or pro-drug thereof, and other chemical components, for example physiological/pharmaceutically acceptable carriers and excipients. The purpose of the pharmaceutical composition is to promote the administration to an organism, which facilitates the absorption of the active ingredient thereby exerting biological activities.

**The present invention will be further described in detail below in conjunction with the embodiments which is not intended to limit the present invention. The present invention is also not limited to the contents of the embodiments.**

The structure of the compound of the present invention is determined by nuclear magnetic resonance (NMR) or/and liquid chromatography-mass spectrometry (LC-MS). The NMR chemical shift (δ) is given in parts per million (ppm). The NMR is measured by a Bruker AVANCE-400/500 nuclear magnetic apparatus, and the solvent is deuterated dimethyl sulfoxide (DMSO-*d₆*), deuterated methanol (CD₃OD) and deuterated chloroform (CDCl₃), and the internal standard is tetramethylsilane (TMS).

The measurement of LC-MS is performed by using an Agilent 6120 mass spectrometer. The measurement of HPLC is performed by using an Agilent 1200 DAD high pressure liquid chromatograph (Sunfire C18 150 × 4.6 mm column) and a Waters 2695-2996 high pressure liquid chromatograph (Gimini C18 150 × 4.6 mm column).

The thin layer chromatography silica gel plate is Yantai Yellow Sea HSGF254 or Qingdao GF254 silica gel plate. The specification of TLC is 0.15 mm - 0.20 mm, and the specification for thin layer chromatography separation and purification is 0.4 mm - 0.5 mm. 200-300 mesh silica gel (Yantai Huanghai silica gel) as a carrier is generally used in column chromatography.

The starting materials in the embodiments of the present invention are known and commercially available or can be synthesized according to methods known in the art.

Unless otherwise stated, all reactions of the present invention are carried out under continuous magnetic stirring in a dry nitrogen or argon atmosphere, the solvent is a dry solvent, and the unit of the reaction temperature is Celsius degree (°C).

### I. Preparation of Intermediates

### Intermediate A: Preparation of 2-isopropyl-4-vinylpyridin-3-amine

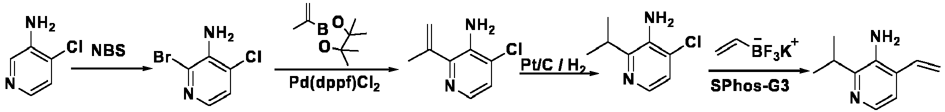

### Step 1: Synthesis of 2-bromo-4-chloropyridin-3-amine

4-chloropyridin-3-amine (20.0 g, 156 mmol) was dissolved in trifluoroacetic acid (60 mL), and NBS (31.0 g, 174 mmol) was added in portions. The mixture was stirred for 5 hrs at room temperature, and the reaction was stopped. About two-thirds of the solvent trifluoroacetic acid was evaporated under reduced pressure. The residue was diluted with 100 mL of water, dropwise added with hydrochloric acid to adjust pH to 8 under an ice bath, and extracted with ethyl acetate (30 mL * 3). The organic layers were combined, washed with saline water (50 mL * 2), dried over anhydrous sodium sulfate, and then concentrated under reduced pressure to remove the solvent. The residue was separated by flash silicagel columns to obtain 2-bromo-4-chloropyridin-3-amine (13.5 g, yield: 41.8%). ESI-MS: 207 [M+1]⁺.

¹H NMR (400 MHz, DMSO-*d*₆) δ 7.57 (d, *J* = 5.0 Hz, 1H), 7.36 (d, *J* = 5.0 Hz, 1H), 5.70 (s, 2H).

### Step 2: Synthesis of 4-chloro-2-(prop-1-ene-2-yl)pyridin-3-amine

2-bromo-4-chloropyridin-3-amine (13.50 g, 65.2 mmol) and 4,4,5,5-tetramethyl-2-(prop-1-ene-2-yl)-1,3,2-dioxaborolane (13.5 g, 71.7 mmol) were dissolved in the mixed solvent of 1,4-dioxane (100 mL) and water (40 mL). [1,1'-bis(diphenylphosphino)ferrocene]palladium dichloride (3.0 g, 4.0 mmol) and cesium carbonate (55 g, 169 mmol) were added. The mixture was stirred for 8 hrs at 90°C under the protection of nitrogen, and the reaction was stopped. The reactant mixture was diluted with 200 mL of ethyl acetate, washed with water (100 mL * 2), washed with saline water (100 mL * 2), dried over anhydrous sodium sulfate, and then concentrated under reduced pressure to remove the solvent. The residue was separated by flash silicagel columns to obtain 4-chloro-2-(prop-1-ene-2-yl)pyridin-3-amine (10.5g, yield: 95.3%). ESI-MS: 169 [M+1]⁺.

### Step 3: Synthesis of 4-chloro-2-isopropylpyridin-3-amine

4-chloro-2-(prop-1-ene-2-yl)pyridin-3-amine (10.5 g, 62 mmol) was dissolved in methanol (30 mL). 5% palladium on carbon (1.1 g) was added. Nitrogen displacement was conducted. Then, hydrogen (1 atm) was introduced. The mixture was stirred at room temperature to react for 16 hrs, and filtered with diatomite. The filtrate was concentrated under reduced pressure to obtain 4-chloro-2-isopropylpyridin-3-amine (10.2 g, yield: 96.43%). ESI-MS: 171 [M+1]⁺.

### Step 4: Synthesis of 2-isopropyl-4-vinylpyridin-3-amine

4-chloro-2-isopropylpyridin-3-amine (6.5 g, 38 mmol) and potassium trifluoro(vinyl)borate (7.1 g, 57 mmol) was dissolved in the mixed solvent of glycol dimethyl ether (40 mL) and water (30 mL). Then, palladium methanesulfonate (2-dicyclohexylphosphino-2', 6'-dimethoxy-1,1'-biphenyl)(2'-amino-1,1'-xenene-2-yl) (II)(3.0 g, 3.5 mmol) and sodium carbonate (11.0 g, 95 mmol) were added. The mixture was stirred for 8 hrs at 90°C under the protection of nitrogen, and the reaction was stopped. After the organic layer was formed, the water phase was extracted with ethyl acetate (30 mL * 2). The organic layers were combined, washed with saline water, dried over anhydrous sodium sulfate, filtered, and concentrated under reduced pressure to remove the solvent. The residue was separated by flash silicagel columns to obtain 2-isopropyl-4-vinylpyridin-3-amine (5.4 g, yield: 87.6%). ESI-MS: 163 [M+1]⁺.

¹H NMR (400 MHz, DMSO-*d*₆) δ 7.11 (dd, *J* = 7.7, 1.5 Hz, 1H), 7.03 - 6.90 (m, 2H), 6.56 (t, *J* = 7.6 Hz, 1H), 5.52 (dd, *J =* 17.2, 1.8 Hz, 1H), 5.16 (dd, *J* = 10.9, 1.8 Hz, 1H), 3.00 (hept, *J* = 6.7 Hz, 1H), 1.14 (d, *J*=7.2Hz, 6H).

### Intermediate B1: Preparation of tert-butyl (S,Z)-4-(2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6-ene-2⁴-yl)-3-methylpiperazine-1-carboxylate

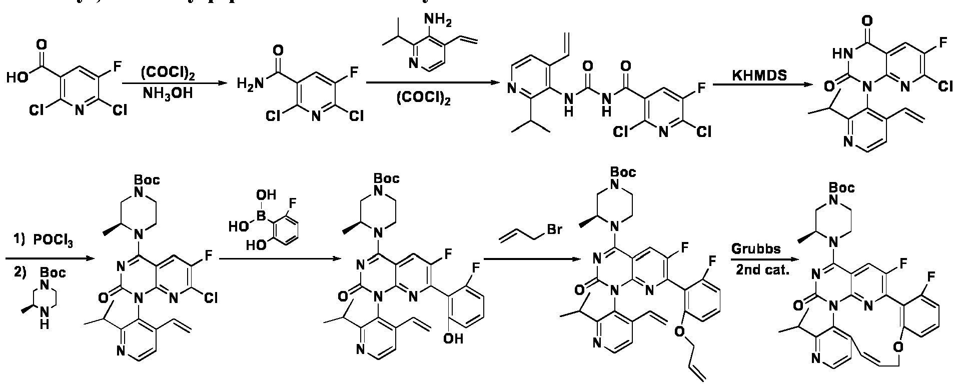

### Step 1: Synthesis of 2,6-dichloro-5-fluoronicotinamide

2,6-dichloro-5-fluoronicotinic acid (10.0 g, 47.6 mmol) was dissolved in dichloromethane (30 mL). A catalytic amount of DMF was added. The mixture was stirred at room temperature while oxalyl chloride (6.3 g, 50 mmol) was added slowly, after which the mixture was stirred for 2 hrs at room temperature. The reactant mixture was cooled to 0°C, slowly added with 25% ammonia water (18 mL), naturally warmed up to room temperature, stirred for 1 hr, and concentrated under reduced pressure to remove the solvent. Ethyl acetate (30 mL) and n-hexane (75 mL) were added to the residue for pulping. The mixture was filtered and dried to obtain 2,6-dichloro-5-fluoronicotinamide (8.0 g, yield: 80.4%). ESI-MS 209 [M+1]⁺.

### Step 2: Synthesis of 2,6-dichloro-5-fluoro-N-((2-isopropyl-4-vinylpyridin-3-yl)carbamyl)nicotinamide

2,6-dichloro-5-fluoronicotinamide (2.9 g, 14 mmol) was dissolved in tetrahydrofuran (30 mL). Oxalyl chloride (1.9 g, 14.8 mmol) was added. The mixture was heated and refluxed for 2 hrs. The reactant mixture was cooled to 0°C, added with 2-isopropyl-4-vinylpyridin-3-amine (2.9 g, 14 mmol), naturally warmed up to room temperature, and then stirred for 1 h. The reaction was stopped, and saturated ammonium chloride in water (30 mL) was added to quench the reaction. Extraction was conducted with ethyl acetate (30 mL*3). The organic phases were combined, washed with water (30 mL), washed with saline water (30 mL), dried over anhydrous sodium sulfate, and then concentrated under reduced pressure to remove the solvent. The residue was separated by flash silicagel columns to obtain 2,6-dichloro-5-fluoro-N-((2-isopropyl-4-vinylpyridin-3-yl)carbamyl)nicotinamide (6.0 g, yield: 94.5%). ESI-MS 397 [M+1]⁺.

### Step 3: Synthesis of 7-chloro-6-fluoro-1-(2-isopropyl-4-vinylpyridin-3-yl)pyrido[2,3-d]pyrimidina-2,4(1H,3H)-dione

2,6-dichloro-5-fluoro-N-((2-isopropyl-4-vinylpyridin-3-yl)carbamyl)nicotinamide (6.0 g, 14 mmol) was dissolved in tetrahydrofuran (60 mL), dropwise added with bis(trimethylsilicyl)potassamide (28 mL, 28 mmol) under an ice bath, and naturally warmed up to room temperature to react for 1 hr. After the reaction was ended, saturated ammonium chloride in water (30 mL) was added for quenching. Extraction was conducted with ethyl acetate (30 mL*3). The organic phases were combined, washed with water (50 mL), washed with saline water (50 mL), dried over anhydrous sodium sulfate, and then concentrated under reduced pressure to remove the solvent. The residue was separated by flash silicagel columns to obtain 7-chloro-6-fluoro-1-(2-isopropyl-4-vinylpyridin-3-yl)pyrido[2,3-d]pyrimidina-2,4(1H,3H)-dione (4.3 g, yield: 84.3%). ESI-MS 361[M+1]⁺.

### Step 4: Synthesis of (S)-tert-butyl 4-(7-chloro-6-fluoro-1-(2-isopropyl-4-vinylpyridin-3-yl)-2-oxo-1,2-dihydropyrido [2,3-d] pyrimidina-4-yl)-3-methylpiperazine-1-carboxylate

7-chloro-6-fluoro-1-(2-isopropyl-4-vinylpyridin-3-yl)pyrido[2,3-d]pyrimidina-2,4(1H,3H)-dione (4.3 g, 11.9 mmol) was dissolved in acetonitrile (50 mL). Diisopropylethylamine (6.0 g, 47.6 mmol) and phosphoryl trichloride (5.5 g, 35.7 mmol) were added. The mixture was stirred for 1 h at 80°C. The mixture was cooled to 25°C, and then added with diisopropylethylamine (4.6 g, 35.7 mmol) and (S)-tert-butyl 3-methylpiperazine-1-carboxylate (4.7 g, 23.8 mmol) to react for 2 hrs at room temperature. The reaction solution was diluted with ethyl acetate (50 mL), washed with saturated sodium chloride water (30 mL * 2), dried over anhydrous sodium sulfate, and then concentrated under reduced pressure to remove the solvent. The residue was separated by flash silicagel columns to obtain (S)-tert-butyl 4-(7-chloro-6-fluoro-1-(2-isopropyl-4-vinylpyridin-3-yl)-2-oxo-1,2-dihydropyrido[2,3-d]pyrimidina-4-yl)-3-methylpiperazine-1-carboxylate (5.2 g, yield: 79.6%). ESI-MS 543 [M+1]⁺.

### Step 5: Synthesis of (3S)-tert-butyl 4-(6-fluoro-7-(2-fluoro-6-hydroxyphenyl)-1-(2-isopropyl-4-vinylpyridin-3-yl)-2-oxo-1,2-dihydropyrido [2,3-d] pyrimidina-4-yl)-3-methylpiperazine-1-carboxylate

(S)-tert-butyl 4-(7-chloro-6-fluoro-1-(2-isopropyl-4-vinylpyridin-3-yl)-2-oxo-1,2-dihydropyrido[2,3-d]pyrimidina-4-yl)-3-methylpiperazine-1-carboxylate (1.0 g, 1.78 mmol) was added into a microwave reaction tube; the mixed solvent of 1,4-dioxane (10 mL) and water (1.0 mL) was added; and (2-fluoro-6-hydroxyphenyl)boric acid (0.8 g, 5.36 mmol), tetra(triphenylphosphine)palladium (0.41 g, 0.35 mmol), and sodium carbonate (0.66 g, 5.36 mmol) were added. The mixture was treated with microwave at 120°C under the protection of nitrogen to react for 45 minutes. The reaction solution was diluted with ethyl acetate (30 mL), washed with saturated sodium chloride aqueous solution (20 mL * 2), dried over anhydrous sodium sulfate, and then concentrated under reduced pressure to remove the solvent. The residue was separated by flash silicagel columns to obtain (3S)-tert-butyl 4-(6-fluoro-7-(2-fluoro-6-hydroxyphenyl)-1-(2-isopropyl-4-vinylpyridin-3-yl)-2-oxo-1,2-dihydropyrido[2,3-d]pyrimidina-4-yl)-3-methylpiperazine-1-carboxylate (0.81 g, yield: 71.3%). ESI-MS 619 [M+1]⁺.

### Step 6: Synthesis of (3S)-tert-butyl 4-(7-(2-(allyloxy)-6-fluorophenyl)-6-fluoro-1-(2-isopropyl-4-vinylpyridin-3-yl)-2-oxo-1,2-dihydropyrido [2,3-d] pyrimidina-4-yl)-3-methylpiperazine-1-carboxylate

(3 S)-tert-butyl 4-(6-fluoro-7-(2-fluoro-6-hydroxyphenyl)-1-(2-isopropyl-4-vinylpyridin-3-yl)-2-oxo-1,2-dihydropyrido[2,3-d]pyrimidina-4-yl)-3-methylpiperazine-1-carboxylate (0.81 g, 1.27 mmol) was dissolved in acetonitrile (30 mL). 3-bromoprop-1-ene (0.23 g, 1.91 mmol) and potassium carbonate (0.44 g, 3.18 mmol) were added. The mixture was stirred for 2 hrs at 65°C, and the reaction was stopped. Ethyl acetate (30 mL) was added to the reaction solution for dilution. The diluted reaction solution was washed with saturated sodium chloride aqueous solution (20 mL * 2), dried over anhydrous sodium sulfate, and then concentrated under reduced pressure to remove the solvent. The residue was separated by flash silicagel columns to obtain (3S)-tert-butyl 4-(7-(2-(allyloxy)-6-fluorophenyl)-6-fluoro-1-(2-isopropyl-4-vinylpyridin-3-yl)-2-oxo-1,2-dihydropyrido[2,3-d]pyrimidina-4-yl)-3-methylpiperazine-1-carboxylate (0.73 g, yield: 67.7%). ESI-MS 659[M+1]⁺.

### Step 7: Synthesis of tert-butyl (S,Z)-4-(2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6-ene-2⁴-yl)-3-methylpiperazine-1-carboxylate

(3 S)-tert-butyl 4-(7-(2-(allyloxy)-6-fluorophenyl)-6-fluoro-1-(2-isopropyl-4-vinylpyridin-3-yl)-2-oxo-1,2-dihydropyrido[2,3-d]pyrimidina-4-yl)-3-methylpiperazine-1-carboxylate (0.73 g, 1.1 mmol) was dissolved in toluene (150 mL). The Grubbs G2 catalyst (0.49 g, 0.11 mmol) was added under the protection of nitrogen to allow the mixture to react for 1 hr at 60°C. The mixture was concentrated under reduced pressure to remove the solvent. The residue was separated by flash silicagel columns to obtain tert-butyl (S,Z)-4-(2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6-ene-2⁴-yl)-3-methylpiperazine-1-carboxylate (0.4 g, yield: 57.2%). ESI-MS 631 [M+1]⁺.

**Intermediate B2 can be prepared by selecting corresponding starting materials according to the complete or partial synthesis method for intermediate B1:**

| **Intermediate No.** | **Structural Formula** | **Chemical Name** | **ESI-MS: [M+1]⁺** |
|---|---|---|---|
| **B2** | 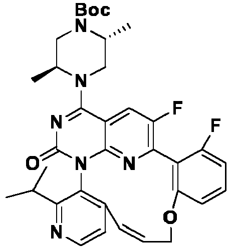 | Tert-butyl (S,Z)-4-(2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6-ene-2⁴-yl)-3,6-dimethylpiperazine-1- carboxylate | 645 |

### Intermediate C1: Preparation of tert-butyl (3S)-4-(2⁶,3⁶-difluoro-6-hydroxy-1²-isopropyl-2²-oxo-7-(propionyl(propionyloxy))-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate and 2⁴-((S)-4-(tert-butoxycarbonyl)-2-methylpiperazine-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6,7-diyl dipropionate

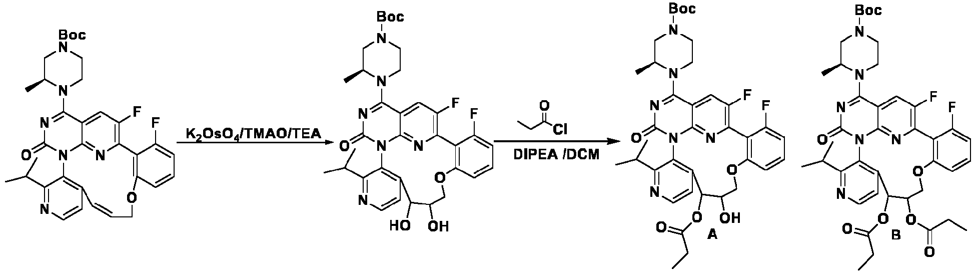

### Step 1: Synthesis of tert-butyl(3S)-4-(2⁶,3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate

Tert-butyl (S,Z)-4-(2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6-ene-2⁴-yl)-3-methylpiperazine-1-carboxylate (280 mg, 0.44 mmol) was dissolved in the mixed solvent of ethyl acetate (2 mL) and dioxane (2 mL). Potassium osmate hydrate (324 mg, 0.88 mmol), trimethylamine N-oxide (83 mg, 1.1 mmol), and triethylamine (278 mg, mmol) were added. The mixture was stirred for 15 minutes at room temperature. 1 mL of water was added. The mixture was continuously stirred to react for 30 minutes. The reaction solution was diluted with ethyl acetate, washed with water, and concentrated under reduced pressure to remove the solvent. The resultant was separated by reversed-phase chromatographic columns to obtain tert-butyl (3S)-4-(2⁶ 3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (140mg, yield: 58.4%). ESI-MS: 665 [M+1]⁺.

### Step 2: Synthesis of tert-butyl (3S)-4-(2⁶,3⁶-difluoro-6-hydroxy-1²-isopropyl-2²-oxo-7-(propionyl(propionyloxy))-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate

Tert-butyl (3S)-4-(2⁶,3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (120 mg, 0.166 mmol) was dissolved in dichloromethane (5 mL). Triethylamine (0.346 mL, 2.491 mmol) was added at room temperature, and then, propionyl chloride (0.029 mL, 0.332 mmol) was added dropwise. The reaction solution was stirred for 2 hrs at room temperature. After the reaction was basically ended, the reaction solution was extracted by layers with dichloromethane (40 mL) and ice water (40 mL). The organic phase was dried and filtered. The filtrate was concentrated. The residue was separated by reversed-phase columns C18 [5-95% acetonitrile: 0.01 mM NH₄HCO₃/H₂O] to obtain tert-butyl(3S)-4-(2⁶,3⁶-difluoro-6-hydroxy-1²-isopropyl-2²-oxo-7-(propionyl(propionyloxy))-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (43 mg, yield: 32%), ESI-MS 721[M+1]⁺. At the same time, 2⁴-((S)-4-(tert-butoxycarbonyl)-2-methylpiperazine-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6,7-diyl dipropionate (51 mg, yield: 33%) was obtained, ESI-MS 777 [M+1]⁺.

**Intermediate C2-C10 can be prepared by selecting corresponding starting materials according to the complete or partial synthesis method for intermediate C1:**

| **Intermediate No.** | **Structural Formula** | **Chemical Name** | **ESI-MS: [M+1]⁺** |
|---|---|---|---|
| **C2** | 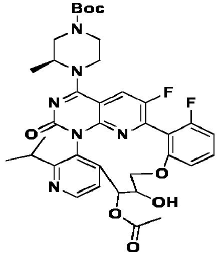 | Tert-butyl (3S)-4-(7-acetoxyl-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 707 |
| **C3** | 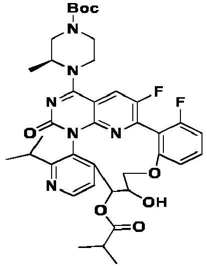 | Tert-butyl (3S)-4-(2⁶,3⁶-difluoro-6-hydroxy-7-(isobutoxy)-1²-isopropyl-2²-oxo-2¹,2²-dihydro-4-oxa-2(1,7)-pyrido[2,3-d]pyrimidina-1(3,4)-pyridina-3(1,2)-benzocycloheptane-24-yl)-3-methylpiperazine-1-carboxylate | 735 |
| **C4** | 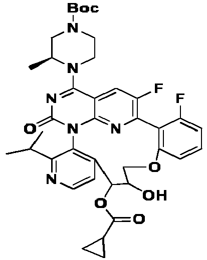 | 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl cyclopropanecarboxylate | 733 |
| **C5** | 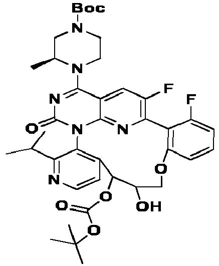 | Tert-butyl (3S)-4-(7-((tert-butoxycarbonyl)oxo)-2⁶ 3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3 -methylpiperazine-1 - carboxylate | 765 |
| **C6** | 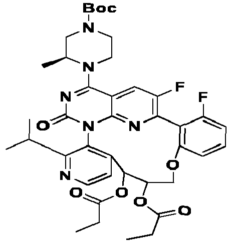 | 2⁴-((S)-4-(tert-butoxycarbonyl)-2-methylpiperazin-1-yl)-2⁶,3⁶-difluoro-1²-isopropyl-2²-oxo-2¹,2²-dihydro-4-oxa-2(1,7)-pyrido[2,3 -d]pyrimidina-1(3,4)-pyridina-3(1,2)-benzocycloheptane-6,7-diyl dipropionate | 777 |
| **C7** | 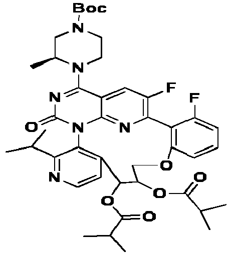 | 2⁴-((S)-4-(tert-butoxycarbonyl)-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6,7-diyl di(2-methylpropionate) | 805 |
| **C8** | 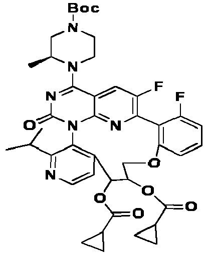 | 2⁴-((S)-4-(tert-butoxycarbonyl)-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6,7-diyl dicyclopropanecarboxylate | 801 |
| **C9** | 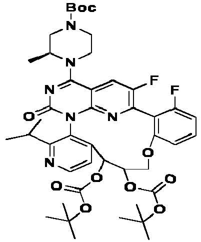 | Tert-butyl (3S)-4-(6,7-di((tert-butoxycarbonyl)oxo)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 865 |
| **C10** | 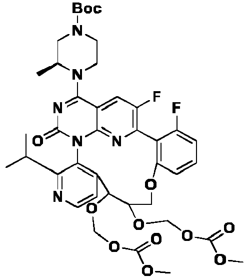 | Tert-butyl (3S)-4-(2⁶,3⁶-difluoro-1²-isopropyl-6,7-di(((carbomethoxy(methoxycarb onyl))oxo)methoxy)-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 841 |

### Intermediate D1: Preparation of tert-butyl (3S)-4-(2⁶,3⁶-difluoro-7-(2-fluoroethoxy)-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate and tert-butyl (3S)-4-(2⁶,3⁶-difluoro-6-(2-fluoroethoxy)-7-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate

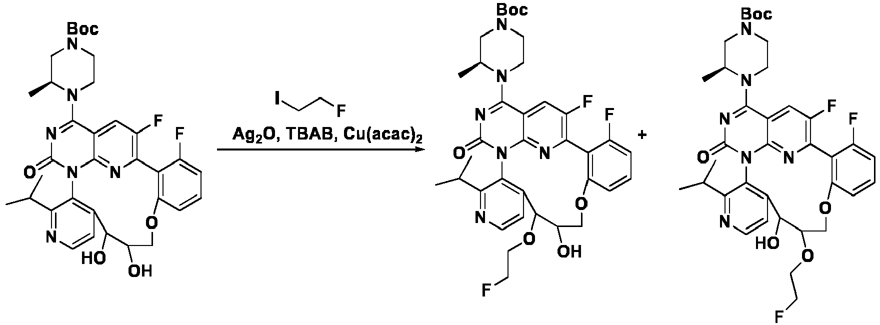

Tert-butyl (3*S*-4-(2⁶,3⁶-difluoro-6,7-dihydroxy-1²-isopropyl-2²-oxo-2¹,2²-dihydro-4-oxa-2(1,7)-pyrido[2,3-*d*]pyrimidina-1(3,4)-pyridina-3(1,2)-benzocycloheptane-2⁴-yl)-3-methylpiperazine-1-carboxylate (100 mg, 0.15 mmol) was dissolved in acetonitrile (2 mL). Silver oxide (23 mg, 0.1 mmol), tetrabutylammonium bromide (16 mg, 0.05 mmol), copper acetylacetonate (13 mg, 0.05 mmol) and 1-fluoro-2-iodoethane (52 mg, 0.3 mmol) were added in sequence. The mixture was stirred for 24 hrs at 60°C. The reaction solution was diluted with ethyl acetate and filtered. The filtrate was concentrated under reduced pressure. The resultant was separated by reversed-phase chromatographic columns to obtain tert-butyl (3*S*)-4-(2⁶,3⁶-difluoro-7-(2-fluoroethoxy)-6-hydroxy-1²-isopropyl-2²-oxo-2¹,2²-dihydro-4-oxa-2(1,7)-pyrido[2,3-*d*]pyrimidina-1(3,4)-pyridina-3(1,2)-benzocycloheptane-2⁴-yl)-3-methylpiperazine-1-carboxylate (41 mg, yield: 39%), ESI-MS 711 [M+1] ⁺. At the same time, tert-butyl (3S)-4-(2⁶,3⁶-difluoro-6-(2-fluoroethoxy)-7-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (35 mg, yield: 33%) was obtained. ESI-MS 711 [M+1]⁺.

**Intermediate D2-D10 can be prepared by selecting corresponding starting materials according to the complete or partial synthesis method for intermediate D1:**

| **Intermedi ate No.** | **Structural Formula** | **Chemical Name** | **ESI-MS: [M+1]⁺** |
|---|---|---|---|
| **D2** | 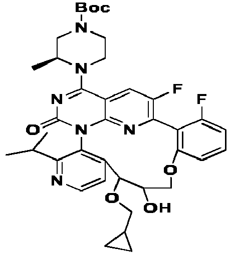 | Tert-butyl (3S)-4-(7-(cyclopropylmethoxy)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 719 |
| **D3** | 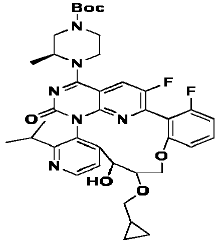 | Tert-butyl (3S)-4-(6-(cyclopropylmethoxy)-2⁶,3⁶-difluoro-7-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 719 |
| **D4** | 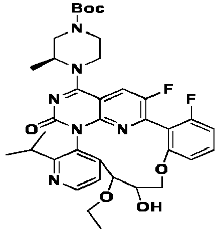 | Tert-butyl (3S)-4-(7-ethoxy-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 693 |
| **D5** | 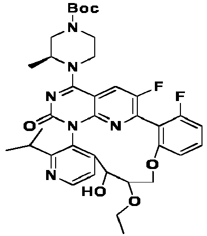 | Tert-butyl (3S)-4-(6-ethoxy-2⁶,3⁶-difluoro-7-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 693 |
| **D6** | 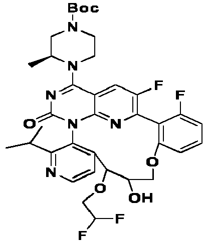 | Tert-butyl (3S)-4-(7-(2,2-difluoroethoxy)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 729 |
| **D7** | 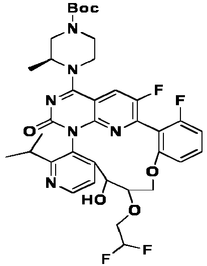 | Tert-butyl (3S)-4-(6-(2,2-difluoroethoxy)-2⁶,3⁶-difluoro-7-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 729 |
| **D8** | 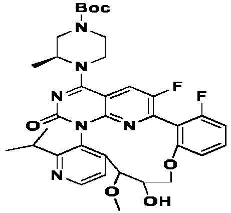 | Tert-butyl (3S)-4-(2⁶,3⁶-difluoro-6-hydroxy-1²-isopropyl-7-methoxy-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 679 |
| **D9** | 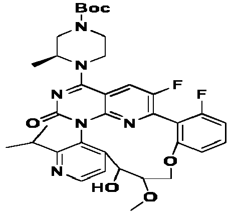 | Tert-butyl (3S)-4-(2⁶,3⁶-difluoro-7-hydroxy-1²-isopropyl-6-methoxy-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 679 |
| **D10** | 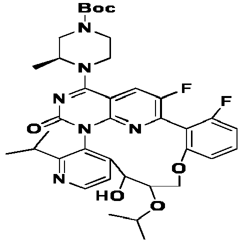 | Tert-butyl (3S)-4-(2⁶,3⁶-difluoro-7-hydroxy-6-isopropoxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate | 707 |

### II. Preparation of examples

### Example 1: Preparation of 2⁴-((S)-4-acryloyl-2-methylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one

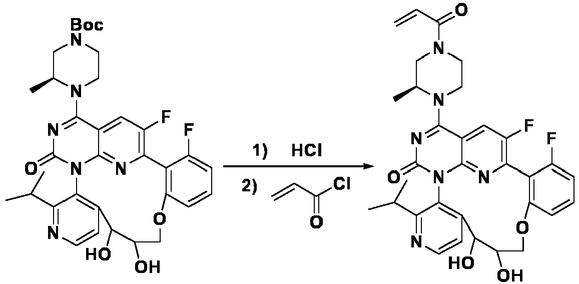

### Step 1: Synthesis of 2⁴-((S)-4-acryloyl-2-methylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one

Tert-butyl (3S)-4-(2⁶,3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (100 mg, 0.15 mmol) was dissolved in hydrogen chloride solution (2M in 1,4-dioxane) (20 mL). The mixture was stirred at room temperature to react for 1.0 hr, and the reaction was stopped. The mixture was concentrated under reduced pressure to remove the solvent. The residue was dissolved in dichloromethane (10 mL), added with diisopropylethylamine (0.5 mL), and cooled to -78°C under the protection of nitrogen. Acryloyl chlorine (14.0 mg, 0.15 mmol) was added. The reaction was conducted for 0.5 h, and then stopped. Dichloromethane (20 mL) was added for dilution. The mixture was washed with saline water (15mL * 2), dried over anhydrous sodium sulfate, and concentrated under reduced pressure to remove the solvent. The residue was separated by flash silicagel columns and preparative HPLC to obtain 2⁴-((S)-4-acryloyl-2-methylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one (89mg, yield: 95.8%). ESI-MS 619[M+1]⁺.

¹H NMR (400 MHz, DMSO-*d*₆) δ 8.50 (d, *J* = 5.2 Hz, 1H), 8.47 - 8.17 (m, 1H), 8.02 (dd, *J* = 4.8, 3.2 Hz, 1H), 7.49 (q, *J* = 8.4 Hz, 1H), 7.15 (d, *J* = 8.4 Hz, 1H), 7.01 (t, *J* = 8.8 Hz, 1H), 6.92 - 6.78 (m, 1H), 6.21 (d, *J* = 16.8 Hz, 1H), 5.77 (dd, *J* = 10.8, 2.4 Hz, 1H), 5.72 (t, *J* = 4.8 Hz, 1H), 5.20 - 4.71 (m, 2H), 4.57 - 4.39 (m, 2H), 4.39 - 4.11 (m, 2H), 4.11 - 4.01 (m, 2H), 4.01 - 3.81 (m, 1H), 3.75 - 3.50 (m, 2H), 3.01 - 2.84 (m, 1H), 1.44 (br, 2H), 1.26 (d, *J* = 6.2 Hz, 2H), 1.15 (d, *J* = 6.7Hz, 3H), 0.85 (d, *J* = 6.7 Hz, 3H).

### Examples 1-R and 1-S are separated by SFC on the basis of the preparation in Example 1:

| **Example No.** | **Structural Formula/Chemical Name** | **MS m/z [M+1]⁺ / ¹H NMR(400 MHz)** |
|---|---|---|
| **1-R** | 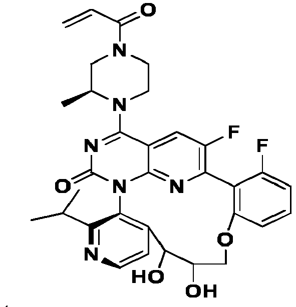 (R)-2⁴-((S)-4-acryloyl-2-methylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 619[M+1]⁺. |
| | | ¹H NMR (400 MHz, CDCl₃) δ 8.64 (d, *J* = 5.2 Hz, 1H), 8.04 (d, *J =* 5.2 Hz, 1H), 7.77 (d, *J* = 8.4 Hz, 1H), 7.38 (dd, *J =* 15.2,7.6 Hz, 1H), 6.88 (d, *J* = 8.4 Hz, 1H), 6.83 (t, *J =* 8.8 Hz, 1H), 6.71 - 6.51 (m, 1H), 6.42 (d, *J =* 16.8 Hz, 1H), 5.83 (d, *J =* 10.0 Hz, 1H), 4.99 - 4.82 (m, 1H), 4.82 - 4.69 (m, 1H), 4.66 - 4.47 (m, 1H), 4.56 (dd, *J*=8.4, 4.8Hz, 1H), 4.42 (d, *J* = 10.1 Hz, 1H), 4.24 (d, *J =* 3.4 Hz, 1H), 4.10-3.85(m, 1H), 3.73 (dd, *J* = 9.6, 7.6Hz, 1H), 3.64 - 3.53 (m, 1H), 3.49 (s, 2H), 3.44 - 3.33 (m, 1H), 3.26 - 3.17 (m, 1H), 2.95 (hept, *J* = 6.4Hz, 1H), 1.57 (d, *J =* 6.2 Hz, 3H), 1.26 (d, *J =* 6.8 Hz, 3H), 1.07 (d, *J* = 6.8 Hz, 3H). |
| **1-S** | 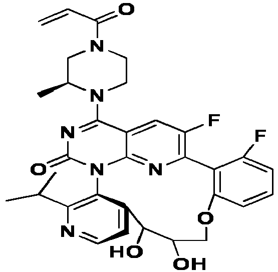 (S)-2⁴-((S)-4-acryloyl-2-methylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 619[M+1]⁺. |
| | | ¹H NMR (400 MHz, CDCl₃) δ 8.63 (d, *J* = 5.0 Hz, 1H), 8.04 (d, *J* = 5.0 Hz, 1H), 7.81 (d, *J* = 8.3 Hz, 1H), 7.39 (dd, *J =* 15.6, 8.0 Hz, 1H), 6.90 (d, *J* = 8.3 Hz, 1H), 6.83 (t, *J* = 8.7 Hz, 1H), 6.68 - 6.54 (m, 1H), 6.42 (d, *J =* 16.7 Hz, 1H), 5.83 (dd, *J* = 10.5, 1.7 Hz, 1H), 5.59 - 5.15 (m, 1H), 4.90 - 4.61 (m, 1H), 4.57 (dd, *J* = 8.2, 4.5 Hz, 1H), 4.43 (dt, *J* = 8.9, 4.2 Hz, 1H), 4.26 (d, *J* = 3.6Hz, 1H), 4.24-4.17 (m, 1H), 4.16 - 3.80 (m, 2H), 3.70 (dd, *J* = 10.6, 8.2 Hz, 2H), 3.48 (s, 1H), 3.43 - 3.33 (m, 1H), 3.30 - 3.19 (m, 1H), 2.95 (hept, *J* = 6.4Hz, 1H),1.37 (d, *J =* 6.7 Hz, 3H), 1.28 (d, *J* = 6.8 Hz, 3H), 1.06 (d, *J* = 6.8 Hz, 3H). |

### Examples 2-3 can be prepared by selecting corresponding starting materials according to the complete or partial synthesis method of Example 1:

| **Example No.** | **Structural Formula/Chemical Name** | **MS m/z [M+1]⁺ / ¹H NMR(400 MHz)** |
|---|---|---|
| **Example 2** | 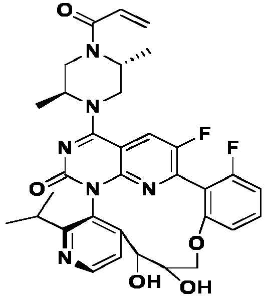 (R)-2⁴-((2S,5R)-4-acryloyl-2,5-dimethylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 633[M+1]⁺. |
| | | ¹H NMR (400 MHz, DMSO-*d*₆) δ 8.49 (d, *J* = 5.0 Hz, 1H), 8.13 (dd, *J* = 14.2, 9.1 Hz, 1H), 8.02 (d, *J* = 5.0 Hz, 1H), 7.54 - 7.45 (m, 1H), 7.17 (d, *J* = 8.4 Hz, 1H), 7.01 (t, *J* = 8.8 Hz, 1H), 6.93 - 6.75 (m, 1H), 6.24 - 6.14 (m, 1H), 5.81 - 5.67 (m, 2H), 4.65 - 4.70 (m, 3H), 4.69 - 4.17 (m, 3H), 4.13 - 4.00 (m, 2H), 3.90 - 3.64 (m, 1H), 3.62 - 3.52 (m, 1H), 3.43 - 3.33 (m, 1H), 2.97 - 2.87 (m, 1H), 1.45 (t, *J* = 6.2 Hz, 3H), 1.33 (dd, *J* = 9.5, 6.8 Hz, 3H), 1.13 (dd, *J* = 6.8Hz, 3H), 0.90 (d, *J* = 6.7 Hz, 3H). |
| **Example 3** | 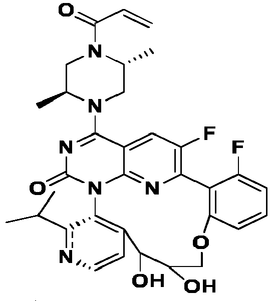 (S)-2⁴-((2S,5R)-4-acryloyl-2,5-dimethylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 633[M+1]⁺. |
| | | ¹H NMR (400 MHz, DMSO-*d*₆) δ 8.53 - 8.43 (m, 2H), 8.04 (d, *J* = 5.0 Hz, 1H), 7.54 - 7.45 (m, 1H), 7.18 (d, *J* = 8.4 Hz, 1H), 7.02 (t, *J* = 8.8 Hz, 1H), 6.90 - 6.78 (m, 1H), 6.19 (dd, *J* = 16.7, 2.4 Hz, 1H), 5.81 - 5.70 (m, 2H), 5.04 - 4.86 (m, 2H), 4.78 - 4.40 (m, 2H), 4.20 - 3.80 (m, 6H), 3.44 - 3.34 (m, 1H), 2.87 - 2.82 (m, 1H), 1.33 - 1.06 (m, 9H), 0.84 (d, *J* = 6.8 Hz, 3H). |

### Example 4: Preparation of 2⁴-((S)-4-acryloyl-2-methylpiperazin-1-yl)-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²,7-dione

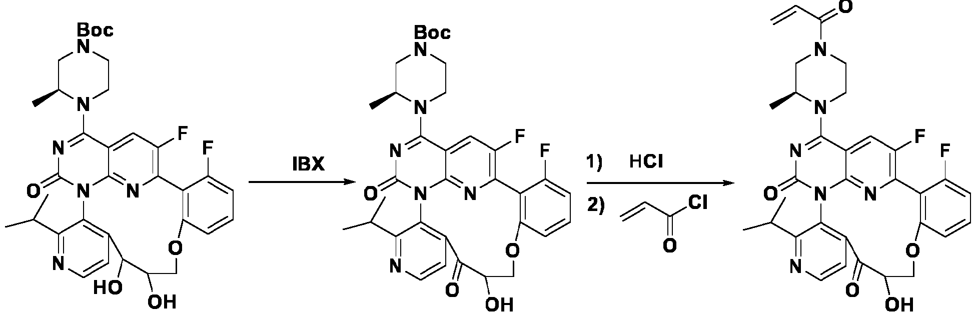

### Step 1: Synthesis of tert-butyl (3S)-4-(2⁶,3⁶-difluoro-6-hydroxy-1²-isopropyl-2²,7-dioxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate

Tert-butyl (3S)-4-(2⁶,3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (10 mg, 0.015 mmol) was dissolved in dimethyl sulfoxide (3 mL), and an IBX oxidant (21 mg, 0.075 mmol) was added. The mixture was stirred for 8 hrs at 40°C, and filtered to remove solids. The filtrate was diluted with ethyl acetate, washed with saline water, and concentrated under reduced pressure to remove the solvent. The residue was separated by reversed-phase chromatographic columns to obtain tert-butyl (3S)-4-(2⁶,3⁶-difluoro-6-hydroxy-1²-isopropyl-2²,7-dioxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (8.0mg, yield: 60%). ESI-MS: 663 [M+1]⁺.

### Step 2: Synthesis of 2⁴-((S)-4-acryloyl-2-methylpiperazin-1-yl)-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²,7-dione

Tert-butyl (3S)-4-(2⁶,3⁶-difluoro-6-hydroxy-1²-isopropyl-2²,7-dioxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (8.0 mg, 0.012 mmol was dissolved in hydrogen chloride solution (2M in 1,4-dioxane) (5 mL), and stirred at room temperature to react for 1.0 hr. The reaction was stopped, and the mixture was concentrated under reduced pressure to remove the solvent. The residue was dissolved in dichloromethane (5 mL), and diisopropylethylamine (0.2 mL) was added. The mixture was cooled to -78°C under the protection of nitrogen. Acryloyl chlorine (1.1 mg, 0.012 mmol) was added. The reaction was conducted for 0.5 hr, and then quenched by adding 1 mL of water. Dichloromethane (15 mL) was added for dilution, and the resultant was washed with saline water (15 mL * 2), dried over anhydrous sodium sulfate, and concentrated under reduced pressure to remove the solvent. The residue was separated by the preparative HPLC to obtain 2⁴-((S)-4-acryloyl-2-methylpiperazin-1-yl)-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²,7-dione (0.9mg, yield: 11.6%). ESI-MS 617[M+1]⁺.

¹H NMR (400 MHz, DMSO-*d*₆) δ 8.59 (d, *J* = 5.1 Hz, 1H), 8.50 - 8.20 (m, 1H), 7.57 - 7.45 (m, 1H), 7.06 (d, *J* = 4.0 Hz, 1H),7.03 (d, *J* = 4.4 Hz, 1H), 6.98 (d, *J* = 8.8 Hz, 1H), 6.96 - 6.80 (m, 1H), 6.21 (d, *J* = 16.8 Hz, 1H), 6.03 - 5.84 (m, 1H), 5.77 (d, *J* = 10.7 Hz, 1H), 5.17 (br, 1H), 4.94 (d, *J* = 8.9 Hz, 1H), 4.85 - 4.76 (m, 2H), 4.60 (dd, *J* = 16.1, 5.2 Hz, 1H), 4.55 - 4.30 (m, 1H), 4.30 - 4.11 (m, 2H), 3.70 - 3.51 (m, 2H), 3.00 - 2.94 (m, 1H), 1.44 (d, *J* = 6.4 Hz, 1H), 1.25 (d, *J* = 6.5 Hz, 2H), 1.17 (d, *J* = 6.1 Hz, 3H), 0.90 (d, *J* = 6.6 Hz, 3H).

### Example 5: Preparation of 2⁴-((S)-4-acryloyl-2-methylpiperazin-1-yl)-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one

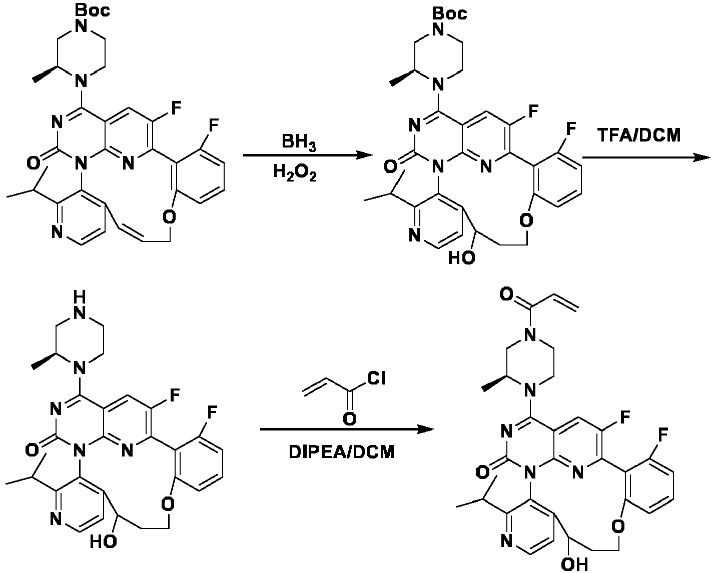

### Step 1: Synthesis of tert-butyl(3S)-4-(2⁶,3⁶-difluoro-7-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate

Under the condition of an ice bath, borane tetrahydrofuran (0.95 mL) was added to tert-butyl (S,Z)-4-(2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6-ene-2⁴-yl)-3-methylpiperazine-1-carboxylate (100 mg, 0.16 mmol) in tetrahydrofuran solution (3 mL) to react for 4 hrs at the current temperature. Then, the mixture was restored to the room temperature, and stirred at room temperature overnight. After the reactive starting materials were detected to basically disappear, the resultant was cooled to 0 °C again. 0.1 mL of saturated sodium bicarbonate in water and 0.1 mL of hydrogen peroxide were added to the above reaction solution, which was then stirred for 4 hrs at room temperature. The reaction was quenched with ethyl acetate (30 mL) and sodium hydrogen sulfite in water (20 mL). The organic phase was separated, dried over anhydrous sodium sulfate, and then concentrated under reduced pressure to remove the solvent. The residue was separated by flash silicagel columns to obtain tert-butyl (3S)-4-(2⁶,3⁶-difluoro-7-hydroxy-1²-isopropyl-2²-oxo-2¹,2²-dihydro-4-oxa-2(1,7)-pyrido[2,3 - d]pyrimidina-1(3,4)-pyridina-3(1,2)-benzocycloheptane-2⁴-yl)-3-methylpiperazine-1-carboxylate (50 mg, yield: 21%). ESI-MS 649 [M+1]⁺.

### Step 2: Synthesis of 2⁶,3⁶-difluoro-7-hydroxy-1²-isopropyl-2⁴-((S)-2-methylpiperazin-1-yl)-2¹,2²-dihydro-4-oxa-2(1,7)-pyrido[2,3-d]pyrimidina-1(3,4)-pyridina-3(1,2)-benzocycloheptane-2²-one

Tert-butyl (3S)-4-(2⁶,3⁶-difluoro-7-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (25.0 mg, 0.04 mmol) was dissolved in 2 mL of dichloromethane and 1 mL of trifluoroacetic acid, and stirred at room temperature to react for 1.5 hrs. The reaction was stopped, and the resultant was concentrated under reduced pressure to remove the solvent to obtain crude 2⁶,3⁶-difluoro-7-hydroxy-1²-isopropyl-2⁴-((S)-2-methylpiperazin-1-yl)-2¹,2²-dihydro-4-oxa-2(1,7)-pyrido[2,3-d]pyrimidina-1(3,4)-pyridina-3(1,2)-benzocycloheptane-2²-one (21mg, yield: 68%), which was directly used in the next step. ESI-MS 549 [M+1]⁺.

### Step 3: Synthesis of 2⁴-((S)-4-acryloyl-2-methylpiperazin-1-yl)-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one

2⁶,3⁶-difluoro-6-hydroxy-1²-isopropyl-2⁴-((S)-2-methylpiperazin-1-yl)-2¹,2²-dihydro-4-oxa-2(1,7)-pyrido[2,3-d]pyrimidina-1(3,4)-pyridina-3(1,2)-benzocycloheptane-2²-one (22 mg, 0.04 mmol) was dissolved in dichloromethane (5 mL). N,N-diisopropylethylamine (0.5 mL) was added. The mixture was cooled to -20°C, and acryloyl chlorine (3.6 mg, 0.04 mmol) was added. During the reaction, the mixture was naturally warmed up to room temperature and stirred to react for 1.5 hrs. The reaction was stopped, and dichloromethane (30 mL) was added for dilution. The resultant was washed with saline water (20 mL * 2), and the organic phase was separated, then dried over anhydrous sodium sulfate, and concentrated under reduced pressure to remove the solvent. The residue was separated by flash silicagel columns and reversed-phase chromatographic columns to obtain 2⁴-((S)-4-acryloyl-2-methylpiperazin-1-yl)-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one (11.2 mg, yield: 44%). ESI-MS 603 [M+1]⁺.

¹H NMR (400 MHz, DMSO-*d*₆) δ 8.42 (d, *J* = 5.4 Hz, 1H), 8.42 - 8.14(m, 1H), 7.92 (d, *J* = 5.1 Hz, 1H), 7.52 - 7.47 (m, 1H), 7.14 (d, *J* = 8.3 Hz, 1H), 6.99 (t, *J* = 8.8 Hz, 1H), 6.95 - 6.80 (m, 1H), 6.21 (d, *J* = 16.7 Hz, 1H), 5.77 (dd, *J* = 10.4, 2.5 Hz, 1H), 5.70 (d, *J* = 3.8 Hz, 1H), 5.20 - 4.70 (m, 1H), 4.61 - 3.81 (m, 5H), 3.73 - 3.45 (m, 1H), 3.05 - 2.85 (m, 3H), 2.70 - 2.57 (m, 1H), 2.34 - 2.25 (m, 2H), 1.47 - 1.18 (m, 3H), 1.12 (d, *J* = 6.8 Hz, 3H), 0.89 (d, *J* = 6.8 Hz, 3H).

### Example 6: Preparation of 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl propionate

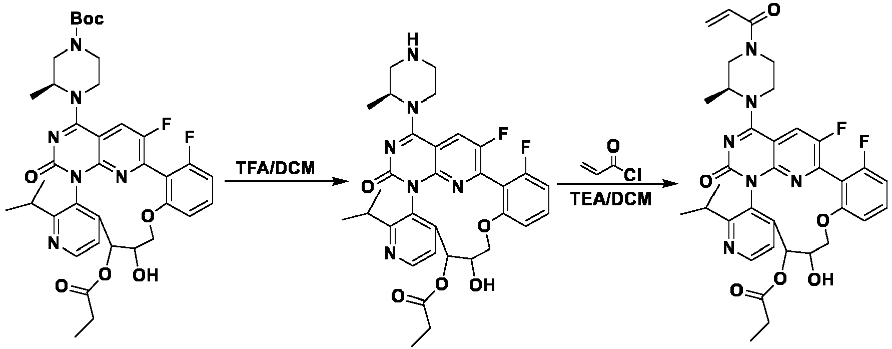

### Step 1: Synthesis of 2⁶,3⁶-difluoro-6-hydroxy-1²-isopropyl-2⁴-((S)-2-methylpiperazin-1-yl)-2²-oxo-2¹,2²-dihydro-4-oxa-2(1,7)-pyrido[2,3-d]pyrimidina-1(3,4)-pyridina-3(1,2)-benzocycloheptane-7-yl propionate

Tert-butyl (3S)-4-(2⁶,3⁶-difluoro-6-hydroxy-1²-isopropyl-2²-oxo-7-(propionyl(propionyloxy))-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (43 mg, 0.054 mmol) was dissolved in dichloromethane (4 mL), and added with trifluoroacetic acid (1.0 mL, 1.76 mmol) to react for 2 hrs over stirring at room temperature. After the reaction was ended, the resultant was concentrated to obtain a product 2⁶,3⁶-difluoro-6-hydroxy-1²-isopropyl-2⁴-((S)-2-methylpiperazin-1-yl)-2²-oxo-2¹,2²-dihydro-4-oxa-2(1,7)-pyrido[2,3-d]pyrimidina-1(3,4)-pyridina-3(1,2)-benzocycloheptane-7-yl propionate (35 mg, yield: 100%). ESI-MS 621[M+1]⁺.

### Step 2: Synthesis of 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido [2,3-d]pyrimidina-1(3,4)-pyridina-3(1,2)-benzocycloheptane-7-yl propionate

2⁶,3⁶-difluoro-6-hydroxy-1²-isopropyl-2⁴-((S)-2-methylpiperazin-1-yl)-2²-oxo-2¹,2²-dihydro-4-oxa-2(1,7)-pyrido[2,3-d]pyrimidina-1(3,4)-pyridina-3(1,2)-benzocycloheptane-7-yl propionate (35 mg, 0.054 mmol) was dissolved in dichloromethane (5 mL), and triethylamine (0.5 mL,3.5 mmol) was added at temperature. The mixture was cooled to -78°C, and then dropwise added with acryloyl chlorine (0.004 mL, 0.051 mmol) in dichloromethane solution. The reaction was conducted for half an hour over stirring at -78°C. After cooling, dichloromethane was added for dilution, and then, the mixture was washed twice with ice water and once with saline water. The resultant was dried and concentrated, and the concentrate was separated by HPLC [5~95% acetonitrile: 0.01mM NH₄HCO₃/H₂O] to obtain 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl propionate (10.7 mg, yield: 29%). ESI-MS 675[M+1]⁺.

¹H NMR (400 MHz, DMSO-*d*₆) δ 8.64 - 8.42 (m, 1H), 8.29 - 8.20 (m, 1H), 7.60 - 7.44 (m, 1H), 7.23 - 7.12 (m, 1H), 7.10 - 6.95 (m, 1H), 6.90 - 6.80 (m, 1H), 6.29 - 6.15 (m, 1H), 6.09 (d, *J* = 5.1 Hz, 1H), 5.77 (d, *J* = 10.7 Hz, 1H), 5.57 (s, 1H), 5.19 - 4.82 (m, 1H), 4.66 - 4.62 (m, 1H), 4.52 - 4.33 (m, 1H), 4.32 - 3.41 (m, 7H), 3.07 - 2.86 (m, 1H), 2.33 (s, 1H), 2.19 - 2.10 (m, 2H), 1.43 (s, 1.5H), 1.22 (s, 1.5H), 1.15 (t, *J* = 5.9 Hz, 3H), 1.00 - 0.80 (m, 6H).

**Examples 7-17 can be prepared by selecting corresponding starting materials according to the complete or partial synthesis method of Example 6:**

| **Example No.** | **Structural Formula/Chemical Name** | **MS m/z [M+1]⁺ / ¹H NMR(400 MHz)** |
|---|---|---|
| **Example 7** | 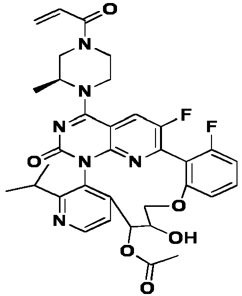 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl acetate | ESI-MS 661 [M+1]⁺. |
| | | ¹H NMR (400 MHz, CDCl₃) δ 8.65 (d, *J* = 5.0 Hz, 1H), 8.14 (t, *J =* 5.1 Hz, 1H), 7.82 (dd, *J =* 16.9, 8.2 Hz, 1H), 7.38 (dd, *J*=8.4,6.8 Hz, 1H), 6.92 - 6.80 (m, 2H), 6.70 - 6.52 (m, 1H), 6.40 (d, *J* = 16.6 Hz, 1H), 5.85 - 5.74 (m, 2H), 4.85 - 4.67 (m, 1H), 4.62 - 4.40 (m, 2H), 4.39-4.3(m, 1H),4.26 - 4.03 (m, 1H), 4.02 - 3.85 (m, 1H), 3.78 (t, *J* = 9.6 Hz, 2H), 3.57 - 3.16 (m, 2H), 2.98 - 2.89 (m, 1H), 2.81 - 2.70 (m, 1H), 2.02 (s, *J* = 1.8 Hz, 3H), 1.52 (d, *J*=6.7 Hz, 1H), 1.39 (d, *J* = 6.7 Hz, 2H), 1.31 (d, *J* = 6.8 Hz, 3H), 1.01 (d, *J* = 7.0 Hz, 3H). |
| **Example 8** | 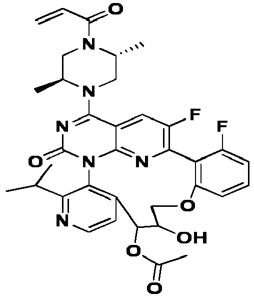 2⁴-((2S,5R)-4-acryloyl-2,5-dimethylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl acetate | ESI-MS 675 [M+1]⁺. |
| | | ¹H NMR (400MHz, DMSO-*d*₆) δ = 8.59 (t, *J* = 4.8 Hz, 1H), 8.49 (br d, *J* = 8.0 Hz, 1H), 8.25 - 8.19 (m, 1H), 8.17 - 8.09 (m, 1H), 7.57 - 7.46 (m, 1H), 7.20 (d, *J* = 8.4 Hz, 1H), 7.03 (t, *J* = 8.8 Hz, 1H), 6.93 - 6.73 (m, 1H), 6.24 - 6.10 (m, 2H), 5.76 (br d, *J* = 9.4 Hz, 1H), 5.59 (t, *J* = 3.1 Hz, 1H), 4.97 - 4.37 (m, 4H), 4.23 - 3.77 (m, 4H), 3.02 - 2.83 (m, 1H), 1.86 (d, *J* = 4.4 Hz, 3H), 1.48 (br t, *J* = 6.5 Hz, 1H), 1.36 - 1.22 (m, 3H), 1.20 - 104 (m, 5H), 0.98 - 0.90 (m, 1H), 0.85 (d, *J* = 6.9 Hz, 2H). |
| **Example 9** | 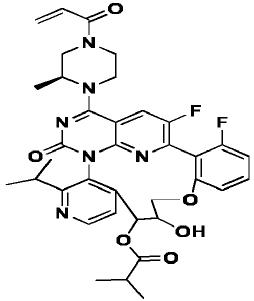 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl isobutyrate | ESI-MS 689 [M+1]⁺. |
| | | ¹H NMR (400 MHz, DMSO-*d*₆) δ 8.58 - 8.47 (m, 1H), 8.35 - 8.17 (m, 1H), 7.50 (q, *J* = 7.8 Hz, 1H), 7.17 (dd, *J* = 8.5, 4.1 Hz, 1H), 7.01 (t, *J* = 8.8 Hz, 1H), 6.86 (dd, *J* = 16.3, 10.6 Hz, 1H), 6.21 (d, *J* = 16.9 Hz, 1H), 6.11 (d, *J* = 5.3 Hz, 1H), 5.76 (d, *J* = 10.5 Hz, 1H), 5.62 - 5.43 (m, 1H), 5.18 - 4.62 (m, 2H), 4.61 - 3.85 (m, 6H), 3.66 - 3.45 (m, 4H), 3.00 - 2.86 (m, 1H), 2.35 (dd, *J* = 13.8, 6.9 Hz, 1H), 1.43 (s, 1.5H), 1.23 (s,1.5H), 1.18 - 1.13 (m, 3H), 1.06 (dd, *J* = 12.3, 6.8 Hz, 1H), 0.98 (dd, *J* = 14.8, 7.0 Hz, 5H), 0.88 (dd, *J* = 7.0, 3.1 Hz, 3H). |
| **Example 10** | 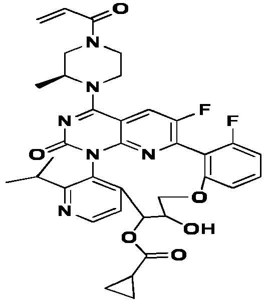 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl cyclopropanecarboxylate | ESI-MS 687 [M+1]⁺. |
| | | ¹H NMR (400 MHz, DMSO-*d*₆) δ 8.58 (d, *J* = 5.1 Hz, 1H), 8.46 (d, *J* = 8.9 Hz, 0.5H), 8.27 (d, *J* = 8.9 Hz, 0.5H), 8.24 (d, *J* = 5.1 Hz, 1H), 7.50 (q, *J* = 8.0 Hz, 1H), 7.18 (dd, *J* = 8.4, 3.5 Hz, 1H), 7.01 (t, *J =* 8.8 Hz, 1H), 6.87 (dd, *J =* 16.4, 10.6 Hz, 1H), 6.27 - 6.12 (m, 2H), 5.77 (dd, *J =* 10.5, 2.2 Hz, 1H), 5.61 (dd, *J* = 7.2, 3.2 Hz, 1H), 5.10 - 4.70 (m, 1H), 4.56 (dd, *J* = 8.5, 4.9 Hz, 1H), 4.51 - 4.20 (m, 2H), 4.10 (d, *J* = 11.6 Hz, 2H), 4.03 - 3.52 (m, 3H), 3.47 (dd, *J* = 11.0, 8.5 Hz, 1H), 3.20 - 2.90 (m, 2H), 1.51 - 1.41 (m, 1H), 1.44 - 1.41 (m, 1.5H), 1.24 - 1.20 (m, 1.5H), 1.15 (dd, *J* = 6.7, 4.3 Hz, 3H), 0.88 (dd, *J* = 6.7, 3.3 Hz, 3H), 0.80 - 0.69 (m, 3H). |
| **Example 11** | 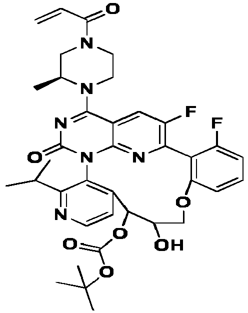 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl tert-butylcarbonate | ESI-MS 719[M+1]⁺. |
| | | ¹H NMR (400 MHz, CDCl₃) δ 8.65 (d, *J* = 4 Hz, 1H), 8.18 (s, 1H),7.89-7.83 (m, 1H), 7.42-7.36 (m, 1H), 6.90 - 6.83 (m, 2H), 6.71 - 6.52 (m, 1H), 6.44 - 6.38 (m, 1H),5.83 (d, *J* = 12 Hz, 1H), 5.55 (s, 1H), 5.26 - 4.49 (m, 3H), 4.41 - 4.39 (m, 1H), 4.21 - 3.38 (m, 5H), 3.23 - 2.85 (m, 3H), 1.58 - 1.39(m, 12H), 1.32 (d, *J* = 4 Hz, 3H), 1.03 (d, *J* = 4 Hz, 3H). |
| **Example 12** | 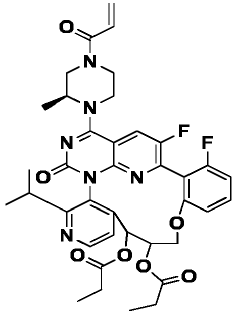 2⁴-((S)-4-acryloyl-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3 (1,2)-benzocycloheptane-6,7-diyl dipropionate | ESI-MS 731[M+1]⁺. |
| | | ¹H NMR (400 MHz, DMSO-*d₆*) δ 8.68 (d, *J* = 5.0 Hz, 1H), 8.54 (d, *J* = 9.3 Hz, 0.5H), 8.39 - 8.26 (m, 0.5H), 7.90 (d, *J* = 5.2 Hz, 1H), 7.52 (q, *J* = 8.0 Hz, 1H), 7.18 (dd, *J* = 8.4, 4.2 Hz, 1H), 7.06 (t, *J* = 8.9 Hz, 1H), 6.86 (dd, *J* = 16.6, 10.7 Hz, 1H), 6.21 (d, *J* = 16.8 Hz, 1H), 5.85 - 5.72 (m, 2H), 5.46 - 5.31 (m, 1H), 5.06 (s, 1H), 4.79 - 4.62 (m, 2H), 4.52 - 3.84 (m, 4H), 3.68 - 3.58 (m, 3H), 3.06 - 2.87 (m, 2H), 2.06 (q, *J* = 7.5 Hz, 2H), 1.50 - 1.37 (m, 1.5H), 1.26 - 1.19 (m, 1.5H), 1.17 - 1.09 (m, 6H), 0.95 - 0.82 (m, 6H). |
| **Example 13** | 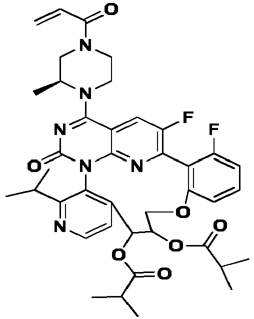 2⁴-((S)-4-acryloyl-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3 (1,2)-benzocycloheptane-6,7-diyl di(2-methylpropionate) | ESI-MS 759[M+1]⁺. |
| | | ¹H NMR (400 MHz, DMSO-*d*₆) δ 8.70 (d, *J* = 4.8 Hz, 1H), 8.59 - 8.52 (m, 1H), 8.35 - 8.23 (m, 1H), 7.91 (d, *J* = 5.2 Hz, 1H), 7.55 - 7.49 (m, 1H), 7.20 - 7.17 (m, 1H), 7.04 (t, *J* = 7.6 Hz, 1H), 6.89 - 6.80 (m, 1H), 6.20 (d, *J* = 16.4 Hz, 1H), 5.80 - 5.75 (m, 2H), 5.57 - 5.05 (m, 2H), 4.67 - 4.63 (m, 2H), 4.47 - 4.11 (m, 4H), 3.72 - 3.59 (m, 2H), 2.98 - 2.93 (m, 2H), 2.79 - 2.67 (m, 1H), 2.28 - 2.24 (m, 1H), 1.45 - 1.43 (m, 2H), 1.29 - 1.14 (m, 10H), 1.08 - 0.89 (m, 7H). |
| **Example 14** | 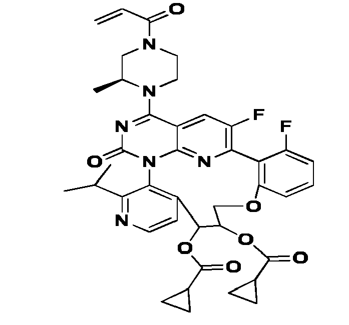 2⁴-((S)-4-acryloyl-2-metnylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3 (1,2)-benzocycloheptane-6,7-diyl dicyclopropanecarboxylate | ESI-MS 755 [M+1]⁺. |
| | | ¹H NMR (400 MHz, DMSO-*d*₆) δ 8.69 (d, *J* = 5.1 Hz, 1H), 8.51 (d, *J* = 9.3 Hz, 0.5H), 8.29 (q, *J* = 9.3, 7.3 Hz, 0.5H), 7.92 (d, *J* = 5.1 Hz, 1H), 7.52 (q, *J* = 7.9 Hz, 1H), 7.20 (dd, *J* = 8.4, 3.9 Hz, 1H), 7.05 (t, *J* = 8.8 Hz, 1H), 6.86 (dd, *J* = 16.5, 10.8 Hz, 1H), 6.21 (d, *J* = 17.1 Hz, 1H), 5.83 - 5.70 (m, 2H), 5.39 - 5.32 (m, 1H), 5.03 - 4.70 (m, 1H), 4.64 (dd, *J* = 8.7, 4.7 Hz, 1H), 4.49 - 3.73 (m, 4H), 3.70 - 3.62 (m, 1H), 3.59 - 3.39 (m, 1.5H), 3.24 - 3.19 (m, 0.5H), 2.95 (p, *J* = 6.9 Hz, 1H), 1.93 - 1.87 (m, 1H), 1.81 - 1.75 (m, 1H), 1.45 - 1.37 (m, 2H), 1.16 (t, *J* = 5.8 Hz, 3H), 1.07 - 0.98 (m, 6H), 0.91 - 0.89 (m, 2H), 0.80 - 0.70 (m, 4H). |
| **Example 15** | 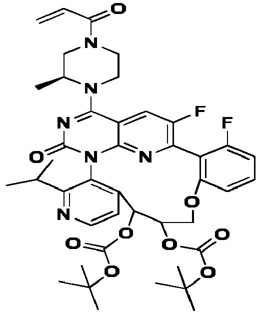 2⁴-((S)-4-acryloyl-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3 (1,2)-benzocycloheptane-6,7-diyl di-tert-butyldi(carbonate) | ESI-MS 819 [M+1]⁺. |
| | | ¹H NMR (400 MHz, MeOH-*d*₄) δ8.55 (d, *J* = 4 Hz, 1H), 8.33 - 8.15 (m, 1H), 7.85 (d, *J* = 4 Hz, 1H), 7.39 - 7.33 (m, 1H), 6.99 (d, *J* = 8 Hz, 1H), 6.84 - 6.68 (m, 2H), 6.24 (d, *J* = 16 Hz, 1H), 5.76 - 5.73 (m, 1H), 5.53 - 5.50 (m, 1H), 5.26 - 5.21 (m, 1H), 4.53 - 3.92 (m, 5H), 3.69 - 3.57 (m, 3H), 3.38 - 3.33 (m, 1H), 3.04 - 2.91(m, 1H),1.49 - 1.41(m, 12H), 1.28 (s, 9H), 1.18 (d, *J* = 8 Hz, 3H), 0.89 (d, *J* = 8 Hz, 3H). |
| **Example 16** | 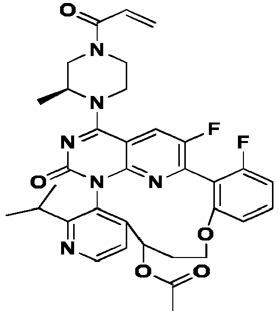 2⁴-((S)-4-acryloyl-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3 (1,2)-benzocycloheptane-7-yl acetate | ESI-MS 645 [M+1]⁺. |
| | | ¹H NMR (400 MHz, DMSO-*d*₆) δ 8.50 (d, *J* = 3.6 Hz, 1H), 8.42 (d, *J* = 6.0 Hz, 1H), 7.61 (d, *J* = 4.0 Hz, 1H), 7.53 - 7.47 (m, 1H), 7.16 (d, *J* = 8.3 Hz, 1H), 7.02 (t, *J* = 8.0 Hz, 1H), 6.90 - 6.83 (m, 1H), 6.21 (d, *J* = 17.2 Hz, 1H), 5.77 (d, *J* = 10.4 Hz, 1H), 5.15 - 5.11 (m, 2H), 4.56 - 3.82 (m, 6H), 3.72 - 3.50 (m, 2H), 3.00 - 2.85 (m, 2H), 2.80 - 2.62 (m, 1H), 2.16 (s, 3H), 1.13 (d, *J* = 6.5 Hz, 3H), 0.95 - 0.80 (m, 6H). |
| Example 17 | 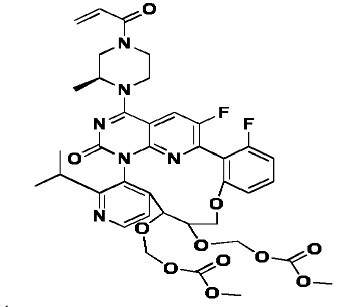 ((2⁴-((S)-4-acryloyl-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6,7-diyl)di(oxo))di(methylene) dimethyldi(carbonate) | ESI-MS 795 [M+1]⁺. |
| | | ¹H NMR (400 MHz, DMSO-*d*₆) δ 8.72 - 8.55 (m, 1H), 8.55 - 8.24 (m, 1H), 7.92 - 7.62 (m, 1H), 7.53 (p, *J* = 6.7, 5.5 Hz, 1H), 7.25 - 7.15 (m, 1H), 7.07 (dd, *J* = 11.7, 7.3 Hz, 1H), 6.93 - 6.78 (m, 1H), 6.21 (d, *J* = 16.6 Hz, 1H), 5.77 (d, *J =* 10.7 Hz, 1H), 5.64 - 5.02 (m, 3H), 5.02 - 3.87 (m, 7H), 3.84 - 3.70 (m, 3H), 3.69 - 3.43 (m, 6H), 3.28 - 3.14 (m, 1H), 3.01 - 2.91 (m, 1H), 1.50 - 1.18 (m, 4H), 1.15 (t, *J* = 6.0 Hz, 3H), 0.95 - 0.83 (m, 3H). |

### Example 18: Preparation of 2⁴-((S)-4-acryloyl-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6,7-diyl diacetate

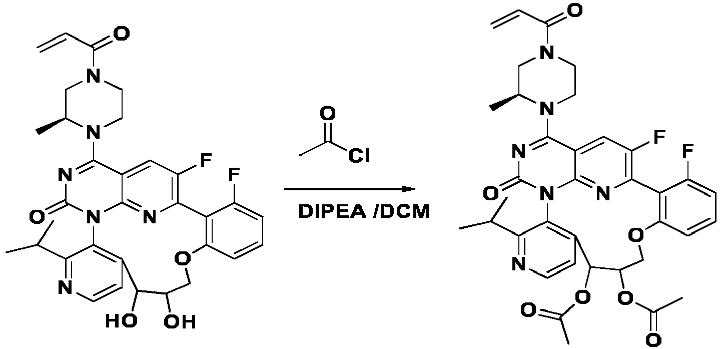

2⁴-((S)-4-acryloyl-2-methylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one (50 mg 0.08 mmol) was dissolved in DCM (15 mL). Diisopropylethylamine (31 mg, 0.24 mmol) was added. Dichloromethane-diluted acetylchloride (13 mg, 0.16 mmol) was added, and the mixture was stirred at room temperature to react for 1.5 hrs. The reaction was stopped, and 30 mL of dichloromethane was added for dilution. The resultant was washed with saline water (15 mL * 2), and the organic phase was dried over anhydrous sodium sulfate, and concentrated under reduced pressure to remove the solvent. The residue was separated by reversed-phase chromatographic columns to obtain 2⁴-((S)-4-acryloyl-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6,7-diyl diacetate (25mg, yield: 45%). ESI-MS 703[M+1]⁺.

¹H NMR (400 MHz, CDCl₃) δ 8.71 (d, *J* = 5.2 Hz, 1H), 7.90 (dd, *J* = 5.2, 3.8 Hz, 1H), 7.82 (dd, *J* = 16.5, 8.4 Hz, 1H), 7.38 (td, *J* = 8.4, 6.5 Hz, 1H), 6.90 - 6.80 (m, 2H), 6.71 - 6.52 (m, 1H), 6.40 (dd, *J* = 16.7, 2.0 Hz, 1H), 5.94 (d, *J* = 3.1 Hz, 1H), 5.81 (dd, *J* = 10.5, 2.0 Hz, 1H), 5.57 (dt, *J* = 10.8, 4.5 Hz, 1H), 4.85 - 4.51 (m, 2H), 4.43 (dt, *J =* 8.3, 4.2 Hz, 1H), 4.26 - 4.01 (m, 1H), 4.00 - 3.84 (m, 1H), 3.74 (dt, *J =* 10.8, 2.4 Hz, 2H), 3.63 - 3.17 (m, 2H), 3.00 - 2.91 (m, 1H), 2.23 (s, 3H), 1.93 (s, 3H), 1.51(d, J=6.8Hz, 1H),1.40 (d, J=6.8Hz, 2H), 1.29 (d, *J =* 6.7Hz, 3H), 1.0 (d, *J =* 6.9 Hz, 3H).

### Examples 18-R and 18-S are separated by SFC on the basis of the preparation in Example 18:

| **Exampl e No.** | **Structural Formula/Chemical Name** | **MS m/z [M+1]⁺** / **¹H NMR(400 MHz)** |
|---|---|---|
| **18**-R | 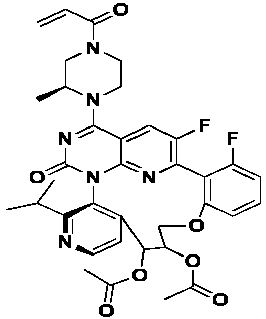 (R)-2⁴-((S)-4-acryloyl-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6,7-diyl diacetate | ESI-MS 703[M+1]⁺. |
| | | ¹H NMR (400 MHz, CDCl₃) δ 8.69 (d, *J* = 5.1 Hz, 1H), 7.90 (d, *J* = 5.1 Hz, 1H), 7.81 (d, *J* = 8.4 Hz, 1H), 7.38 (dd, *J* = 15.2, 8.0 Hz, 1H), 6.88 - 6.80 (m, 2H), 6.68 - 6.54 (m, 1H), 6.40 (d, *J* = 16 Hz, 1H), 5.93 (d, *J* = 3.8 Hz, 1H), 5.80 (dd, *J* = 10.5, 1.9 Hz, 1H), 5.57 (dt, *J* = 11.0, 4.4 Hz, 1H), 4.87 - 4.51 (m, 3H), 4.42 (dd, *J =* 8.1, 4.7 Hz, 1H), 4.05 - 3.85 (m,1H), 3.79 (dd, *J =* 11.0, 8.0 Hz, 1H), 3.61 (m, 1H), 3.45 (m, 1H), 3.32 - 3.11 (m, 1H), 3.00 - 2.87 (m, 1H), 2.23 (s, 3H), 1.92 (s, 3H), 1.55(d, *J* = 6.7 Hz, 2H), 1.32 - 1.3 (d, *J* = 6.7 Hz, 4H), 1.01 (d, *J =* 6.8 Hz, 3H). |
| **18**-S | 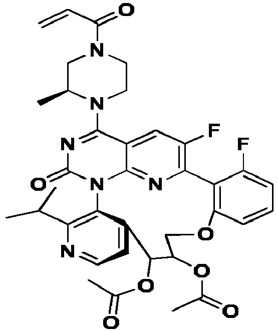 (S)-2⁴-((S)-4-acryloyl-2-methylpiperazin-1-yl)-2⁶,3⁶-difluoro-1²-isopropyl-2²-oxo-2¹,2²-dihydro-4-oxa-2(1,7)-pyrido[2,3-d]pyrimidina-1(3,4)-pyridina-3(1,2)-benzocycloheptane-6,7-diyl diacetate | ESI-MS 703[M+1]⁺. |
| | | ¹H NMR (400 MHz, CDCl₃) δ 8.70 (d, *J* = 4.9 Hz, 1H), 7.91 (d, *J* = 4.9 Hz, 1H), 7.83 (d, *J* = 8.3 Hz, 1H), 7.39 (dd, *J* = 15.6, 8.0Hz, 1H), 6.89 - 6.81 (m, 2H), 6.71 - 6.53 (m, 1H), 6.41 (d, *J* = 16.4 Hz, 1H), 6.01 - 5.89 (m, 1H), 5.81 (d, *J* = 10.4 Hz, 1H), 5.56 (dd, *J* = 10.4, 5.1 Hz, 1H), 5.31 - 4.88 (m, 1H), 4.78-4.52 (m, 1H), 4.42 (dd, *J* = 8.1, 4.6 Hz, 1H), 4.27 - 4.14 (m, 1H), 4.14 - 3.83 (m, 2H), 3.78 (t, *J* = 9.6 Hz, 2H), 3.56 - 3.34 (m, 1H), 3.01 - 2.90 (m, 2H), 2.24 (s, 3H), 1.93 (s, 3H), 1.39 (d, *J* = 6.6 Hz, 3H), 1.32 (d, *J* = 6.7 Hz, 3H), 1.0 (d, *J* = 6.6 Hz, 3H). |

### Example 19: Preparation of 2⁴-((S)-4-acryloyl-2-methylpiperazin-1-yl)-2⁶,3⁶-difluoro-1²-isopropyl-2²,6-dioxo-2¹,2²-dihydro-4-oxa-2(1,7)-pyrido[2,3-d]pyrimidina-1(3,4)-pyridina-3(1,2)-benzocycloheptane-7-yl acetate

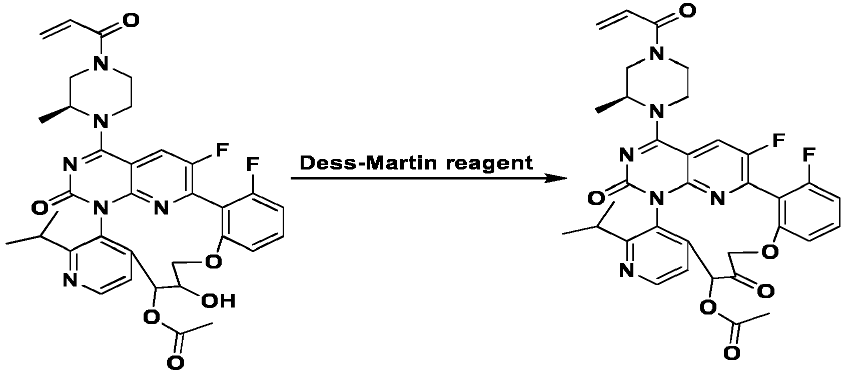

2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl acetate (50 mg, 0.08 mmol) was dissolved in DCM (15 mL). The Dess-Martin oxidant (32 mg, 0.08 mmol) was added. The mixture was stirred at room temperature to react for 2 hrs. The reaction was stopped, and the resultant was filtered with diatomite, and concentrated under reduced pressure to remove the solvent. The residue was separated by reversed-phase chromatographic columns to obtain 2⁴-((S)-4-acryloyl-2-methylpiperazin-1-yl)-2⁶,3⁶-difluoro-1²-isopropyl-2²,6-dioxo-2¹,2²-dihydro-4-oxa-2(1,7)-pyrido[2,3-d]pyrimidina-1(3,4)-pyridina-3(1,2)-benzocycloheptane-7-yl acetate (50 mg, yield: 100%). ESI-MS 659[M+1]⁺.

¹H NMR (400 MHz, CDCl₃) δ 8.68 (d, *J* = 5.0 Hz, 1H), 7.85 (d, *J* = 8.8 Hz, 1H), 7.41 (dd, *J* = 8.4, 6.4 Hz, 1H), 6.97 (d, *J* = 5.0 Hz, 1H), 6.88 (t, *J* = 8.7 Hz, 1H), 6.65 (d, *J* = 8.3 Hz, 1H), 6.42 (d, *J* = 16.7 Hz, 1H), 6.31 (d, *J* = 10.3 Hz, 1H), 5.81 (dd, *J* = 10.4, 2.0 Hz, 1H), 4.93 - 4.72 (m, 1H), 4.68 (dd, *J* = 14.7, 1.9 Hz, 1H), 4.51 (dd, *J* = 15.0, 1.6 Hz, 1H), 4.5(m, 1H), 4.28 - 3.97 (m, 1H), 3.97 - 3.82 (m, 1H), 3.82 - 3.68 (m, 1H), 3.68 - 3.55 (m, 1H), 3.55 - 3.39 (m, 1H), 3.28 - 2.93 (m, 2H), 2.05 (s, 3H), 1.52 (d, *J* = 6.8 Hz, 1.5H),1.45 (d, *J* = 6.8 Hz, 1.5H), 1.33 (dd, *J* = 6.7 Hz, 3H), 1.05 (t, *J* = 6.2 Hz, 3H).

**Example 20 is prepared by SFC separation on the basis of the preparation in Example 19:**

| **Example No.** | **Structural Formula/Chemical Name** | **MS m/z [M+1]⁺ / ¹H NMR(400 MHz)** |
|---|---|---|
| **Example 20** | 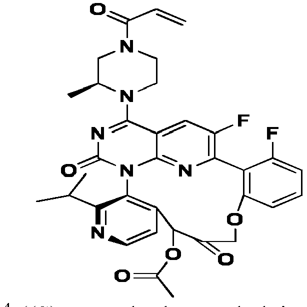 (R)-2⁴-((S)-4-acryloyl-2-methylpiperazin-1- yl)-2⁶,3⁶-difluoro-1²-isopropyl-2²,6-dioxo-2¹,2²-dihydro-4-oxa-2(1,7)-pyrido[2,3-d]pyrimidina-(3,4)-pyridina-3 (1,2)-benzocycloheptane-7-yl acetate | ESI-MS 659[M+1]⁺. |
| | | ¹H NMR (400 MHz, DMSO-*d*₆) δ 8.65 (d, *J* = 5.0 Hz, 1H), 8.37 - 8.21 (m, 1H), 7.60 - 7.52 (m, 1H), 7.12 - 6.94 (m, 3H), 6.93 - 6.78 (m, 1H), 6.25 - 6.15 (m, 2H), 5.77 (d, *J* = 10.9 Hz, 1H), 4.94 - 4.67 (m, 3H), 4.52 (d, *J* = 13.8 Hz, 1H), 4.41 - 3.92 (m, 3H), 3.70 - 3.50 (m, 2H), 3.10 - 3.02 (m, 1H), 1.90 (s, 3H), 1.43 (t, *J* = 6.0 Hz, 3H), 1.18 (d, *J* = 6.6 Hz, 3H), 0.95 (d, *J* = 6.8 Hz, 3H). |

**Examples 21-22 can be prepared by selecting corresponding starting materials according to the complete or partial synthesis method of Examples 19-20:**

| **Example No.** | **Structural Formula/Chemical Name** | **MS m/z [M+1]⁺ / ¹H NMR(400 MHz)** |
|---|---|---|
| **Example 21** | 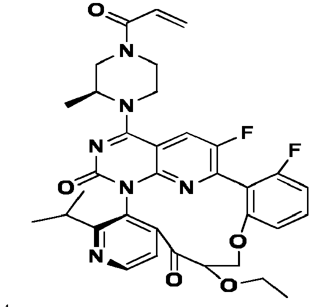 (R)-2⁴-((S)-4-acryloyl-2-methylpiperazin-1-yl)-6-ethoxy-2⁶,3⁶-difluoro-1²-isopropyl-2¹,2²-dihydro-4-oxa-2(1,7)-pyrido[2,3-d]pyrimidina-1(3,4)-pyridina-3 (1,2)-benzocycloheptane-22,7-dione | ESI-MS 645[M+1]⁺. |
| | | ¹H NMR (400 MHz, DMSO-*d*₆) δ 8.81 (d, *J* = 5.0 Hz, 1H), 8.21 (d, *J* = 5.0 Hz, 1H), 8.20 - 8.12 (m, 1H), 7.55 - 7.47 (m 1H), 7.17 (d, *J* = 8.4 Hz, 1H), 6.99 (t, *J* = 8.8 Hz, 1H), 6.93 - 6.78 (m, 1H), 6.20 (d, *J* = 16.5 Hz, 1H), 5.77 (d, *J* = 10.3 Hz, 1H), 4.85 - 4.68 (m, 2H), 4.58 - 3.95 (m, 6H), 3.72 - 3.46 (m, 4H), 3.14 (m, 1H), 1.50 - 1.40 (m, 3H), 1.28 - 1.12 (m, 6H), 0.92 (d, *J* = 6.7 Hz, 3H). |
| **Example 22** | 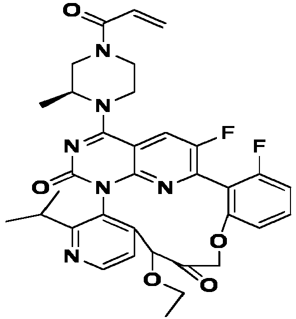 2⁴-((S)-4-acryloyl-2-methylpiperazin-1-yl)-7-ethoxy-2⁶,3⁶-difluoro-1²-isopropyl-2¹,2²-dihydro-4-oxa-2(1,7)-pyrido[2,3-dpyrimidina-1(3,4)-pyridina-3(1,2)-benzocycloheptane-22,6-dione | ESI-MS 645[M+1]⁺. |
| | | ¹H NMR (400 MHz, DMSO-*d*₆) δ 8.60 (d, *J* = 4.9 Hz, 1H), 8.55 - 8.23 (m, 1H), 7.57 - 7.47 (m, 1H), 7.14 - 7.01 (m, 2H), 6.98 (d, *J* = 8.4 Hz, 1H), 6.94 - 6.79 (m, 1H), 6.21 (d, *J =* 16.8 Hz, 1H), 5.77 (d, *J* = 10.5 Hz, 1H), 5.12 (br, 1H), 4.88 - 4.72 (m, 2H), 4.62 (dd, *J* = 16.1, 2.6 Hz, 1H), 4.58 - 3.83 (m, 5H), 3.76 - 3.52 (m, 2H), 3.26 - 3.11 (m, 1H), 3.05 - 2.93 (m, 1H), 1.50 - 1.15 (m, 6H), 1.02 - 0.85 (m, 6H). |

### Example 23: Preparation of 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl L-valine ester

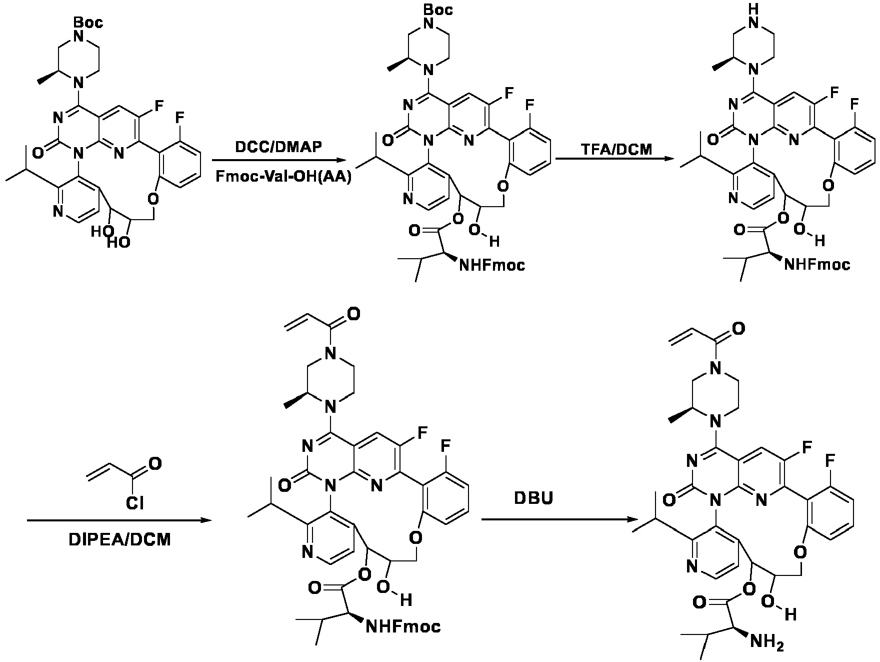

### Step 1: Synthesis of tert-butyl(3S)-4-(7-(((((9H-fluorene-9-yl)methoxy)carbonyl)-L-valyl)oxo)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate

N-fluorenemethoxycarbonyl-L-alanine (207 mg, 0.68 mmol), dicyclohexylcarbodiimide (140 mg, 0.68 mmol) and 4-dimethylaminopyridin (27.6 mg, 0.226 mmol) were added to tert-butyl (3S)-4-(2⁶,3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (150 mg, 0.226 mmol) in dichloromethane (10 mL), to subsequently react for 16 hrs at room temperature. The reaction was stopped, and dichloromethane (30 mL) was added for dilution. The resultant was washed with saline water (20 mL * 2). The organic phase was separated, then dried over anhydrous sodium sulfate, and concentrated under reduced pressure to remove the solvent. The residue was separated by flash silicagel columns and reversed-phase chromatographic columns to obtain tert-butyl (3S)-4-(7-(((((9H-fluorene-9-yl)methoxy)carbonyl)-L-valyl)oxo)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (182 mg, yield: 44%). ESI-MS 986 [M+1]⁺.

### Step 2: Synthesis of 2⁶,3⁶-difluoro-6-hydroxy-1²-isopropyl-2⁴-((S)-2-methylpiperazin-1-yl)-2²-oxo-2¹,2²-dihydro-4-oxa-2(1,7)-pyrido[2,3-d]pyrimidina-1(3,4)-pyridina-3(1,2)-benzocycloheptane-7-yl (((9H-fluorene-9-yl)methoxy)carbonyl)-L-valine ester

Tert-butyl (3S)-4-(7-(((((9H-fluorene-9-yl)methoxy)carbonyl)-L-valyl)oxo)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (180.0 mg, 0.27 mmol) was dissolved in 5 mL of dichloromethane and 2 mL of trifluoroacetic acid. The mixture was stirred at room temperature to react for 1.5 hrs. The reaction was stopped, and the resultant was concentrated under reduced pressure to remove the solvent to obtain crude 2⁶,3⁶⁻difluoro-6-hydroxy-1²-isopropyl-2⁴-((S)-2-methylpiperazin-1-yl)-2²-oxo-2¹,2²-dihydro-4-oxa-2(1,7)-pyrido[2,3-d]pyrimidina-1(3,4)-pyridina-3(1,2)-benzocycloheptane-7-yl(((9H-fluorene-9-yl)methoxy)carbonyl)-L-valine ester (170 mg), which was directly used in the next step. ESI-MS 886 [M+1]⁺.

### Step 3: Synthesis of 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl(((9H-fluorene-9-yl)methoxy)carbonyl)-L-valine ester

2⁶,3⁶⁻difluoro-6-hydroxy-1²-isopropyl-2⁴-((S)-2-methylpiperazin-1-yl)-2²-oxo-2¹,2²-dihydro-4-oxa-2(1,7)-pyrido[2,3-d]pyrimidina-1(3,4)-pyridina-3(1,2)-benzocycloheptane-7-yl (((9H-fluorene-9-yl)methoxy)carbonyl)-L-valine ester (180 mg, 0.27 mmol) was dissolved in dichloromethane (10 mL), and N,N-diisopropylethylamine (1.0 mL) was added. The mixture was cooled to 0°C, and acryloyl chlorine (10.0 mg, 0.10 mmol) was added. During the reaction, the mixture was naturally warmed up to room temperature and stirred to react for 1.5 hrs. The reaction was stopped, and dichloromethane (30 mL) was added for dilution. The resultant was washed with saline water (20 mL * 2), and the organic phase was separated, then dried over anhydrous sodium sulfate, and concentrated under reduced pressure to remove the solvent. The residue was separated by flash silicagel columns and reversed-phase chromatographic columns to obtain 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl (((9H-fluorene-9-yl)methoxy)carbonyl)-L-valine ester (90 mg, yield: 88%). ESI-MS 940 [M+1]⁺.

### Step 4: Synthesis of 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl L-valine ester

2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl (((9H-fluorene-9-yl)methoxy)carbonyl)-L-valine ester (88 mg, 0.094 mmol) was dissolved in dichloromethane (10 mL), and added with 0.1 mL of 1,8-diazacyclo[5,4,0]hendecene-7 to react for 2 minutes at room temperature. After the reaction was ended, the resultant was washed with water, and the organic phase was separated, then dried over anhydrous sodium sulfate, and concentrated under reduced pressure to remove the solvent. The residue was separated by flash silicagel columns and reversed-phase chromatographic columns to obtain a product 2⁴-((S)-4-acryloyl-2-methylpiperazin-1-yl)-2⁶,3⁶-difluoro-6-hydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-7-yl L-valine ester (21 mg, yield: 27%). ESI-MS 718 [M+1]⁺.

¹H NMR (400 MHz, DMSO-*d*₆) δ 8.58 (d, *J* = 5.1 Hz, 1H), 8.52 - 8.27(m, 1H), 8.24 (dd, *J* = 10.8, 5.1 Hz, 1H), 7.57 - 7.45 (m, 1H), 7.18 (d, *J* = 8.3 Hz, 1H), 7.02 (t, *J* = 8.8 Hz, 1H), 6.93 - 6.77 (m, 1H), 6.21 (d, *J* = 16.9 Hz, 1H), 6.09 (dd, *J* = 9.8, 5.0 Hz, 1H), 5.83 - 5.72 (m, 1H), 5.58 (d, *J* = 9.8 Hz, 1H), 5.21 - 4.96 (m, 1H), 4.80 - 3.82 (m, 6H), 3.76 - 3.43 (m, 6H), 3.05 - 2.85 (m, 2H), 1.48 - 1.05 (m, 6H), 0.98 - 0.70 (m, 9H).

### Example 24: Preparation of 2⁴-((S)-4-acryloyl-2-methylpiperazin-1-yl)-2⁶,3⁶-difluoro-7-(2-fluoroethoxy)-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one

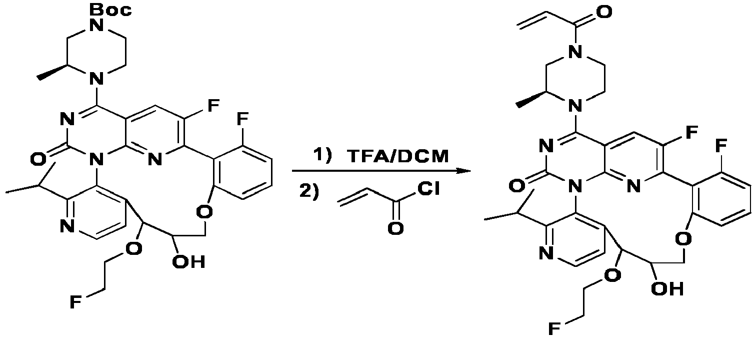

Tert-butyl (3S)-4-(2⁶,3⁶-difluoro-7-(2-fluoroethoxy)-6-hydroxy-1²-isopropyl-2²-oxo-2¹,2²-oxo-2¹,2²-dihydro-4-oxa-2(1,7)-pyrido[2,3-*d*]pyrimidina-1(3,4)-pyridina-3(1,2)-benzocycloheptane-2⁴-yl)-3-methylpiperazine-1-carboxylate (35 mg, 0.05 mmol) was dissolved in dichloromethane (3.5 mL), and trifluoroacetic acid (0.5 mL) was added. The mixture was stirred at room temperature to react for 2 hrs. The reaction was stopped, and the resultant was concentrated under reduced pressure to remove the solvent. The residue was dissolved in dichloromethane (3.5 mL), added with N,N-diisopropylethylamine (26 mg, 0.2 mmol), cooled to 0°C, added with acryloyl chlorine (9.1 mg, 0.1 mmol), and continued to react for 2 hrs at 0°C. The reaction was stopped, and dichloromethane (3.5 mL) was added for dilution. The resultant was washed with saturated sodium chloride aqueous solution (3 mL * 2), dried over anhydrous sodium sulfate, and concentrated under reduced pressure to remove the solvent. The residue was separated by the preparative HPLC to obtain 2⁴-((*S*)-4-acryloyl-2-methylpiperazin-1-yl)-2⁶,3⁶-difluoro-7-(2-fluoroethoxy)-6-hydroxy-1²-isopropyl-2¹,2²-dihydro-4-oxa-2(1,7)-pyrido[2,3-*d*]pyrimidina-1(3,4)-pyridina-3(1,2)-benzocycloheptane-2²-one (21 mg, yield: 63%). ESI-MS 665 [M+1]⁺.

¹HNMR (400 MHz, DMSO-*d*₆) δ 8.54 (d, *J* = 5.1 Hz, 1H), 8.44 (d, *J* = 10.1 Hz, 0.5H), 8.28 (t, *J* = 11.2 Hz, 0.5H), 8.04 (d, *J* = 5.0 Hz, 1H), 7.50 (td, *J* = 8.4, 6.9 Hz, 1H), 7.21 (d, *J* = 8.4 Hz, 1H), 7.03 (t, *J* = 8.9 Hz, 1H), 6.95 - 6.77 (m, 1H), 6.20 (d, *J* = 16.6 Hz, 1H), 5.77 (dd, *J* = 10.3, 2.4 Hz, 1H), 5.65 (dd, *J* = 5.1, 1.8 Hz, 1H), 5.09 - 4.96 (m, 0.5H), 4.80 (br, 0.5H), 4.46 - 4.38 (m, 3H), 4.31 - 4.27 (m, 3H), 4.15 - 4.12 (m, 1H), 3.92 (dt, *J* = 68.3, 13.8 Hz, 1H), 3.64 - 3.48 (m, 4H), 3.43 (t, *J* = 9.3 Hz, 1H), 2.87 - 2.84 (m, 1H), 1.41 (d, *J* = 6.6 Hz, 1H), 1.25 - 1.24 (m, 3H), 1.17 (dd, *J* = 6.6, 2.1 Hz, 3H), 0.83 (dd, *J* = 6.8, 4.0 Hz, 3H).

**Examples 25-36 can be prepared by selecting corresponding starting materials according to the complete or partial synthesis method of Example 24:**

| **Example No.** | **Structural Formula/Chemical Name** | **MS m/z [M+1]⁺ / ¹H NMR(400 MHz)** |
|---|---|---|
| **Example 25** | 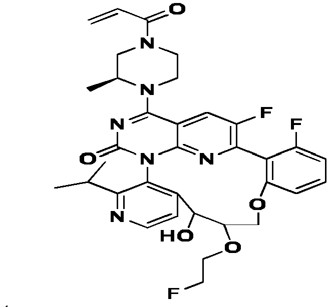 2⁴-((*S*)-4-acryloyl-2-methylpiperazin-1-yl)-2⁶,3⁶-difluoro-6-(2-fluoroethoxy)-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 665 [M+1]⁺. |
| | | ¹H NMR (400 MHz, DMSO-*d*₆) δ 8.52 (d, *J* = 5.1 Hz, 1H), 8.45 (d, *J* = 9.3 Hz, 0.5H), 8.28 - 8.22 (m, 0.5H), 7.91 (dd, *J* = 5.0, 1.5 Hz, 1H), 7.49 (dd, *J* = 8.4, 6.9 Hz, 1H), 7.20 (d, *J* = 8.4 Hz, 1H), 7.02 (t, *J* = 8.9 Hz, 1H), 6.88 - 6.84 (m, 1H), 6.21 (d, *J* = 16.7 Hz, 1H), 5.77 (dd, *J* = 10.4, 2.4 Hz, 1H), 5.13 - 5.11 (m, 2H), 4.73 - 4.46 (m, 4H), 4.23 - 4.20 (m, 2H), 4.11 - 4.03 (m, 1H), 4.03 - 3.82 (m, 4H), 3.67-3.61 (m, 2H), 2.97 - 2.86 (m, 1H), 1.43 (s, 1H), 1.30 - 1.20 (m, 3H), 1.15 (dd, *J* = 6.8, 2.3 Hz, 3H), 0.87 (d, *J* = 6.7 Hz, 3H). |
| Example 26 | 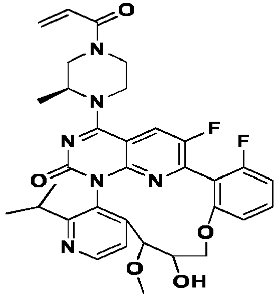 2⁴-((*S*)-4-acryloyl-2-methylpiperazin-1-yl)-7-methoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 633 [M+1]⁺. |
| | | ¹H NMR (400 MHz, CDCl₃) δ 8.64 (d, *J* = 5.1 Hz, 1H), 7.84 (d, *J* = 8.4 Hz, 1H), 7.71 (d, *J =* 5.2 Hz, 1H), 7.39 (td, *J=* 8.5, 6.6 Hz, 1H), 6.98 (d, *J =* 8.4 Hz, 1H), 6.94 - 6.80 (m, 1H), 6.61 (s, 1H), 6.41 (dd, *J* = 16.7, 2.0 Hz, 1H), 5.82 (dd, *J* = 10.4, 1.9 Hz, 1H), 4.99 - 4.75 (m, 2H), 4.52 - 4.48 (m, 1H), 4.33 (dt, *J* = 9.6, 2.3 Hz, 1H), 4.27 - 4.11 (m, 2H), 3.97 - 3.92 (m, 2H), 3.74 - 3.48 (m, 3H), 3.30 (s, 3H), 2.79 (p, *J* = 6.7 Hz, 1H), 1.41 (d, *J* = 6.8 Hz, 2H), 1.32 (dd, *J* = 6.7, 1.6 Hz, 4H), 0.97 (d, *J* = 6.8 Hz, 3H). |
| **Example** 27 | 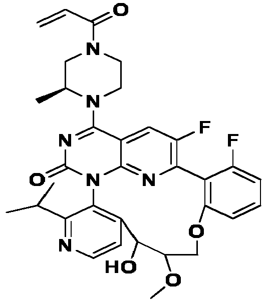 2⁴-((*S*)-4-acryloyl-2-methylpiperazin-1-yl)-6-methoxy-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 633 [M+1]⁺. |
| | | ¹H NMR (400 MHz, CDCl₃) δ 8.63 (d, *J* = 5.1 Hz, 1H), 7.99 (dd, *J* = 5.1, 2.6 Hz, 1H), 7.79 (dd, *J =* 16.4, 8.4 Hz, 1H), 7.38 (td, *J* = 8.3, 6.5 Hz, 1H), 6.90 (d, *J* = 8.4 Hz, 1H), 6.84 (t, *J =* 8.6 Hz, 1H), 6.61 (br, 1H), 6.42 (dt, *J* = 16.7, 1.8 Hz, 1H), 5.82 (dd, *J =* 10.4, 1.9 Hz, 1H), 4.97 - 4.56 (m, 2H), 4.55 - 4.48 (m, 1H), 4.38 (t, *J* = 3.8 Hz, 1H), 4.22 - 4.18 (m, 1H), 3.97 - 3.93 (m, 1H), 3.68 (s, 3H), 3.57 (ddd, *J =* 10.5, 8.5, 1.8 Hz, 2H), 3.51 - 3.17 (m, 3H), 2.92 (p, *J* = 6.6 Hz, 1H), 1.29 - 1.26 (m, 6H), 1.05 (d, *J* = 6.6 Hz, 3H). |
| **Example 28** | 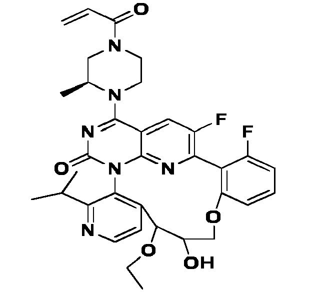 2⁴-((*S*)-4-acryloyl-2-methylpiperazin-1-yl)-7-ethoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 647 [M+1]⁺ . |
| | | ¹H NMR (400 MHz, CDCl₃) δ 8.66 (d, *J* = 5.2 Hz, 1H), 7.97 - 7.69 (m, 2H), 7.45 - 7.34 (m, 1H), 6.98 (d, *J* = 8.4 Hz, 1H), 6.86 (t, *J* = 8.7 Hz, 1H), 6.61 (br, 1H), 6.42 (d, *J* = 16.7 Hz, 1H), 5.83 (dd, *J* = 10.4, 1.9 Hz, 1H), 5.27 - 4.97 (m, 1H), 4.84 - 4.74 (m, 1H), 4.51 - 4.49 (m, 1H), 4.38 - 4.26 (m, 2H), 4.13 (s, 1H), 3.92 (br, 2H), 3.79 - 3.74 (m, 1H), 3.64 - 3.56 (m, 2H), 3.38 - 3.23 (m, 3H), 2.81 (br, 1H), 1.41 - 1.35 (m, 6H), 1.11 (td, *J* = 7.0, 2.0 Hz, 3H), 1.01 (s, 3H). |
| **Example 29** | 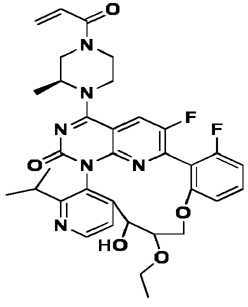 2⁴-((*S*)-4-acryloyl-2-methylpiperazin-1-yl)-6-ethoxy-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 647 [M+1]⁺. |
| | | ¹H NMR (400 MHz, CDCl₃) δ 8.65 (d, *J* = 5.2 Hz, 1H), 8.08 (s, 1H), 7.80 (dd, *J* = 16.3, 8.3 Hz, 1H), 7.47 - 7.32 (m, 1H), 6.90 (d, *J* = 8.4 Hz, 1H), 6.84 (t, *J* = 8.7 Hz, 1H), 6.61 (br, 1H), 6.42 (dd, *J* = 16.9, 2.1 Hz, 1H), 5.83 (dd, *J* = 10.4, 1.9 Hz, 1H), 5.47 - 5.18 (m, 1H), 4.80 - 4.60 (m, 2H), 4.49 (dd, *J* = 8.5, 4.7 Hz, 1H), 4.36 (s, 1H), 4.22 - 4.19 (m, 1H), 4.06 - 4.03 (m, 1H), 3.98 - 3.86 (m, 1H), 3.84 - 3.77 (m, 2H), 3.58 (t, *J* = 9.7 Hz, 1H), 3.47 - 3.16 (m, 2H), 2.98 -2.95 (m, 2H), 1.41 - 1.22 (m, 9H), 1.07 (s, 3H). |
| **Example 30** | 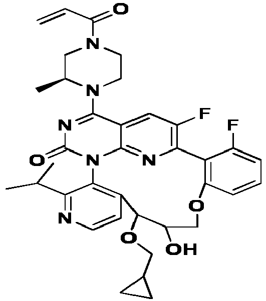 2⁴-((*S*)-4-acryloyl-2-methylpiperazin-1-yl)-7-(cyclopropylmethoxy)-2⁶,3⁶-difluoro-6-hydroxy-1²-isopropyl-2¹,2²-dihydro-4-oxa-2(1,7)-pyrido[2,3-*d*]pyrimidina-1(3,4)-pyridina-3 (1,2)-benzocycloheptane-2²-one | ESI-MS 673 [M+1]⁺. |
| | | ¹H NMR (400 MHz, DMSO-*d*₆) δ 8.52 (d, *J* = 5.0 Hz, 1H), 8.46 - 8.43 (m, 0.5H), 8.31 - 8.26 (m, 0.5H), 8.04 (t, *J* = 4.6 Hz, 1H), 7.50 (q, *J* = 8.0 Hz, 1H), 7.19 (d, *J* = 8.3 Hz, 1H), 7.02 (t, *J* = 8.8 Hz, 1H), 6.86 (dd, *J* = 16.7, 10.4 Hz, 1H), 6.21 (d, *J* = 16.5 Hz, 1H), 5.77 (dd, *J* = 10.3, 2.4 Hz, 1H), 5.61 (s, 1H), 5.12 - 4.69 (m, 1H), 4.47 - 4.35 (m, 1H), 4.28 - 4.23 (m, 2H), 4.16 - 3.96 (m, 2H), 3.90 - 3.87 (m, 1H), 3.77 - 3.54 (m, 1H), 3.53 - 3.28 (m, 2H), 3.20 (dt, *J* = 10.2, 7.0 Hz, 1H), 2.98 (dd, *J* = 10.3, 7.2 Hz, 1H), 2.91 -2.80 (m, 1H), 1.41 (d, *J* = 6.6 Hz, 1H), 1.24 (d, *J* = 6.6 Hz, 3H), 1.18 (dd, *J =* 6.8, 2.8 Hz, 3H), 0.83 (dd, *J* = 6.7, 2.4 Hz, 3H), 0.32 - 0.22 (m, 2H), 0.04 - 0.01 (m, 2H). |
| **Example 31** | 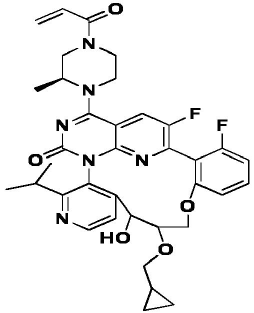 2⁴-((*S*)-4-acryloyl-2-methylpiperazin-1-yl)-6-(cyclopropylmethoxy)-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 673 [M+1]⁺. |
| | | ¹H NMR (400 MHz, DMSO-*d*₆) δ 8.52 (d, *J* = 5.0 Hz, 1H), 8.44 (d, *J* = 9.3 Hz, 0.5H), 8.27 - 8.21 (m, 0.5H), 7.95 (d, *J* = 5.1 Hz, 1H), 7.49 (td, *J* = 8.3, 6.7 Hz, 1H), 7.20 (d, *J* = 8.3 Hz, 1H), 7.01 (t, *J* = 8.8 Hz, 1H), 6.95 - 6.74 (m, 1H), 6.21 (d, *J* = 16.6 Hz, 1H), 5.77 (dd, *J* = 10.4, 2.3 Hz, 1H), 5.16 - 5.09 (m, 1H), 5.01 - 4.97 (m, 1H), 4.77 (br, 1H), 4.59 (dd, *J* = 8.5, 4.6 Hz, 1H), 4.54 - 4.29 (m, 2H), 4.22 - 4.17 (m, 2H), 4.08 - 3.97 (m, 1H), 3.94 - 3.81 (m, 2H), 3.63 (dd, *J* = 10.3, 6.8 Hz, 2H), 3.49 (dd, *J* = 10.3, 6.8 Hz, 1H), 3.00 - 2.85 (m, 1H), 1.25 (t, *J* = 5.4 Hz, 3H), 1.15 (dd, *J* = 6.7, 2.0 Hz, 3H), 1.12 -1.05 (m, 1H), 0.87 (d, *J* = 6.7 Hz, 3H), 0.50 - 0.47 (m, 2H), 0.28 - 0.23 (m, 2H). |
| **Example 32** | 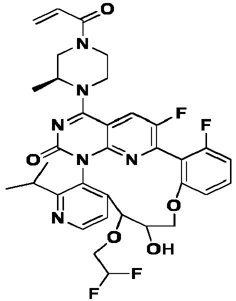 2⁴-((*S*)-4-acryloyl-2-methylpiperazin-1-yl)-7-(2,2-difluoroethoxy)-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 683 [M+1]⁺. |
| | | ¹H NMR (400 MHz, CDCl₃) δ 8.67 (d, *J* = 5.2 Hz, 1H), 7.85 (dd, *J* = 15.2, 8.3 Hz, 1H), 7.76 (dd, *J* = 5.2, 2.1 Hz, 1H), 7.41 (td, *J* = 8.5, 6.6 Hz, 1H), 6.99 (d, *J* = 8.5 Hz, 1H), 6.86 (t, *J* = 8.7 Hz, 1H), 6.61 (br, 1H), 6.42 (dd, *J* = 16.8, 1.9 Hz, 1H), 5.96 - 5.72 (m, 2H), 5.44 - 4.67 (m, 2H), 4.48 - 4.46 (m, 1H), 4.37 (dd, *J* = 10.0, 2.2 Hz, 1H), 4.24 (s, 1H), 4.00 - 3.91 (m, 2H), 3.85 - 3.71 (m, 2H), 3.68 - 3.58 (m, 1H), 3.47 - 3.29 (m, 4H), 2.79 (p, *J* = 6.7 Hz, 1H), 1.41 (d, *J* = 6.8 Hz, 2H), 1.31 (dd, *J* = 6.6, 3.6 Hz, 4H), 1.05 - 0.96 (m, 3H). |
| **Example 33** | 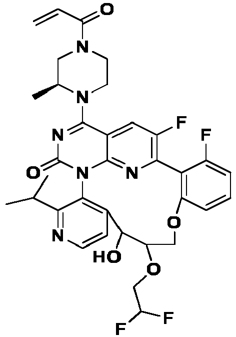 2⁴-((*S*)-4-acryloyl-2-methylpiperazin-1-yl)-6-(2,2-difluoroethoxy)-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 683 [M+1]⁺. |
| | | ¹H NMR (400 MHz, CDCl₃) δ 8.67 (s, 1H), 7.97 (d, *J* = 5.1 Hz, 1H), 7.79 (dd, *J* = 17.0, 8.3 Hz, 1H), 7.38 (td, *J* = 8.4, 6.5 Hz, 1H), 6.89 (d, *J* = 8.3 Hz, 1H), 6.84 (t, *J* = 8.7 Hz, 1H), 6.61 (br, 1H), 6.49 - 6.37 (m, 1H), 6.23 - 5.94 (m, 1H), 5.83 (dd, *J* = 10.5, 1.9 Hz, 1H), 4.81 - 4.71 (m, 2H), 4.52 (dd, *J* = 8.5, 4.9 Hz, 1H), 4.38 (dd, *J* = 5.7, 3.3 Hz, 1H), 4.30 - 4.15 (m, 3H), 4.02 - 3.92 (m, 2H), 3.64 (ddd, *J* = 10.3, 8.4, 1.6 Hz, 1H), 3.51 - 3.09 (m, 3H), 2.92 (p, *J* = 7.0 Hz, 1H), 1.37 (d, *J* = 6.7 Hz, 3H), 1.33 - 1.23 (m, 3H), 1.06 (dd, *J* = 6.8, 3.7 Hz, 3H). |
| **Example 34** | 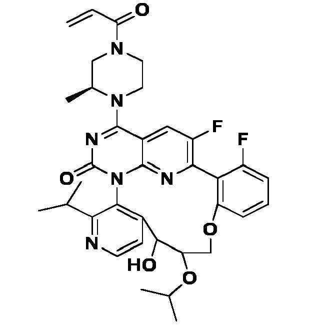 2⁴-((*S*)-4-acryloyl-2-methylpiperazin-1-yl)-6-isopropoxy-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 661 [M+1]⁺. |
| | | ¹H NMR (400 MHz, MeOH-*d*₄) δ 8.63 (d, *J* = 5.1 Hz, 1H), 8.05 (dd, *J* = 5.1, 3.7 Hz, 1H), 7.78 (dd, *J* = 16.5, 8.4 Hz, 1H), 7.37 (td, *J=* 8.4, 6.5 Hz, 1H), 6.89 (d, *J* = 8.3 Hz, 1H), 6.83 (t, *J* = 8.7 Hz, 1H), 6.61 (br, 1H), 6.41 (dt, *J* = 16.7, 2.0 Hz, 1H), 5.82 (dd, *J* = 10.5, 1.8 Hz, 1H), 5.52 - 5.06 (m, 1H), 4.79 - 4.59 (m, 2H), 4.44 - 4.37 (m, 1H), 4.32 (t, *J* = 4.1 Hz, 1H), 4.20 (d, *J* = 13.2 Hz, 1H), 4.10 (dt, *J* = 10.9, 4.2 Hz, 1H), 4.02 - 3.92 (m, 2H), 3.74 (br, 1H), 3.59 (ddd, *J* = 10.6, 8.3, 2.2 Hz, 1H), 3.50 - 3.27 (m, 1H), 2.92 (p, *J* = 6.5 Hz, 1H), 1.37 (d, *J* = 6.6 Hz, 2H), 1.29 - 1.23 (m, 10H), 1.05 - 1.02 (m, 3H). |
| **Example 35** | 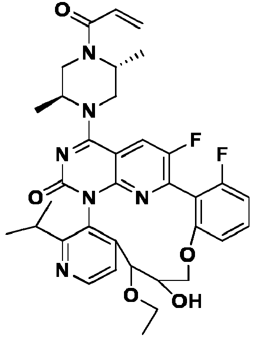 2⁴-((2S,5R)-4-acryloyl-2,5-dimethylpiperazin-1-yl)-7-ethoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 661 [M+1]⁺. |
| | | ¹H NMR (400 MHz, DMSO-*d*₆) δ 8.60 - 8.50 (m, 1H), 8.47 - 8.17 (m, 1H), 8.08 - 7.97 (m, 1H), 7.50 (q, *J* = 8.0 Hz, 1H), 7.21 (d, *J* = 8.3 Hz, 1H), 7.03 (t, *J* = 8.9 Hz, 1H), 6.93 - 6.72 (m, 1H), 6.18 (d, *J* = 16.3 Hz, 1H), 5.81 - 5.70 (m, 1H), 5.63 - 5.47 (m, 1H), 5.0 - 4.65 (m, 2H), 4.57 - 4.35 (m, 2H), 4.30 - 3.97 (m, 4H), 3.94 - 3.64 (m, 2H), 2.86 (dd, *J* = 13.2, 7.0 Hz, 3H), 1.50 - 1.07 (m, 9H), 1.06 - 0.74 (m, 6H). |
| **Example 36** | 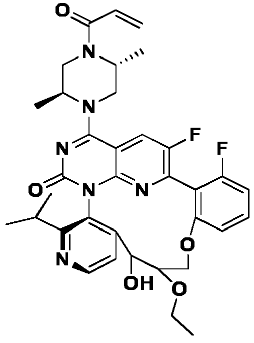 (R)-2⁴-((2S,5R)-4-acryloyl-2,5-dimethylpiperazin-1-yl)-6-ethoxy-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 661 [M+1]⁺. |
| | | ¹H NMR (400 MHz, DMSO-*d₆*) δ 8.51 (d, *J* = 5.1 Hz, 1H), 8.13 (dd, *J* = 14.5, 9.2 Hz, 1H), 7.91 (d, *J* = 5.0 Hz, 1H), 7.55-7.42 (m, 1H), 7.21 (d, *J* = 8.3 Hz, 1H), 7.02 (t, *J* = 8.8 Hz, 1H), 6.95 - 6.73 (m, 1H), 6.24 - 6.14 (m, 1H), 5.78 - 5.72 (m, 1H), 4.96 (t, *J* = 7.3 Hz, 1H), 4.92 - 4.72 (m, 2H), 4.68 - 4.51 (m, 3H), 4.25 - 4.17 (m, 1H), 3.90 - 3.54 (m, 6H), 2.98 - 2.86 (m, 1H), 1.45 (t, *J=* 6.4 Hz, 3H), 1.33 (dd, *J =* 9.7, 6.7 Hz, 3H), 1.19 (t, *J* = 7.0 Hz, 3H), 1.12 (dd, *J =* 6.7, 1.8 Hz, 3H), 0.90 (t, *J* = 5.9 Hz, 3H). |

**Examples 37-58 are prepared by SFC separation on the basis of the preparation in Examples 24-36:**

| **Example No.** | **Structural Formula/Chemical Name** | **MS m/z [M+1]⁺ / ¹H NMR(400 MHz)** |
|---|---|---|
| **Example 37** | 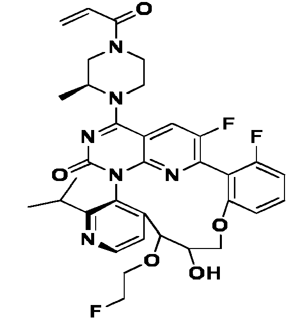 (*R*)-2⁴-((*S*)-4-acryloyl-2-methylpiperazin-1-yl)-2⁶,3⁶-difluoro-7-(2-fluoroethoxy)-6-hydroxy-1²-isopropyl-21,2²-dihydro-4-oxa-2(1,7)-pyrido[2,3-d]pyrimidina-1(3,4)-pyridina-3(1,2)-benzocycloheptane-2²-one | ESI-MS 665 [M+1]⁺. |
| | | ¹H NMR (400 MHz, CDCl₃) δ 8.65 (d, *J* = 5.1 Hz, 1H), 7.84 (d, *J =* 8.4 Hz, 1H), 7.79 (d, *J =* 5.1 Hz, 1H), 7.40 (td, *J=* 8.4, 6.6 Hz, 1H), 6.98 (d, *J=* 8.3 Hz, 1H), 6.86 (t, *J =* 8.7 Hz, 1H), 6.61 (br, 1H), 6.42 (dd, *J =* 16.8, 2.0 Hz, 1H), 5.82 (dd, *J* = 10.4, 1.9 Hz, 1H), 4.85 -4.74 (m, 2H), 4.58 - 4.46 (m, 2H), 4.45 - 4.34 (m, 3H), 4.24 (s, 1H), 4.02 - 3.93 (m, 2H), 3.86 - 3.55 (m, 4H), 3.36 (d, *J =* 4.7 Hz, 1H), 3.23 (s, 1H), 2.79 (p, *J =* 6.7 Hz, 1H), 1.32 - 1.26 (m, 6H), 0.98 (d, *J =* 6.8 Hz, 3H). |
| **Example 38** | 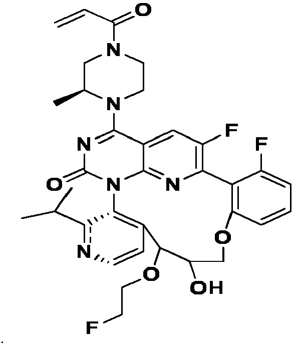 (*S*)-2⁴-((*S*)-4-aeryloyl-2-methylpiperazin-1-yl)-2⁶,3⁶-difluoro-7-(2-fluoroethoxy)-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 665 [M+1]⁺. |
| | | ¹H NMR (400 MHz, CDCl₃) δ 8.64 (d, *J* = 5.1 Hz, 1H), 7.88 (d, J = 8.2 Hz, 1H), 7.78 (d, J = 5.1 Hz, 1H), 7.40 (q, J = 8.1 Hz, 1H), 6.98 (d, J = 8.4 Hz, 1H), 6.86 (t, J = 8.7 Hz, 1H), 6.61 (br, 1H), 6.42 (dd, J = 16.8, 1.9 Hz, 1H), 5.82 (dd, J = 10.2, 1.9 Hz, 1H), 5.46 - 4.65 (m, 2H), 4.62-4.33 (m, 4H), 4.25 (s, 2H), 4.16 - 3.56 (m, 5H), 3.56 - 3.26 (m, 1H), 3.56 - 3.26 (m, 1H), 3.09-2.71 (m, 2H), 1.41 (d*, J* = 6.8 Hz, 3H), 1.31 (d, *J* = 6.6 Hz, 3H), 0.98 (s, 3H). |
| **Example 39** | 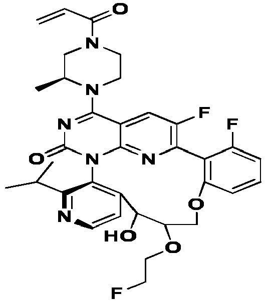 (*R*)-2⁴ -((*S*)-4-aeryloyl-2-methylpiperazin-1-yl)-2⁶,3⁶-difluoro-6-(2-fluoroethoxy)-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 665 [M+1]⁺. |
| | | ¹H NMR (400 MHz, CDCl₃) δ 8.65 (d, *J* = 5.2 Hz, 1H), 8.06 (d, *J =* 5.1 Hz, 1H), 7.77 (d, *J =* 8.3 Hz, 1H), 7.38 (td, *J=* 8.4, 6.5 Hz, 1H), 6.90 (d, *J =* 8.3 Hz, 1H), 6.84 (t, *J =* 8.7 Hz, 1H), 6.61 (br, 1H), 6.42 (dd, *J =* 16.7, 1.9 Hz, 1H), 5.83 (dd, *J* = 10.5, 1.9 Hz, 1H), 5.35 (dd, *J =* 5.3, 3.9 Hz, 1H), 4.76-4.70 (m, 1H), 4.68 - 4.58 (m, 1H), 4.56 - 4.48 (m, 2H), 4.38 (d, *J* = 3.5 Hz, 1H), 4.34 - 4.21 (m, 1H), 4.19-4.13 (m, 1H), 4.09 - 3.90 (m, 2H), 3.69-3.61 (m, 3H), 3.28 - 3.16 (m, 2H), 2.92 (p, *J =* 6.7 Hz, 1H), 1.32 - 1.20 (m, 6H), 1.06 (d, *J* = 6.8 Hz, 3H). |
| **Example 40** | 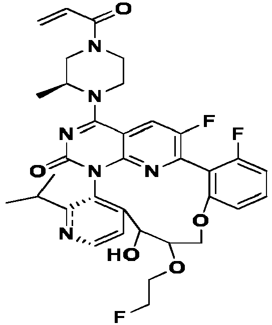 (*S*)-2⁴-((*S*)-4-acryloyl-2-methylpiperazin-1-yl-2⁶,3⁶-difluoro-6-(2-fluoroethoxy)-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 665 [M+1]⁺. |
| | | ¹H NMR (400 MHz, CDCl₃) δ 8.66 (d, *J* = 5.2 Hz, 1H), 8.06 (d, *J* = 5.1 Hz, 1H), 7.81 (d, *J=* 8.3 Hz, 1H), 7.38 (td, *J* = 8.3, 6.6 Hz, 1H), 6.90 (d, *J* = 8.3 Hz, 1H), 6.84 (t, *J* = 8.6 Hz, 1H), 6.61 (br, 1H), 6.42 (dd, *J* = 16.7, 1.9 Hz, 1H), 5.83 (dd, *J* = 10.4, 1.8 Hz, 1H), 5.50 - 5.17 (m, 1H), 4.85 - 4.70 (m, 1H), 4.69 - 4.58 (m, 1H), 4.58 - 4.49 (m, 1H), 4.40 (d, *J* = 3.4 Hz, 1H), 4.32 - 4.11 (m, 3H), 4.07 - 3.95 (m, 2H), 3.73 (br, 1H), 3.63 (dd, *J* = 10.7, 8.5 Hz, 1H), 3.38 - 3.12 (m, 2H), 2.94 (s, 2H), 1.37 (d, *J* = 6.8 Hz, 3H), 1.29 (d, *J* = 6.8 Hz, 3H), 1.06 (d, *J* = 6.8 Hz, 3H). |
| **Example 41** | 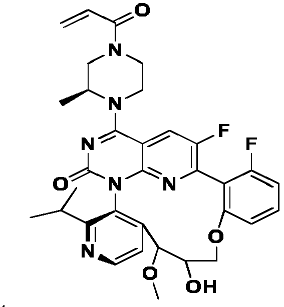 (*R*)-2⁴-((*S*)-4-aeryloyl-2-methylpiperazin-1-yl)-7-methoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 633 [M+1]⁺. |
| | | ¹H NMR (400 MHz, CDCl₃) δ 8.64 (d, *J* = 5.1 Hz, 1H), 7.84 (d, *J* = 8.3 Hz, 1H), 7.71 (d, *J* = 5.1 Hz, 1H), 7.39 (td, *J* = 8.4, 6.5 Hz, 1H), 6.98 (d, *J* = 8.3 Hz, 1H), 6.86 (t, *J* = 8.6 Hz, 1H), 6.62 (br, 1H), 6.41 (dd, *J* = 16.8, 2.0 Hz, 1H), 5.82 (dd, *J* = 10.5, 2.0 Hz, 1H), 4.80 - 4,74 (m, 2H), 4.50 (br, 1H), 4.34 (dd, *J =* 9.6, 2.3 Hz, 1H), 4.18 - 4.13 (m, 2H), 3.94 (t, *J* = 8.4 Hz, 1H), 3.78 - 3.45 (m, 3H), 3.33 (d, *J* = 4.6 Hz, 1H), 3.30 (s, 3H), 2.79 (p, *J* = 6.7 Hz, 1H), 1.32 - 1.26 (m, 6H), 0.97 (d, *J* = 6.8 Hz, 3H). |
| **Example 42** | 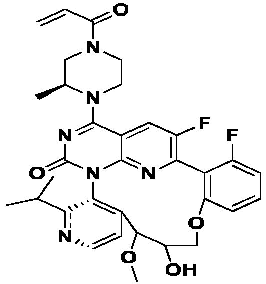 (*S*)-2⁴-((*S*)-4-acryloyl-2-methylpiperazin-1-yl)-7-methoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 633 [M+1]⁺. |
| | | ¹H NMR (400 MHz, CDCl₃) δ 8.64 (d, *J* = 5.1 Hz, 1H), 7.88 (d, *J* = 8.3 Hz, 1H), 7.70 (d, *J* = 5.2 Hz, 1H), 7.39 (td, *J* = 8.4, 6.6 Hz, 1H), 6.98 (d, *J* = 8.3 Hz, 1H), 6.86 (t, *J* = 8.6 Hz, 1H), 6.61 (br, 1H), 6.41 (dd, *J* = 16.6, 1.9 Hz, 1H), 5.82 (dd, *J* = 10.5, 1.9 Hz, 1H), 5.40 - 4.90 (m, 2H), 4.78 - 4.54 (m, 1H), 4.33 (dd, *J* = 9.7, 2.5 Hz, 1H), 4.25 - 4.09 (m, 3H), 3.95 - 3.77 (m, 3H), 3.48 - 3.37 (m, 1H), 3.31 (s, 3H), 2.79 (s, 1H), 1.41 (d, *J* = 6.7 Hz, 3H), 1.32 (d, *J=* 6.7 Hz, 3H), 0.96 (d, *J=* 6.8 Hz, 3H). |
| **Example 43** | 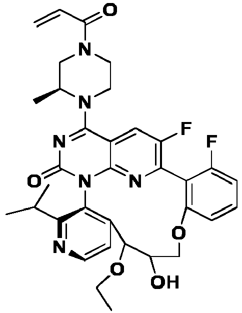 (*R*)-2⁴-((*S*)-4-acryloyl-2-methylpiperazin-1-yl)-7-ethoxy-2⁶,3⁶-difluoro-6-hydroxy-1²-isopropyl-2¹,2²-dihydro-4-oxa-2(1,7)-pyrido[2,3-*d*]pyrimidina-1(3,4)-pyridina-3(1,2)-benzocycloheptane-2²-one | ESI-MS 647 [M+1]⁺. |
| | | ¹H NMR (400 MHz, CDCl₃) δ 8.65 (d, *J* = 5.1 Hz, 1H), 7.84 (d, *J* = 8.4 Hz, 1H), 7.75 (d, *J* = 5.3 Hz, 1H), 7.40 (td, *J* = 8.4, 6.5 Hz, 1H), 6.98 (d, *J* = 8.4 Hz, 1H), 6.86 (t, *J* = 8.7 Hz, 1H), 6.62 (br, 1H), 6.41 (dd, *J* = 16.7, 1.9 Hz, 1H), 5.82 (dd, *J* = 10.3, 1.9 Hz, 1H), 4.86 - 4.74 (m, 2H), 4.50 (br, 1H), 4.34 (dd, *J* = 9.7, 2.7 Hz, 1H), 4.29 (d, *J* = 5.4 Hz, 1H), 4.14 - 4.10 (m, 1H), 4.01 - 3.91 (m, 2H), 3.74 - 3.71 (m, 1H), 3.60 (dq, *J* = 9.2, 6.9 Hz, 2H), 3.51 (br, 1H), 3.33 (dq, *J* = 9.2, 6.9 Hz, 1H), 3.23 (br, 1H), 2.79 (p, *J* = 6.7 Hz, 1H), 1.64 - 1.57 (m, 3H), 1.32 (d, *J* = 6.7 Hz, 3H), 1.11 (t, *J* = 6.9 Hz, 3H), 0.98 (d, *J* = 6.8 Hz, 3H). |
| **Example 44** | 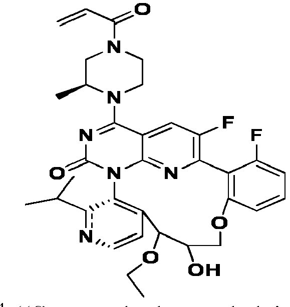 (*S*)-2⁴-((*S*)-4-acryloyl-2-methylpiperazin-1-yl)-7-ethoxy-2⁶,3⁶-difluoro-6-hydroxy-1²-isopropyl-2¹,2²-dihydro-4-oxa-2(1,7)-pyrido[2,3-*d*]pyrimidina-1(3,4)-pyridina-3(1,2)-benzocycloheptane-2²-one | ESI-MS 647 [M+1]⁺. |
| | | ¹H NMR (400 MHz, CDCl₃) δ 8.66 (d, *J* = 5.1 Hz, 1H), 7.88 (d, *J* = 8.3 Hz, 1H), 7.74 (d, *J* = 5.2 Hz, 1H), 7.40 (td, *J* = 8.4, 6.5 Hz, 1H), 6.98 (d, *J* = 8.3 Hz, 1H), 6.86 (t, *J* = 8.7 Hz, 1H), 6.61 (br, 1H), 6.42 (dd, *J* = 16.7, 1.9 Hz, 1H), 5.82 (dd, *J* = 10.5, 1.9 Hz, 1H), 5.26 - 4.99 (m, 1H), 4.78 - 4.53 (m, 1H), 4.35 - 4.31 (m, 2H), 4.27 - 4.21 (m, 1H), 4.12 (s, 1H), 3.93 (br, 2H), 3.78 - 3.75 (m, 1H), 3.60 (dq, *J* = 9.2, 7.0 Hz, 1H), 3.52 - 3.41 (m, 1H), 3.34 (dq, *J* = 8.4, 6.5, 6.1 Hz, 1H), 2.98-2.94 (m, 1H), 2.79 (s, 1H), 1.41 (d, *J* = 6.8 Hz, 3H), 1.32 (d, *J* = 6.6 Hz, 3H), 1.11 (t, *J* = 6.9 Hz, 3H), 0.97 (d, *J* = 6.6 Hz, 3H). |
| **Example 45** | 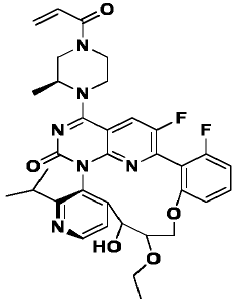 (*R*)-2⁴-((*S*)-4-acryloyl-2-methylpiperazin-1-yl)-6-ethoxy-2⁶,3⁶-difluoro-7-hydroxy-1²-isopropyl-2¹,2²-dihydro-4-oxa-2(1,7)-pyrido[2,3-*d*]pyrimidina-1(3,4)-pyridina-3(1,2)-benzocycloheptane-2²-one | ESI-MS 647 [M+1]⁺. |
| | | ¹H NMR (400 MHz, CDCl₃) δ 8.64 (d, *J* = 5.1 Hz, 1H), 8.06 (d, *J =* 5.2 Hz, 1H), 7.77 (d, *J =* 8.5 Hz, 1H), 7.37 (td, *J =* 8.4, 6.5 Hz, 1H), 6.90 (d, *J* = 8.4 Hz, 1H), 6.83 (t, *J =* 8.7 Hz, 1H), 6.61 (br, 1H), 6.41 (dd, *J =* 16.8, 2.0 Hz, 1H), 5.82 (dd, *J* = 10.3, 1.9 Hz, 1H), 4.87 - 4.58 (m, 3H), 4.48 (dd, *J =* 8.4, 4.7 Hz, 1H), 4.35 (d, *J* = 3.5 Hz, 1H), 4.08 - 4.03 (m, 2H), 3.97 - 3.86 (m, 1H), 3.83 - 3.76 (m, 1H), 3.58 (dd, *J* = 10.7, 8.3 Hz, 2H), 3.47 (br, 1H), 3.27 - 3.18 (m, 1H), 2.93 (p, *J* = 6.7 Hz, 1H), 1.66 - 1.58 (m, 3H), 1.32 - 1.26 (m, 6H), 1.05 (d, *J* = 6.8 Hz, 3H). |
| **Example 46** | 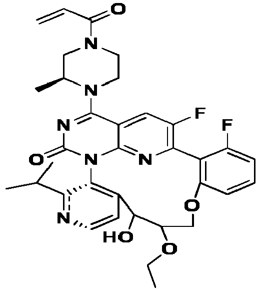 (*S*)-2⁴-((*S*)-4-acryloyl-2-methylpiperazin-1-yl)-6-ethoxy-2⁶,3⁶-difluoro-7-hydroxy-1²-isopropyl-2¹,2²-dihydro-4-oxa-2(1,7)-pyrido[2,3-*d*]pyrimidina-1(3,4)-pyridina-3(1,2)-benzocycloheptane-2²-one | ESI-MS 647 [M+1]⁺. |
| | | ¹H NMR (400 MHz, DMSO-*d₆*) δ 8.52 (d, *J =* 5.0 Hz, 1H), 8.31 - 8.25 (m, 1H), 8.03 (d, *J =* 5.1 Hz, 1H), 7.50 (td, *J =* 8.4, 6.8 Hz, 1H), 7.20 (d, *J =* 8.4 Hz, 1H), 7.03 (t, *J =* 8.8 Hz, 1H), 6.84 (dd, *J =* 16.1, 11.0 Hz, 1H), 6.20 (d, *J =* 16.6 Hz, 1H), 5.77 (dd, *J =* 10.4, 2.5 Hz, 1H), 5.58 (s, 1H), 4.80 (br, 1H), 4.46 - 4.38 (m, 3H), 4.28 - 4.08 (m, 2H), 3.96 (d, *J =* 3.7 Hz, 1H), 3.63 - 3.56 (m, 2H), 3.45 - 3.36 (m, 2H), 3.28 - 3.20 (m, 2H), 2.87 (p, *J =* 6.7 Hz, 1H), 1.41 (d, *J* = 6.6Hz, 3H), 1.17 (d, *J =* 6.6 Hz, 3H), 0.94 (t, *J =* 7.0 Hz, 3H), 0.84 (d, *J* = 6.8 Hz, 3H). |
| **Example 47** | 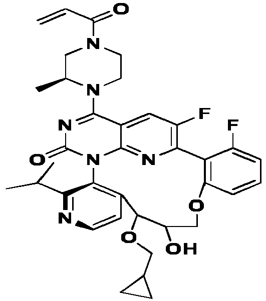 (*R*)-2⁴-((*S*)-4-acryloyl-2-methylpiperazin-1-yl)-7-(cyclopropylmethoxy)-2⁶,3⁶-difluoro-6-hydroxy-1²-isopropyl-2¹,2²-dihydro-4-oxa-2(1,7)-pyrido[2,3-d]pyrimidina-1(3,4)-pyridina-3 (1,2)-benzocycloheptane-2²-one | ESI-MS 673 [M+1]⁺. |
| | | ¹H NMR (400 MHz, CDCl₃) δ 8.63 (d, *J* = 5.1 Hz, 1H), 7.82 (d, *J =* 8.3 Hz, 1H), 7.69 (d, *J =* 5.2 Hz, 1H), 7.39 (td, *J=* 8.3, 6.5 Hz, 1H), 6.98 (d, *J =* 8.3 Hz, 1H), 6.85 (t, *J* = 8.6 Hz, 1H), 6.62 (br, 1H), 6.41 (dd, *J =* 16.8, 1.9 Hz, 1H), 5.82 (dd, *J* = 10.3, 2.0 Hz, 1H), 4.84 - 4.74 (m, 2H), 4.50 (br, 1H), 4.41 - 4.30 (m, 2H), 4.09 (d, *J* = 6.2 Hz, 1H), 4.10 - 4.00 (m, 1H), 3.78 - 3.39 (m, 2H), 3.44 (d, *J =* 5.7 Hz, 2H), 3.32 (dd, *J =* 10.2, 7.3 Hz, 1H), 3.19 (dd, *J =* 10.2, 6.7 Hz, 1H), 2.77 (p, *J =* 6.8 Hz, 1H), 1.30 - 1.36 (m, 7H), 0.98 (d, *J* = 6.8 Hz, 3H), 0.46 - 0.42 (m, 2H), 0.12 - 0.11 (m, 2H). |
| **Example 48** | 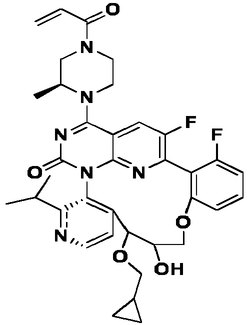 (*S*)-2⁴-((*S*)-4-acryloyl-2-methylpiperazin-1-yl)-7-(cyclopropylmethoxy)-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 673 [M+1]⁺. |
| | | ¹H NMR (400 MHz, CDCl₃) δ 8.63 (d, *J* = 5.1 Hz, 1H), 7.87 (d, *J =* 8.2 Hz, 1H), 7.68 (d, *J =* 5.1 Hz, 1H), 7.39 (q, *J =* 7.9 Hz, 1H), 6.98 (d, *J =* 8.5 Hz, 1H), 6.87 (d, *J* = 8.8 Hz, 1H), 6.61 (br, 1H), 6.42 (d, *J* = 16.6 Hz, 1H), 5.82 (d, *J =* 10.4 Hz, 1H), 5.42 - 4.71 (m, 2H), 4.36 - 4.33 (m, 2H), 4.22 (br, 1H), 4.15 - 4.06 (m, 1H), 3.98-3.91 (m, 2H), 3.77 (br, 1H), 3.44 (d, *J* = 5.7 Hz, 1H), 3.32 (dd, *J =* 10.2, 7.3 Hz, 1H), 3.19 (dd, *J =* 10.2, 6.7 Hz, 1H), 2.97 - 2.77 (m, 2H), 1.40 (d, *J =* 6.9 Hz, 3H), 1.33 - 1.26 (m, 4H), 0.97 - 0.94 (m, 3H), 0.45 - 0.43 (m, 2H), 0.12 - 0.11 (m, 2H). |
| **Example 49** | 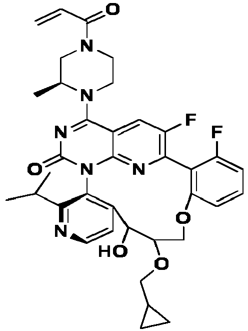 (*R*)-2⁴-((*S*)-4-acryloyl-2-methylpiperazin-1-yl)-6-(cyclopropylmethoxy)-2⁶,3⁶-difluoro-7-hydroxy-1²-isopropyl-2¹,2²-dihydro-4-oxa-2(1,7)-pyrido[2,3-*d*]pyrimidina-1(3,4)-pyridina-3 (1,2)-benzocycloheptane-2²-one | ESI-MS 673 [M+1]⁺. |
| | | ¹H NMR (400 MHz, CDCl₃) δ 8.68 (d, *J* = 4.8 Hz, 1H), 8.17 (s, 1H), 7.78 (d, *J=* 8.4 Hz, 1H), 7.38 (q, *J =* 7.9 Hz, 1H), 6.89 (d, *J =* 8.3 Hz, 1H), 6.84 (t, *J =* 8.6 Hz, 1H), 6.61 (br, 1H), 6.41 (dd, *J =* 16.7, 1.7 Hz, 1H), 5.82 (dd, *J =* 10.5, 1.8 Hz, 1H), 4.87 - 4.74 (m, 2H), 4.60 - 4.50 (m, 2H), 4.36 (d, *J =* 3.4 Hz, 1H), 4.21 - 3.84 (m, 2H), 3.73 (dd, *J =* 10.3, 7.1 Hz, 1H), 3.66 - 3.43 (m, 3H), 3.28 - 3.17 (m, 3H), 2.96 (s, 1H), 1.32 (d, *J =* 6.5 Hz, 3H), 1.27 (s, 3H), 1.15 - 1.09 (m, 4H), 0.58 (d, *J =* 7.8 Hz, 2H), 0.28 (d, *J =* 4.7 Hz, 2H). |
| **Example 50** | 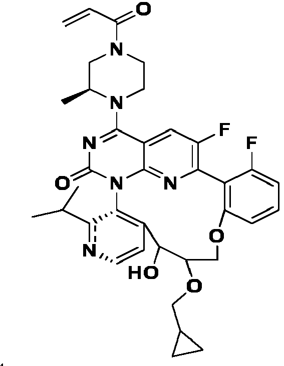 (*S*)-2⁴-((*S*)-4-aeryloyl-2-methylpiperazin-1-yl)-6-(cyclopropylmethoxy)-2⁶,3⁶-difluoro-7-hydroxy-1²-isopropyl-2¹,2²-dihydro-4-oxa-2(1,7)-pyrido[2,3-*d*]pyrimidina-1(3,4)-pyridina-3 (1,2)-benzocycloheptane-2²-one | ESI-MS 673 [M+1]⁺. |
| | | ¹H NMR (400 MHz, CDCl₃) δ 8.65 (d, *J* = 5.1 Hz, 1H), 8.10 (d, *J* = 5.2 Hz, 1H), 7.81 (d, *J =* 8.4 Hz, 1H), 7.37 (td, *J=* 8.4, 6.6 Hz, 1H), 6.90 (d, *J =* 8.3 Hz, 1H), 6.83 (t, *J =* 8.6 Hz, 1H), 6.61 (br, 1H), 6.42 (dd, *J =* 16.8, 1.9 Hz, 1H), 5.82 (dd, *J* = 10.4, 1.9 Hz, 1H), 5.56 - 5.07 (m, 2H), 4.79 - 4.61 (m, 1H), 4.51 (dd, *J =* 8.4, 4.7 Hz, 1H), 4.37 (d, *J =* 3.4 Hz, 1H), 4.21 (d, *J =* 13.2 Hz, 1H), 4.09 (dt, *J =* 10.7, 4.2 Hz, 1H), 4.03 - 3.84 (m, 1H), 3.72 (dd, *J* = 10.3, 7.0 Hz, 1H), 3.63 - 3.57 (m, 2H), 3.42 (br, 1H), 2.94 (s, 2H), 1.37 (d, *J =* 6.8 Hz, 3H), 1.30 (d, *J* = 6.7 Hz, 3H), 1.18 - 1.12 (m, 1H), 1.05 (d, *J =* 6.7 Hz, 3H), 0.63 - 0.55 (m, 2H), 0.29 - 0.26 (m, 2H). |
| **Example 51** | 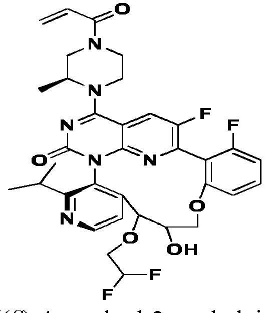 (*R*)-2⁴-((*S*)-4-acryloyl-2-metnylpiperazin-1-yl)-7-(2,2-difluoroethoxy)-2⁶,3⁶-difluoro-6-hydroxy-1²-isopropyl-2¹,2²-dihydro-4-oxa-2(1,7)-pyrido[2,3-*d*]pyrimidina-1(3,4)-pyridina-3 (1,2)-benzocycloheptane-2²-one | ESI-MS 683 [M+1]⁺. |
| | | ¹H NMR (400 MHz, CDCl₃) δ 8.67 (d, *J* = 5.2 Hz, 1H), 7.84 (d, *J* = 11.1 Hz, 1H), 7.77 (d, *J =* 2.6 Hz, 1H), 7.41 (td, *J=* 8.5, 6.6 Hz, 1H), 6.99 (d, *J* = 8.4 Hz, 1H), 6.88 (t, *J =* 8.6 Hz, 1H), 6.61 (br, 1H), 6.42 (dd, *J =* 16.6, 1.8 Hz, 1H), 5.90 - 5.61 (m, 2H), 4.80 - 4.75 (m, 2H), 4.46 (d, *J=* 2.6 Hz, 1H), 4.37 (dd, *J =* 8.8, 2.4 Hz, 1H), 4.27 - 4.24 (m, 1H), 4.02 - 3.98 (m, 2H), 3.84 - 3.54 (m, 4H), 3.53 (br, 1H), 3.30 (d, *J =* 5.3 Hz, 1H), 3.22 (br, 1H), 2.78 (p, *J* = 6.6 Hz, 1H), 1.32 - 1.26 (m, 6H), 1.00 (d, *J* = 6.6 Hz, 3H). |
| **Example 52** | 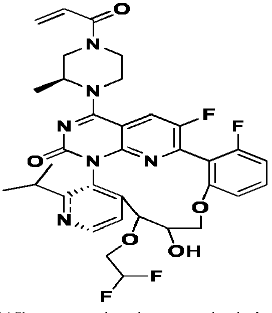 (*S*)-2⁴-((*S*)-4-acryloyl-2-methylpiperazin-1-yl)-7-(2,2-difluoroethoxy)-2⁶,3⁶-difluoro-6-hydroxy-1²-isopropyl-2¹,2²-dihydro-4-oxa-2(1,7)-pyrido[2,3-*d*]pyrimidina-1(3,4)-pyridina-3(1,2)-benzocycloheptane-2²-one | ESI-MS 683 [M+1]⁺. |
| | | ¹H NMR (400 MHz, CDCl₃) δ 8.68 (d, *J* = 5.3 Hz, 1H), 7.88 (d, *J* = 8.3 Hz, 1H), 7.82 (s, 1H), 7.41 (td, *J* = 8.4, 6.6 Hz, 1H), 6.98 (d, *J =* 8.7 Hz, 1H), 6.86 (t, *J =* 8.6 Hz, 1H), 6.61 (br, 1H), 6.42 (dd, *J* = 16.7, 1.9 Hz, 1H), 6.03 - 5.73 (m, 2H), 5.27 (s, 1H), 4.98 - 4.78 (m, 1H), 4.48 (d, *J =* 5.2 Hz, 1H), 4.44 - 4.36 (m, 1H), 4.26 (s, 2H), 3.05 - 3.97 (m, 2H), 3.84 - 3.73 (m, 2H), 3.70 - 3.58 (m, 1H), 3.38 (br, 1H), 3.29 (d, *J =* 5.1 Hz, 1H), 2.82 (s, 1H), 1.42 (d, *J* = 6.8 Hz, 3H), 1.34 (d, *J* = 6.7 Hz, 3H), 1.00 (s, 3H). |
| **Example 53** | 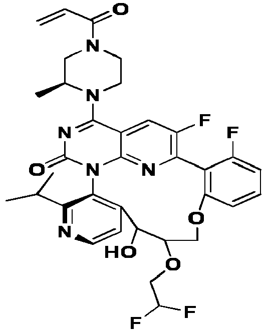 (*R*)-2⁴-((*S*)-4-acryloyl-2-methylpiperazin-1-yl)-6-(2,2-difluoroethoxy)-2⁶,3⁶-difluoro-7-hydroxy-1²-isopropyl-2¹,2²-dihydro-4-oxa-2(1,7)-pyrido[2,3-*d*]pyrimidina-1(3,4)-pyridina-3 (1,2)-benzocycloheptane-2²-one | ESI-MS 683 [M+1]⁺. |
| | | ¹H NMR (400 MHz, DMSO-*d₆*) δ 8.53 *(d, J =* 5.0 Hz, 1H), 8.25 (dd, *J* = 17.6, 9.1 Hz, 1H), 7.84 (d, *J =* 5.1 Hz, 1H), 7.60-7.45 (m, 1H), 7.20 (d, *J* = 8.3 Hz, 1H), 7.03 (t, *J =* 8.8 Hz, 1H), 6.94 - 6.79 (m, 1H), 6.45 - 6.05 (m, 2H), 5.77 (d, *J* = 10.4 Hz, 1H), 4.80 - 4.76 (m, 1H), 4.64 (dd, *J =* 8.5, 4.7 Hz, 1H), 4.52 - 4.37 (m, 2H), 4.27 - 4.21 (m, 2H), 4.16 - 4.07 (m, 2H), 4.02 - 3.94 (m, 3H), 3.61 - 3.51 (m, 2H), 2.91 (p, *J =* 6.7 Hz, 1H), 1.45 - 1.42 (m, 3H), 1.15 (d, *J =* 6.6 Hz, 3H), 0.87 (d, *J* = 6.7 Hz, 3H). |
| **Example 54** | 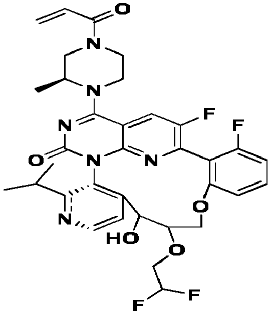 (*S*)-2⁴-((*S*)-4-aeryloyl-2-methylpiperazin-1-yl)-6-(2,2-difluoro ethoxy)-2⁶,3⁶-difluoro-7-hydroxy-1²-isopropyl-2¹,2²-dihydro-4-oxa-2(1,7)-pyrido[2,3-*d*]pyrimidina-1(3,4)-pyridina-3(1,2)-benzocycloheptane-2²-one | ESI-MS 683 [M+1]⁺. |
| | | ¹H NMR (400 MHz, CDCl₃) δ 8.70 (s, 1H), 8.08 (s, 1H), 7.83 - 7.81 (d, *J* = 5.1 Hz, 1H), 7.40 - 7.38 (m, 1H), 6.90 - 6.84 (m, 2H), 6.61 (br, 1H), 6.41 (dd, *J=* 16.7, 1.8 Hz, 1H), 6.12 - 5.82 (m, 2H), 4.55-4.51 (m, 1H), 4.40 (s, 1H), 4.26 - 4.18 (m, 3H), 3.99 - 3.95 (m, 2H), 3.64 (br, 1H), 3.62 (ddd, *J=* 10.2, 8.1, 1.5 Hz, 1H), 3.41 - 3.26 (m, 4H), 2.99 - 2.90 (m, 2H), 1.39 - 1.26 (m, 6H), 1.12 (s, 3H). |
| **Example 55** | 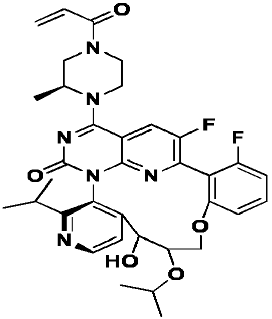 (*R*)-2⁴-((*S*)-4-acryloyl-2-methylpiperazin-1-yl)-6-isopropoxy-2⁶,3⁶-difluoro-7-hydroxy-1²-isopropyl-2¹,2²-dihydro-4-oxa-2(1,7)-pyrido[2,3-*d*]pyrimidina-1(3,4)-pyridina-3(1,2)-benzocycloheptane-2²-one | ESI-MS 661 [M+1]⁺. |
| | | ¹H NMR (400 MHz, DMSO-*d₆*) δ 8.51 (d*, J =* 5.1 Hz, 1H), 8.25 (dd, *J* = 17.3, 9.2 Hz, 1H), 7.94 (d, *J =* 5.1 Hz, 1H), 7.49 (dd, *J =* 15.3, 8.3 Hz, 1H), 7.20 (d, *J =* 8.4 Hz, 1H), 7.01 (t, *J* = 8.8 Hz, 1H), 6.87 (dd, *J* = 26.6, 16.1 Hz, 1H), 6.21 (dd, *J =* 17.3, 3.5 Hz, 1H), 5.77 (d, *J =* 10.4 Hz, 1H), 4.97 (d, *J =* 8.3 Hz, 1H), 4.77 (d, *J =* 16.1 Hz, 1H), 4.50 (dd, *J =* 8.4, 4.8 Hz, 2H), 4.32 (dd, *J* = 54.1, 13.5 Hz, 1H), 4.14 (dd, *J =* 8.2, 3.5 Hz, 1H), 4.00 (d, *J* = 13.8 Hz, 1H), 3.92 - 3.82 (m, 2H), 3.69 - 3.48 (m, 2H), 3.21 - 3.18 (m, 1H), 2.91 (dt, *J =* 13.3, 6.6 Hz, 2H), 1.26 - 1.23 (m, 3H), 1.18 (d, *J =* 6.1 Hz, 3H), 1.15 (dd, *J* = 6.3, 2.7 Hz, 6H), 0.87 (d, *J* = 6.7 Hz, 3H). |
| **Example 56** | 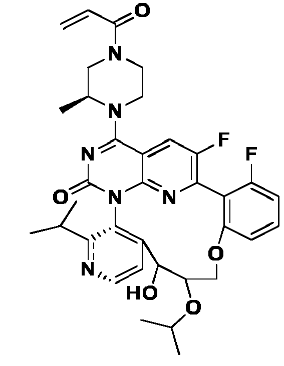 (*S*)-2⁴-((*S*)-4-acryloyl-2-methylpiperazin-1-yl)-6-isopropoxy-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 661 [M+1]⁺. |
| | | ¹H NMR (400 MHz, DMSO-*d₆*) δ 8.51 (d, *J =* 5.1 Hz, 1H), 8.46 (d, *J =* 9.1 Hz, 1H), 7.94 (d, *J =* 5.1 Hz, 1H), 7.49 (dd, *J* = 15.4, 8.3 Hz, 1H), 7.20 (d, *J =* 8.3 Hz, 1H), 7.01 (t, *J =* 8.8 Hz, 1H), 6.94 - 6.82 (m, 1H), 6.21 (dd, *J =* 17.0, 5.0 Hz, 1H), 5.77 (dd, *J* = 10.4, 2.3 Hz, 1H), 5.12 (s, 1H), 4.99 (d, *J =* 5.8 Hz, 1H), 4.50 (dd, *J* = 8.5, 4.8 Hz, 1H), 4.46 - 4.00 (m, 4H), 3.89 - 3.83 (m, 3H), 3.68 (d, *J =* 12.0 Hz, 1H), 3.03 - 2.83 (m, 2H), 1.26 - 1.24 (m, 4H), 1.18 (d, *J* = 6.1 Hz, 3H), 1.15 (d, *J* = 6.3 Hz, 5H), 0.87 (d, *J=* 6.7 Hz, 3H). |
| **Example 57** | 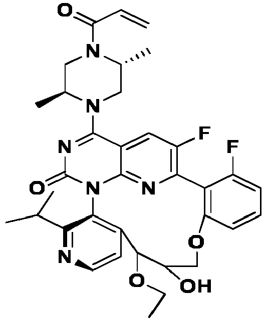 (R)-2⁴-((2S,5R)-4-acryloyl-2,5-dimethylpiperazin-1-yl)-7-ethoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3 (1,2)-benzocycloheptane-2²-one | ESI-MS 661 [M+1]⁺. |
| | | ¹H NMR (400 MHz, DMSO-*d₆*) δ 8.51 (d, *J =* 5.0 Hz, 1H), 8.27 - 8.17 (m, 1H), 8.02 (d, *J =* 4.9 Hz, 1H), 7.57 - 7.43 (m, 1H), 7.21 (d, *J* = 8.3 Hz, 1H), 7.03 (t, *J=* 8.9 Hz, 1H), 6.92 - 6.75 (m, 1H), 6.18 (d, *J =* 16.6 Hz, 1H), 5.75 (d, *J =* 10.2 Hz, 1H), 5.57 (t, *J =* 5.8 Hz, 1H), 4.92 - 4.35 (m, 4H), 4.32 - 4.12 (m, 2H), 4.01 (d, *J* = 3.8 Hz, 1H), 3.87 - 3.65 (m, 2H), 3.50-3.36 (m, 2H), 3.30 - 3.20 (m, 1H), 2.95-2.80 (m, 1H), 1.40 (d, *J =* 6.6 Hz, 3H), 1.36 - 1.12 (m, 6H), 0.93 (t, *J =* 6.9 Hz, 3H), 0.83 (t, *J* = 6.4 Hz, 3H). |
| **Example 58** | 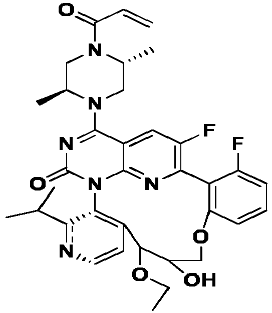 | ESI-MS 661 [M+1]⁺. |
| | | ¹H NMR (400 MHz, DMSO-*d₆*) δ 8.52 (d, *J =* 5.1 Hz, 1H), 8.43 (d, *J =* 9.2 Hz, 1H), 7.99 (d, *J* = 5.2 Hz, 1H), 7.55 - 7.45 (m, 1H), 7.21 (d, *J* = 8.3 Hz, 1H), 7.03 (t, *J* = 8.9 Hz, 1H), 6.90 - 6.77 (m, 1H), 6.19 (d, *J =* 16.6 Hz, 1H), 5.79 - 5.71 (m, 1H), 5.50 (t, *J =* 5.4 Hz, 1H), 5.00 - 4.65 (m, 2H), 4.52 - 4.35 (m, 1H), 4.30 - 3.80 (m, 6H), 3.65 - 3.45 (m, 3H), 2.85 - 2.75 (m, 1H), 1.43 - 1.07 (m, 9H), 0.94 (t, *J =* |
| | (S)-2⁴-((2S,5R)-4-acryloyl-2,5-dimethylpiperazin-1-yl)-7-ethoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | 6.9 Hz, 3H), 0.81 (d, *J* = 6.7 Hz, 3H). |

### Example 59: Preparation of 2⁴-((S)-4-acryloyl-2-methylpiperazin-1-yl)-2⁶,3⁶-difluoro-1²-isopropyl-6,7-dimethoxy-2¹,2²-dihydro-4-oxa-2(1,7)-pyrido[2,3-d]pyrimidina-1(3,4)-pyridina-3(1,2)-benzocycloheptane-2²-one

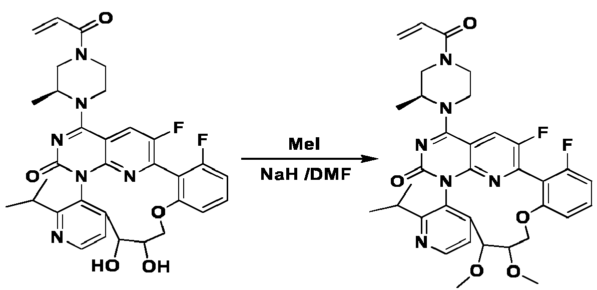

2⁴-((S)-4-acryloyl-2-methylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one (60 mg, 0.09 mmol) was dissolved in DMF (5 mL), and sodium hydride (7 mg, 0.18 mmol) was added under the protection of nitrogen. The mixture was stirred for 20 minutes at room temperature, and added with dichloromethane-diluted iodomethane (25 mg, 0.18 mmol) to react for 2 hrs at room temperature. The reaction was stopped, ammonium chloride in water was added under an ice bath for quenching, and ethyl acetate (15 mL ^{∗} 2) was added for extraction. The organic phases were combined, washed with saline water (20 mL ^{∗} 2), dried over anhydrous sodium sulfate, and concentrated under reduced pressure to remove the solvent. The residue was separated by reversed-phase chromatographic columns to obtain 2⁴-((S)-4-acryloyl-2-methylpiperazin-1-yl)-2⁶,3⁶-difluoro-1²-isopropyl-6,7-dimethoxy-2¹,2²-dihydro-4-oxa-2(1,7)-pyrido[2,3-d]pyrimidina-1(3,4)-pyridina-3(1,2)-benzocycloheptane-2²-one (30 mg, yield: 69%). ESI-MS 647[M+1]⁺.

¹H NMR (400 MHz, CDCl₃) δ 8.63 (d, *J=* 5.2 Hz, 1H), 7.99 (s, 1H), 7.86 (d, *J=* 10.2 Hz, 1H), 7.39 (td, *J=* 8.3, 6.5 Hz, 1H), 6.96 (d, *J=* 8.3 Hz, 1H), 6.88 (t, *J=* 8.7 Hz, 1H), 6.70 - 6.53 (m, 1H), 6.40 (d, *J* = 16.7 Hz, 1H), 5.81 (dd, *J* = 10.3, 2.0 Hz, 1H),5.3-4.95(m, 1H), 4.82 - 4.65 (m, 1H), 4.58 - 4.43 (m, 1H), 4.30 (dd, *J* = 9.1, 3.5 Hz, 1H), 4.15 (d, *J* = 8.8 Hz, 1H), 4.05-3.95(m, 1H), 3.89 (dd, *J* = 9.1, 4.2 Hz, 2H),3.8-3.7(m, 1H), 3.62 (s, 3H), 3.6-3.5(m, 2H),3.28 (s,3H), 2.90 - 2.76 (m, 1H),1.52(d,J=6.8,1.5H), 1.43 (d,J=6.8 1.5H), 1.34 (d, *J=* 6.6 Hz, 3H), 0.98 (d, *J=* 7.0 Hz, 3H).

**Examples 60-61 can be prepared by selecting corresponding starting materials according to the complete or partial synthesis method of Example 59:**

| **Example No.** | **Structural Formula/Chemical Name** | **MS m/z [M+1]⁺ / ¹H NMR(400 MHz)** |
|---|---|---|
| **Example 60** | 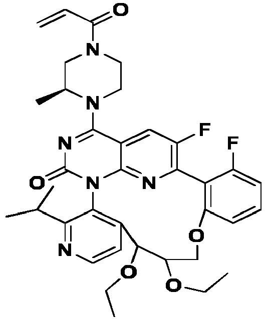 2⁴-((S)-4-acryloyl-2-methylpiperazin-1-yl)-6,7-diethoxy-2⁶,3⁶-difluoro-1²-isopropyl-2¹,2²-dihydro-4-oxa-2(l,7)-pyrido[2,3-d]pyrimidina-1 (3,4)-pyridina-3(1,2)-benzocycloheptane-2²-one | ESI-MS 675[M+1]⁺. |
| | | ¹H NMR (400 MHz, CDCl₃) δ8.44 (d, *J* = 8 Hz, 1H), 8.28 - 8.12 (m, 1H), 8.04 (d, *J* = 4 Hz, 1H), 7.39 - 7.33 (m, 1H), 7.01 (d, *J* = 8 Hz, 1H), 6.83 - 6.67 (m, 2H), 6.24 (d, *J* = 16 Hz, 1H), 5.76 - 5.73 (m, 1H), 5.18 - 4.78 (m, 1H),4.58 - 4.26 (m, 4H), 4.14 - 3.90 (m, 4H), 3.80 - 3.60 (m, 3H), 3.43 - 3.33 (m, 3H), 2.88 - 2.84(m, 1H),1.49 - 1.28(m, 3H), 1.20 - 1.14(m, 6H), 0.97 (t, *J* = 8 Hz, 3H), 0.85 (d, *J* = 8 Hz, 3H). |
| **Example 61** | 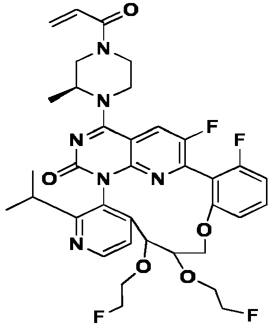 2⁴-((S)-4-acryloyl-2-methylpiperazin-1-yl)-2⁶,3⁶-difluoro-6,7-di(2-fluoroethoxy)-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 711[M+1]⁺. |
| | | ¹H NMR (400 MHz, CDCl₃) δ 8.64 (d, *J =* 5.1 Hz, 1H), 8.03 (dd, *J =* 5.1, 2.7 Hz, 1H), 7.82 (dd, *J =* 12.8, 8.3 Hz, 1H), 7.38 (td, *J* = 8.4, 6.5 Hz, 1H), 6.95 (d, *J =* 8.3 Hz, 1H), 6.86 (t, *J* = 8.7 Hz, 1H), 6.68 - 6.54 (m, 1H), 6.40 (d, *J =* 16.6 Hz, 1H), 5.81 (dd, *J =* 10.4, 1.9 Hz, 1H), 4.72 (dd, *J =* 12.7, 5.7 Hz, 1H), 4.66 - 4.57 (m, 1H), 4.55 - 4.46 (m, 2H), 4.43 - 4.34 (m, 3H), 4.25 (d, *J =* 12.0 Hz, 1H), 4.16 (dt, *J =* 10.0, 3.8 Hz, 2H), 4.07-3.92 (m, 2H), 3.85-3.8 (m,1H), 3.74 (d, *J* = 12.5 Hz, 1H), 3.62 (t, *J =* 9.4 Hz, 2H), 3.55-3.45(m, 2H),3.38 - 3.18 (m, 1H), 2.88 - 2.79 (m, 1H), 1.51(d,J=6.8Hz, 1H),1.41 (d, *J* = 6.8 Hz, 2H), 1.33 (dd, *J =* 6.8, 3H), 0.95 (d, *J* = 7.2 Hz, 3H). |

### Example 62: Preparation of 11-((S)-4-acryloyl-2-methylpiperazin-1-yl)-15,21-difluoro-7-isopropyl-3a,20a-dihydro-9H,20H-12,14-(epiethane[1,2]diylidene)benzo[b] [1,3]dioxazolo[4,5-j]pyrido[3,4-h]pyrimido[6,1-f][1]oxa[5,7]diazacyclodidecyne-2,9-dione

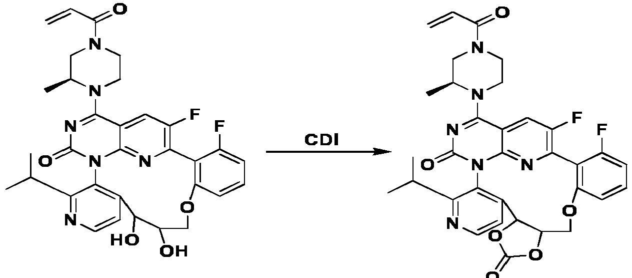

N,N-diisopropylethylamine (57.3 mg, 0.445 mmol) and N,N'-carbonyldiimidazole (17.3 mg, 0.11 mmol) were added to 2⁴-((S)-4-acryloyl-2-methylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one (55 mg, 0.089 mmol) in tetrahydrofuran solution (15 mL), which was stirred for 5 hrs at room temperature and then added with 1,8-diazabicyclo[5.4.0]undecane-7-ene (0.013 mL, 0.089 mmol). The reaction solution was heated to 70°C, at which the reaction was conducted for half an hour. After complete reaction was detected, reverse preparation and separation was conducted to obtain a product 11-((S)-4-acryloyl-2-methylpiperazin-1-yl)-15,21-difluoro-7-isopropyl-3a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclodidecyne-2,9-dione (28 mg, yield: 47%). ESI-MS 645 [M+1]⁺.

¹H NMR (400 MHz, DMSO-*d₆*) δ 8.61 (d, *J* = 5.1 Hz, 1H), 8.46 - 8.20 (m, 1H), 7.50 - 7.40 (m, 1H), 7.29 (d, *J* = 5.0 Hz, 1H), 7.12 (d, *J* = 8.3 Hz, 1H), 7.05 (t, *J* = 8.8 Hz, 1H), 6.87 - 6.72 (m, 1H), 6.14 (d, *J* = 16.7 Hz, 1H), 5.84 (t, *J* = 7.7 Hz, 1H), 5.71 (d, *J* = 12.0 Hz, 1H), 5.10 - 4.73 (m, 2H), 4.62 - 4.53 (m, 1H), 4.48 - 3.50 (m, 7H), 2.83 - 2.71 (m, 1H), 1.29 (dd, *J* = 47.6, 6.5 Hz, 3H), 1.06 (d, *J* = 6.6 Hz, 3H), 0.77 (d, *J* = 6.3 Hz, 3H).

### Example 63: Preparation of 11-((S)-4-acryloyl-2-methylpiperazin-1-yl)-15,21-difluoro-7-isopropyl-2,2-dimethyl-3a,20a-dihydro-9H,20H-12,14-(eiethane[1,2]diylidene)benzo[b] [1,3]dioxazolo[4,5-j]pyrido[3,4-h]pyrimido[6,1-f][1]oxa[5,7]diazacyclododecin-9-one

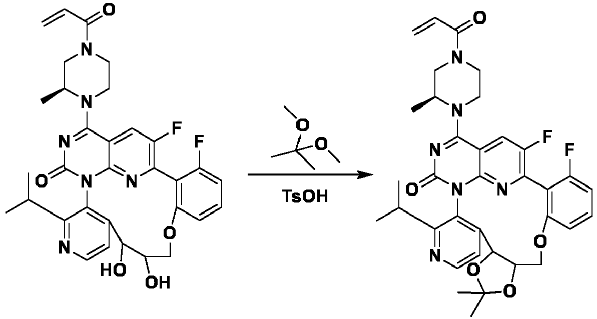

P-methylbenzenesulfonic acid (15 mg, 0.079 mmol) and 2,2-dimethoxypropane (1 mL, 8.06 mmol) were added to 2⁴-((S)-4-acryloyl-2-methylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one (55 mg, 0.089 mmol) in acetone solution (10 mL). The mixture was heated to 50°C, and then reacted for 10 hrs at the current temperature. After the reaction was detected to be basically completed, the resultant was concentrated, and a crude product was reversely prepared to obtain a product 11-((S)-4-acryloyl-2-methylpiperazin-1 -yl)-15,21 -difluoro-7 -isopropyl -2,2-dimethyl-3 a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclododecin-9-one (29.9 mg, yield: 51%). ESI-MS 659[M+1]⁺.

¹H NMR (400 MHz, DMSO-*d₆*) δ 8.58 (d, *J=* 5.1 Hz, 1H), 8.49 - 8.24 (m, 1H), 7.53 - 7.45(m, 1H), 7.30 (dd, *J* = 5.2, 2.2 Hz, 1H), 7.19 (dd, *J* = 8.3, 2.6 Hz, 1H), 7.07 (t, *J* = 8.8 Hz, 1H), 6.96-6.79 (m, 1H), 6.22 (d, *J=* 17.1 Hz, 1H), 5.77 (dd, *J* = 10.5, 2.4 Hz, 1H), 5.15 - 4.81 (m, 2H), 4.57-4.12 (m, 5H), 4.10 - 3.75 (m, 1H), 3.74 - 3.50 (m, 3H), 2.84 - 2.74 (m, 1H), 1.54 (s, 3H), 1.46-1.26 (m, 6H), 1.12 (d, *J* = 6.7 Hz, 3H), 0.80 (dd, *J=* 6.9, 3.1 Hz, 3H).

### Example 64: Preparation of 11-((S)-4-acryloyl-2-methylpiperazin-1-yl)-2-((dimethylamino)methyl)-15,21-difluoro-7-isopropyl-3a,20a-dihydro-9H,20H-12,14-(epiethane[1,2]diylidene)benzo[b] [1,3]dioxazolo[4,5-j]pyrido[3,4-h]pyrimido[6,1-f][1]oxa[5,7]diazacyclododecin-9-one

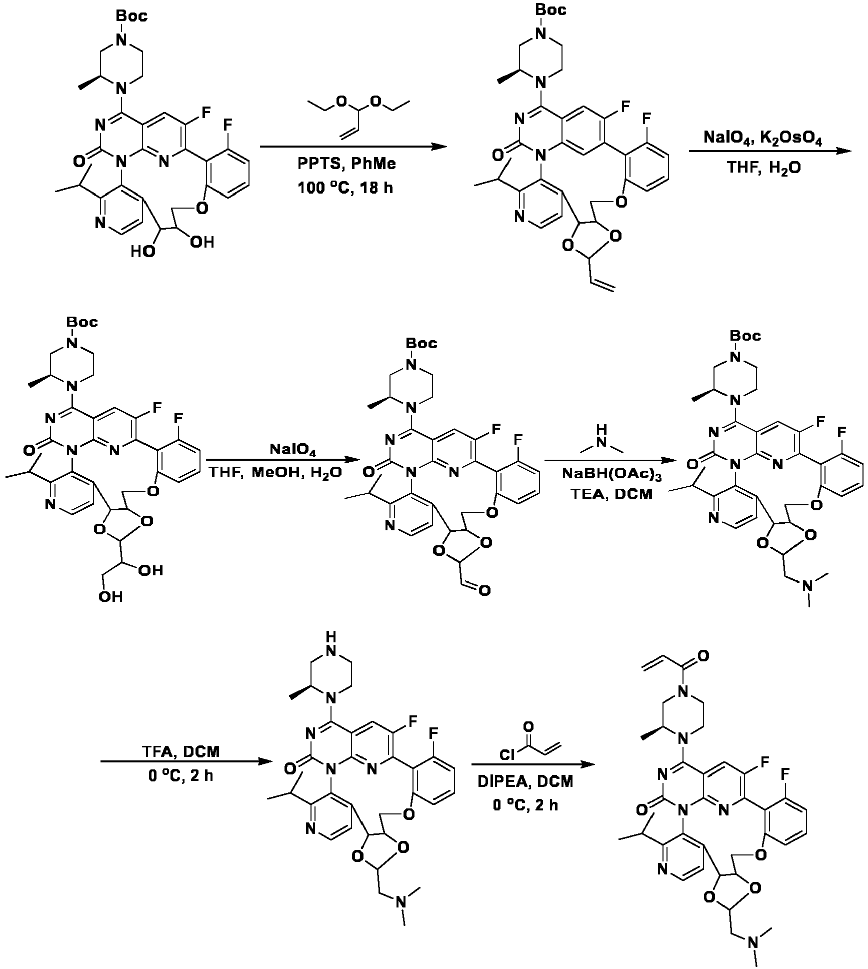

### Step 1: Synthesis of tert-butyl (3S)-4-(15,21-difluoro-7-isopropyl-9-oxo-2-vinyl-3a,20a-dihydro-9H,20H-12,14-(epiethane[1,2]diylidene)benzo[b][1,3]dioxazolo[4,5-j]pyrido[3,4-h]pyrimido[6,1-f][1]oxa[7]azacyclododecin-11-yl)-3-methylpiperazine-1-carboxylate

Tert-butyl (3S)-4-(2⁶,3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-3-methylpiperazine-1-carboxylate (1000 mg, 1.51 mmol) was dissolved in toluene (20 mL), and pyridine p-toluenesulfonate (37.8 mg, 0.15 mmol) and acrolein diethylacetal (1560 mg, 12.0 mmol) were added. Under the protection of nitrogen, the reaction was conducted overnight at 100°C. The reaction solution was diluted with ethyl acetate (100 mL), washed with water (100 mL) and saturated sodium chloride aqueous solution (100 mL). The organic phase was dried over anhydrous sodium sulfate, and then concentrated under reduced pressure to remove the solvent. The residue was separated with flash silicagel columns to obtain tert-butyl (3S)-4-(15,21-difluoro-7-isopropyl-9-oxo-2-vinyl-3a,20a-dihydro-9H,20H-12,14-(epiethane[1,2]diylidene)benzo[b][1,3]dioxazolo[4,5-j]pyrido[3,4-h]pyrimido[6,1-f][1]oxa[7]azacyclododecin-11-yl)-3-methylpiperazine-1-carboxylate (860 mg, yield: 81%). ESI-MS 703 [M+1]⁺.

### Step 2: Synthesis of tert-butyl (3S)-4-(2-(1,2-dihydroxyethyl)-15,21-difluoro-7-isopropyl-9-oxo-3a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclododecin-11-yl)-3-methylpiperazine-1-carboxylate

Tert-butyl (3S)-4-(15,21-difluoro-7-isopropyl-9-oxo-2-vinyl-3a,20a-dihydro-9H,20H-12,14-(epiethane[1,2]diylidene)benzo[b][1,3]dioxazolo[4,5 j]pyrido[3,4-h]pyrimido[6,1-f][1]oxa[7]azacyclododecin-11-yl)-3-methylpiperazine-1-carboxylate (400 mg, 0.569 mmol) was dissolved in tetrahydrofuran (30 mL) and water (10 mL). At 0°C, sodium periodate (243 mg, 1.14 mmol) and potassium osmate dihydrate (52.4 mg, 0.142 mmol) were added respectively. The reaction solution was slowly warmed up to room temperature and stirred overnight. The reaction solution was diluted with water (20 mL), and extracted with ethyl acetate (30 mL). The organic phase was dried over anhydrous sodium sulfate, and then concentrated under reduced pressure to remove the solvent. The residue was separated with flash silicagel columns to obtain tert-butyl (3S)-4-(2-(1,2-dihydroxyethyl)-15,21-difluoro-7-isopropyl-9-oxo-3a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclododecin-11-yl)-3-methylpiperazine-1-carboxylate (600 mg). Two portions were added in total, and the combined yield was 72%. ESI-MS 737 [M+1]⁺.

### Step 3: Synthesis of tert-butyl (3S)-4-(15,21-difluoro-2-formyl-7-isopropyl-9-oxo-3a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclododecin-11-yl)-3-methylpiperazine-1-carboxylate

Tert-butyl (3S)-4-(2-(1,2-dihydroxyethyl)-15,21-difluoro-7-isopropyl-9-oxo-3a,20a-dihydro-9H,20H-12,14-(epiethane[1,2]diylidene)benzo[b][1,3]dioxazolo[4,5 j]pyrido[3,4-h]pyrimido[6,1-f][1]oxa[5,7]diazacyclododecin-11-yl)-3-methylpiperazine-1-carboxylate (300 mg, 0.407 mmol) was dissolved in the mixed solvent of tetrahydrofuran (5 mL), methanol (5 mL), and water (5 mL). Sodium periodate (696 mg, 3.26 mmol) was added. The resultant was stirred overnight at room temperature. The reaction solution was diluted with water (20 mL), and extracted with ethyl acetate (30 mL). The organic phase was dried over anhydrous sodium sulfate, and then concentrated under reduced pressure to remove the solvent. The residue was separated with flash silicagel columns to obtain tert-butyl (3S)-4-(15,21-difluoro-2-formyl-7-isopropyl-9-oxo-3a,20a-dihydro-9H,20H-12,14-(epiethane[1,2]diylidene)benzo[b][1,3]dioxazolo[4,5 j]pyrido[3,4-h]pyrimido[6,1-f][1]oxa[5,7]diazacyclododecin-11-yl)-3-methylpiperazine-1-carboxylate (240 mg, yield: 84%). ESI-MS 723 [M+1]⁺.

### Step 4: Synthesis of tert-butyl (3S)-4-(2-((dimethylamino)methyl)-15,21-difluoro-7-isopropyl-9-oxo-3a,20a-dihydro-9H,20H-12,14-(epiethane[1,2]diylidene)benzo[b] [1,3]dioxazolo[4,5-j]pyrido[3,4-h]pyrimido[6,1-f][1]oxa[5,7]diazacyclododecin-11-yl)-3-methylpiperazine-1-carboxylate

Tert-butyl (3S)-4-(15,21-difluoro-2-formyl-7-isopropyl-9-oxo-3a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclododecin-11-yl)-3-methylpiperazine-1-carboxylate (190 mg, 0.270 mmol) was dissolved in anhydrous dichloromethane (10 mL), and dimethylamine (2 M) in tetrahydrofuran solution (0.135 mL, 0.270 mmol), triethylamine (81.8 mg, 0.809 mmol), and triacetoxyl sodium borohydride (171 mg, 0.809 mmol) were added respectively. The resultant was stirred overnight at room temperature. The reaction solution was directly concentrated under reduced pressure to remove the solvent. The residue was separated with flash silicagel columns to obtain tert-butyl (3S)-4-(2-((dimethylamino)methyl)-15,21-difluoro-7-isopropyl-9-oxo-3a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclododecin-11-yl)-3-methylpiperazine-1-carboxylate (120 mg, yield: 61%). ESI-MS 734 [M+1]⁺.

### Step 5: Synthesis of 2-((dimethylamino)methyl)-15,21-difluoro-7-isopropyl-11-((S)-2-methylpiperazin-1-yl)-3a,20a-dihydro-9H,20H-12,14-(epiethane[1,2]diylidene)benzo[b] [1,3]dioxazolo[4,5-j]pyrido[3,4-h]pyrimido[6,1-f][1]oxa[5,7]diazacyclododecin-9-one

Tert-butyl (3S)-4-(2-((dimethylamino)methyl)-15,21-difluoro-7-isopropyl-9-oxo-3a,20a-dihydro-9H,20H-12,14-(epiethane[l,2]diylidene)benzo[b][1,3]dioxazolo[4,5-j]pyrido[3,4-h]pyrimido[6,1-f][1]oxa[5,7]diazacyclododecin-11-yl)-3-methylpiperazine-1-carboxylate (120 mg, 0.164 mmol) was dissolved in dichloromethane (5 mL). The solution was cooled to 0°C, and under the protection of nitrogen, trifluoroacetic acid (1 mL) was dropwise added. The reaction was conducted for 2 hrs at 0°C. The reaction solution was directly concentrated under reduced pressure to remove the solvent to obtain a crude product, which was directly used in the next step of the reaction. ESI-MS 634 [M+1]⁺.

### Step 6: Synthesis of 11-((S)-4-acryloyl-2-methylpiperazin-1-yl)-2-((dimethylamino)methyl)-15,21-difluoro-7-isopropyl-3a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclododecin-9-one

2-((dimethylamino)methyl)-15,21-difluoro-7-isopropyl-11-((S)-2-methylpiperazin-1-yl)-3a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclododecin-9-one (100 mg, 0.158 mmol) was dissolved in anhydrous dichloromethane (5 mL), and N,N-diisopropylethylamine (85.6 mg, 0.66 mmol) was added. The mixture was cooled to 0°C, and under the protection of nitrogen, prop-2-enoyl chloride (14.3 mg, 0.158 mmol) was dropwise added to allow reaction for 2 hrs. The reaction solution was diluted with ethyl acetate (20 mL), and washed with saturated sodium chloride aqueous solution (30 mL). The organic phase was dried over anhydrous sodium sulfate, and then concentrated under reduced pressure to remove the solvent. The residue was separated with reversed-phase columns to obtain 11-((S)-4-acryloyl-2-methylpiperazin-1-yl)-2-((dimethylamino)methyl)-15,21-difluoro-7-isopropyl-3a,20a-dihydro-9H,20H-12,14-(epiethane[1,2]diylidene)benzo[b][1,3]dioxazolo[4,5-j]pyrido[3,4-h]pyrimido[6,1-f][1]oxa[5,7]diazacyclododecin-9-one (42 mg, yield: 37%, purity: 96.0%). ESI-MS 688 [M+1]⁺.

¹H NMR (400MHz, DMSO-*d₆*) δ8.61 - 8.55 (m, 1H), 8.46 - 8.38 (m, 1H), 8.33 (br d, *J=* 10.1 Hz, 1H), 7.55 - 7.45 (m, 1H), 7.33 - 7.26 (m, 1H), 7.21 (d, *J=* 8.3 Hz, 1H), 7.08 (t, *J=* 8.9 Hz, 1H), 6.87 (br d, *J=* 5.6 Hz, 1H), 6.22 (br d, *J=* 17.6 Hz, 1H), 5.78 (br d, *J=* 12.5 Hz, 1H), 5.48 (t, *J=* 4.5 Hz, 1H), 5.06 (br s, 1H), 4.98 (t, *J*=4.3 Hz, 1H), 4.95 - 4.86 (m, 1H), 4.55 (br d, *J* = 4.3 Hz, 1H), 4.50 - 4.38 (m, 1H), 4.35 - 4.25 (m, 1H), 4.18 (br d, *J* = 7.4 Hz, 1H), 4.04 (br t, *J* = 15.1 Hz, 1H), 3.83 (br s, 1H), 3.73 - 3.55 (m, 2H), 2.81 (br s, 1H), 2.66 - 2.60 (m, 1H), 2.25 - 2.19 (m, 6H), 1.41 (br s, 1H), 1.31 (br d, *J=* 6.1 Hz, 2H), 1.12 (d, *J=* 6.6 Hz, 3H), 0.81 (br d, *J=* 6.1 Hz, 3H).

### Example 65: Preparation of (R)-2⁴-((2S,SR)-4-acryloyl-2,5-dimethylpiperazin-1-yl)-3⁵-chloro-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one

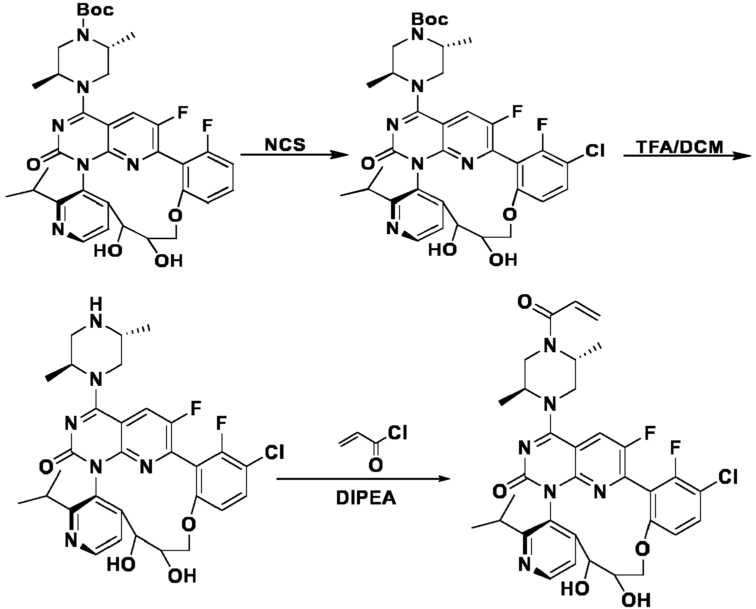

### Step 1: Synthesis of (R)-tert-butyl (2R,5S)-4-(3⁵-chloro-2⁶,3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-2,5-dimethylpiperazine-1-carboxylate

N-chlorosuccinimide (278.6 mg,2.1 mmol) was added to (R)-tert-butyl (2R, 5S)-4-(3⁵-chloro-2⁶3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-2,5-dimethylpiperazine-1-carboxylate (440 mg, 0.7 mmol) in acetonitrile solution (40 mL). Then, the mixture was heated to 70°C to react for 6 hrs at the current temperature. The reaction was stopped, and the concentrated residue was separated by flash silicagel columns to obtain a product (R)-tert-butyl (2R,5S)-4-(3⁵-chloro-2⁶,3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-2,5-dimethylpiperazine-1-carboxylate (450 mg, yield: 76%). ESI-MS 713 [M+1]⁺.

### Step 2: Synthesis of (R)-3⁵-chloro-2⁴-((2S,SR)-2,5-dimethylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one

**(R)-**tert-butyl (2R, 5S)-4-(3⁵-chloro-2⁶,3⁶-difluoro-6,7-dihydroxy-1²-isopropyl-2²-oxo-2¹,2²-dihydro-4-oxa-2(1,7)-pyrido[2,3-d]pyrimidina-1(3,4)-pyridina-3(1,2)-benzocycloheptane-2⁴-yl)-2,5-dimethylpiperazine-1-carboxylate (450.0 mg, 0.63 mmol) was dissolved in 5 mL of dichloromethane and 2 mL of trifluoroacetic acid. The mixture was stirred at room temperature to react for 1.5 hrs. The reaction was stopped, and the resultant was concentrated under reduced pressure to remove the solvent to obtain crude (R)-3⁵-chloro-2⁴-((2S,SR)-2,5-dimethylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one (400 mg), which was directly used in the next step. ESI-MS 613 [M+1]⁺.

### Step 3: Synthesis of (R)-2⁴-((2S,SR)-4-acryloyl-2,5-dimethylpiperazin-1-yl)-3⁵-chloro-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one

(R)-3⁵-chloro-2⁴-((2S,SR)-2,5-dimethylpiperazin-1-yl)-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one (368 mg, 0.6 mmol) was dissolved in dichloromethane (15 mL), and N,N-diisopropylethylamine (1.0 mL) was added. The mixture was cooled to 0°C, and acryloyl chlorine (60.0 mg, 0.66 mmol) was added. During the reaction, the mixture was naturally warmed up to room temperature and stirred to react for 1.5 hrs. The reaction was stopped, and dichloromethane (30 mL) was added for dilution. The resultant was washed with saline water (20 mL * 2), and the organic phase was separated, then dried over anhydrous sodium sulfate, and concentrated under reduced pressure to remove the solvent. The residue was separated by flash silicagel columns and reversed-phase chromatographic columns to obtain (R)-2⁴-((2S,SR)-4-acryloyl-2,5-dimethylpiperazin-1-yl)-3⁵-chloro-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one (216 mg, yield: 51%). ESI-MS 667 [M+1]⁺.

¹H NMR (400 MHz, DMSO-*d₆*) δ 8.50 (d, *J=* 5.0 Hz, 1H), 8.17 (dd, *J=* 14.0, 9.2 Hz, 1H), 8.01 (d, *J=* 5.1 Hz, 1H), 7.68 (t, *J* = 8.8 Hz, 1H), 7.24 (d, *J* = 9.0 Hz, 1H), 6.94 - 6.74 (m, 1H), 6.23-6.14 (m, 1H), 5.81 - 5.68 (m, 2H), 4.95 - 4.45 (m, 5H), 4.25 - 3.79 (m, 3H), 3.76 - 3.53 (m, 2H), 3.43 - 3.33 (m, 1H), 3.00 - 2.85 (m, 1H), 1.46 (t, *J* = 5.8 Hz, 3H), 1.33 (dd, *J* = 10.8, 6.7 Hz, 3H), 1.13 (dd, *J=* 6.6, 1.6 Hz, 3H), 0.91 (dd, *J=* 6.7, 4.5 Hz, 3H).

**Examples 66-71 can be prepared by selecting corresponding starting materials according to the complete or partial synthesis method of Example 65:**

| **Example No.** | **Structural Formula/Chemical Name** | **MS m/z [M+1]⁺ / ¹H NMR(400 MHz)** |
|---|---|---|
| **Example 66** | 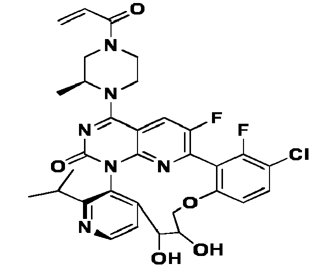 (R)-2⁴-((S)-4-acryloyl-2-methylpiperazin-1-yl)-3⁵-chloro-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2² -one | ESI-MS 653 [M+1]⁺. |
| | | ¹H NMR (400 MHz, MeOH-*d₄*) δ 8.53 (d, *J =* 4 Hz, 1H), 8.22 - 8.17 (m, 2H), 7.56 (t, *J* = 8 Hz, 1H), 7.08 (d, *J* = 8 Hz, 1H), 6.90 - 6.78 (m, 1H), 6.33 (d, *J =* 16Hz, 1H), 5.85 (d, *J=* 12 Hz, 1H), 4.91 (br, 1H), 4.70 - 4.63 (m, 1H), 4.52-4.48 (m, 2H), 4.22 - 4.05 (m, 3H), 3.73 - 3.69 (m, 2H), 3.58 - 3.48 (m, 2H), 3.02 - 2.95 (m, 1H), 1.59 - 1.55 (m, 3H), 1.29 (d, *J* = 8 Hz, 3H) 0.97 (d, *J* = 8 Hz, 3H). |
| **Example 67** | 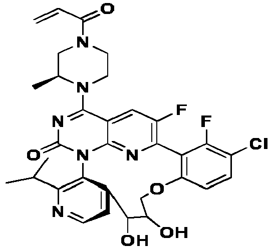 (S)-2⁴-((S)-4-acryloyl-2-methylpiperazin-1-yl)-3⁵-chloro-2⁶,3⁶-difluoro-6,7-dihydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 653.20 [M+1]⁺. |
| | | ¹H NMR (400 MHz, MeOH-*d₄*) δ8.53 (d, *J* = 4 Hz, 1H), 8.40 (d, *J* = 12 Hz, 1H), 8.19 (d, *J* = 4Hz, 1H), 7.56 (t, *J* =8 Hz, 1H), 7.08 (d, *J* =8 Hz, 1H), 6.90-6.80 (m, 1H), 6.34 (d, *J =* 16Hz, 1H), 5.85 (d, *J =* 12 Hz, 1H), 5.36 - 5.33 (m, 1H), 4.61 -4.46 (m, 2H), 4.37-4.33 (m, 1H), 4.23-4.01 (m, 4H), 3.81 -3.43 (m, 2H), 3.14- 2.98 (m, 2H), 1.39 (d, *J =* 4 Hz, 3H). 1.28 (d, *J* = 8 Hz, 3H).0.92 (d, *J* = 8 Hz, 3H). |
| **Example 68** | 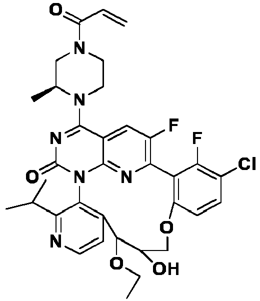 2⁴-((S)-4-acryloyl-2-methylpiperazin-1-yl)-3⁵-chloro-7-ethoxy-2⁶,3⁶-difluoro-6-hydroxy-1²-isopropyl-21,2²-dihydro-4-oxa-2(1, 7)-pyrido[2,3-d]pyrimidina-1 (3,4)-pyridina-3(1,2)-benzocycloheptane-2²-one | ESI-MS 681[M+1]⁺. |
| | | ¹H NMR (400 MHz, DMSO-*d₆*) δ 8.52 (d, *J* = 8.0 Hz, 1H), 8.51 - 8.29 (m, 1H), 8.00 (d, *J =* 5.2 Hz, 1H), 7.69 (t, *J=* 8.9 Hz, 1H), 7.27 (d, *J =* 9.0 Hz, 1H), 6.93 - 6.79 (m, 1H), 6.21 (d, *J* = 16.6 Hz, 1H), 5.77 (dd, *J* = 10.3, 2.4 Hz, 1H), 5.59 (dd, *J =* 5.0, 2.0 Hz, 1H), 5.12 - 5.00 (m, 1H), 4.90 - 4.72 (m, 1H), 4.54 - 3.79 (m, 9H), 3.70 - 3.50 (m, 2H), 2.93 - 2.82 (m, 1H), 1.33 (dd, *J =* 61.5, 6.7 Hz, 3H), 1.17 (dd, *J* = 6.6, 2.1 Hz, 3H), 0.93 (td, *J =* 7.0, 2.5 Hz, 3H), 0.84 (dd, *J* = 6.8, 3.6 Hz, 3H). |
| **Example 69** | 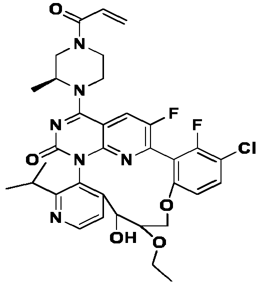 2⁴-((S)-4-acryloyl-2-methylpiperazin-1-yl)-3⁵-chloro-6-ethoxy-2⁶,3⁶-difluoro-7-hydroxy-1²-isopropyl-2¹,2²-dihydro-4-oxa-2(1, 7)-pyrido[2,3-d]pyrimidina-1(3,4)-pyridina-3(1,2)-benzocycloheptane-2²-one | ESI-MS 681[M+1]⁺. |
| | | ¹H NMR (400 MHz, DMSO-*d₆*) δ 8.59 - 8.21 (m, 2H), 7.90 (d, *J =* 5.3 Hz, 1H), 7.69 (t, *J =* 8.9 Hz, 1H), 7.28 (d, *J=* 9.1 Hz, 1H), 6.95 - 6.76 (m, 1H), 6.21 (d, *J* = 16.1 Hz, 1H), 5.77 (d, *J =* 10.8 Hz, 1H), 5.20 - 4.98 (m, 2H), 4.84 - 3.52 (m, 11H), 3.02 - 2.85 (m, 2H), 1.48 - 1.07 (m, 9H), 0.88 (d, *J* = 6.8 Hz, 3H). |
| **Example 70** | 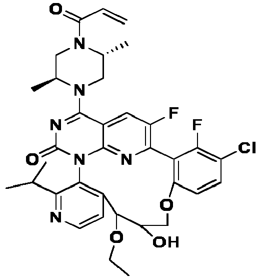 2⁴-((2S,5R)-4-acryloyl-2,5-dimethylpiperazin-1-yl)-3⁵-chloro-7-ethoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2 ² -one | ESI-MS 695[M+1]⁺. |
| | | ¹H NMR (400 MHz, DMSO-*d₆*) δ 8.55 - 8.20 (m, 2H), 8.03 - 7.95 (m, 1H), 7.69 (t, *J =* 8.8 Hz, 1H), 7.28 (d, *J =* 9.0 Hz, 1H), 6.91 - 6.75 (m, 1H), 6.18 (d, *J* = 16.7 Hz, 1H), 5.81 - 5.71 (m, 1H), 5.62 - 5.46 (m, 1H), 4.97 - 4.65 (m, 2H), 4.43 (s, 2H), 4.31 - 3.96 (m, 4H), 3.91 - 3.44 (m, 4H), 3.00 - 2.75 (m, 1H), 1.45 - 1.07 (m, 9H), 0.98 - 0.79 (m, 6H). |
| **Example 71** | 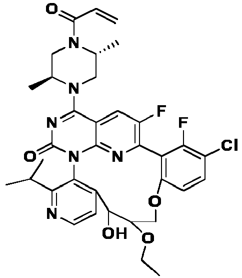 2⁴-((2S,5R)-4-acryloyl-2,5-dimethylpiperazin-1-yl)-3⁵-chloro-6-ethoxy-2⁶,3⁶-difluoro-7-hydroxy-1²-isopropyl-2¹,2²-dihydro-4-oxa-2(l,7)-pyrido[2,3-d]pyrimidina-1(3,4)-pyridina-3(1,2)-benzocycloheptane-2²-one | ESI-MS 695[M+1]⁺. |
| | | ¹H NMR (400 MHz, DMSO-*d₆*) δ 8.55 - 8.12 (m, 2H), 7.95 - 7.85 (m, 1H), 7.70 (t, *J =* 8.8 Hz, 1H), 7.30 (dd, *J* = 9.1, 6.1 Hz, 1H), 6.95 - 6.75 (m, 1H), 6.19 (d, *J* = 16.7 Hz, 1H), 5.81 - 5.72 (m, 1H), 5.08 - 4.40 (m, 6H), 4.27-3.54 (m, 6H), 2.99 - 2.78 (m, 2H), 1.57 - 1.07 (m, 12H), 0.98 - 0.81 (m, 3H). |

| **Examples 72-79 are prepared by SFC separation on the basis of the preparation in Examples 68-71:** | | |
|---|---|---|
| **Example No.** | **Structural Formula/Chemical Name** | **MS m/z [M+1]⁺ / ¹H NMR(400 MHz)** |
| **Example 72** | 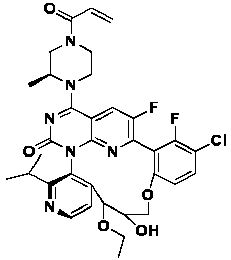 (R)-2⁴-((S)-4-acryloyl-2-methylpiperazin-1-yl)-3⁵-chloro-7-ethoxy-2⁶,3⁶-difluoro-6-hydroxy-1²-isopropyl-2¹,2²-dihydro-4-oxa-2(1, 7)-pyrido[2,3-d]pyrimidina-1 (3,4)-pyridina-3(1,2)-benzocycloheptane-2²-one | ESI-MS 681[M+1]⁺. |
| | | ¹H NMR (400 MHz, DMSO-*d₆*) δ 8.52 (d, *J* = 5.1 Hz, 1H), 8.37 - 8.25 (m, 1H), 8.00 (d, *J =* 5.1 Hz, 1H), 7.69 (t, *J=* 8.8 Hz, 1H), 7.27 (d, *J =* 9.0 Hz, 1H), 6.97 - 6.76 (m, 1H), 6.20 (d, *J* = 17.0 Hz, 1H), 5.77 (d, *J =* 10.4 Hz, 1H), 5.57 (d, *J* = 5.2 Hz, 1H), 4.90 - 4.72 (m, 1H), 4.53 - 3.88 (m, 8H), 3.75 - 3.40 (m, 3H), 3.26 - 3.18 (m, 1H), 2.91 - 2.82 (m, 1H), 1.41 (d, *J* = 6.6 Hz, 3H), 1.17 (d, *J =* 6.7 Hz, 3H), 0.93 (t, *J* = 6.9 Hz, 3H), 0.84 (d, *J* = 6.7 Hz, 3H). |
| **Example 73** | 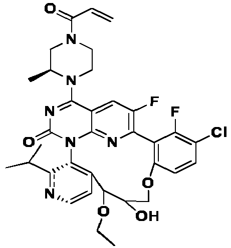 (S)-2⁴-((S)-4-acryloyl-2-methylpiperazin-1-yl)-3⁵-chloro-7-ethoxy-2⁶,3⁶-difluoro-6-hydroxy-1²-isopropyl-2¹,2²-dihydro-4-oxa-2(1, 7)-pyrido[2,3-d]pyrimidina-1 (3,4)-pyridina-3(1,2)-benzocycloheptane-2²-one | ESI-MS 681[M+1]⁺. |
| | | ¹H NMR (400 MHz, DMSO-*d₆*) δ 8.52 (d, *J* = 5.1 Hz, 1H), 8.51-8.43 (m, 1H), 8.00 (d, *J =* 5.0 Hz, 1H), 7.69 (t, *J* = 8.9 Hz, 1H), 7.32 - 7.21 (m, 1H), 6.86 (t, *J* = 13.6 Hz, 1H), 6.21 (d, *J =* 16.7 Hz, 1H), 5.77 (dd, *J* = 10.4, 2.4 Hz, 1H), 5.57 (d, *J =* 5.0 Hz, 1H), 5.05 (d, *J =* 6.6 Hz, 1H), 4.51 - 3.60 (m, 8H), 3.48-3.38 (m, 3H), 3.00 - 2.80 (m, 2H), 1.25 (t, *J* = 5.0 Hz, 3H), 1.17 (d, *J* = 6.6 Hz, 3H), 0.92 (t, *J =* 7.0 Hz, 3H), 0.83 (d, *J* = 6.7 Hz, 3H). |
| **Example 74** | 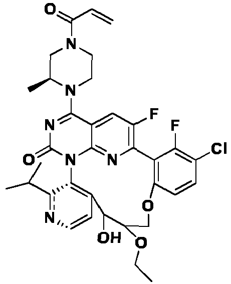 (S)-2⁴-((S)-4-acryloyl-2-methylpiperazin-1-yl)-3⁵-chloro-6-ethoxy-2⁶,3⁶-difluoro-7-hydroxy-1²-isopropyl-2¹,2²-dihydro-4-oxa-2(1, 7)-pyrido[2,3-d]pyrimidina-1 (3,4)-pyridina-3(1,2)-benzocycloheptane-2²-one | ESI-MS 681[M+1]⁺. |
| | | ¹H NMR (400 MHz, DMSO-*d₆*) δ 8.54 - 8.44 (m, 2H), 7.90 (d, *J =* 4.0 Hz, 1H), 7.69 (t, *J =* 8.0 Hz, 1H), 7.28 (d, *J =* 8.0 Hz, 1H), 6.95 - 6.80 (m, 1H), 6.21 (d, *J* = 16.5 Hz, 1H), 5.79 - 5.74 (m, 1H), 5.19 - 4.98 (m, 2H), 4.65 - 4.60 (m, 1H), 4.45 - 3.60 (m, 9H), 3.10 - 2.80 (m, 3H), 1.23 - 1.07 (m, 9H), 0.88 (d, *J* = 6.7 Hz, 3H). |
| **Example 75** | 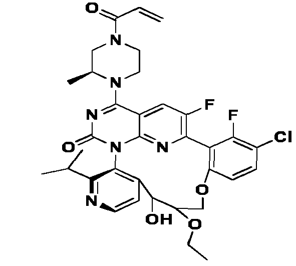 (R)-2⁴-((S)-4-acryloyl-2-methylpiperazin-1-yl)-3⁵-chloro-6-ethoxy-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 681[M+1]⁺. |
| | | ¹H NMR (400 MHz, DMSO-*d₆*) δ 8.51 (d, *J* = 5.0 Hz, 1H), 8.35 - 8.21 (m, 1H), 7.90 (d, *J =* 5.1 Hz, 1H), 7.69 (t, *J=* 8.8 Hz, 1H), 7.28 (d, *J =* 9.1 Hz, 1H), 6.93 - 6.79 (m, 1H), 6.20 (d, *J* = 16.8 Hz, 1H), 5.77 (d, *J =* 10.5 Hz, 1H), 5.02 (d, *J* = 8.1 Hz, 1H), 4.88 - 4.71(m, 1H), 4.62 (dd, *J =* 8.5, 4.7 Hz, 1H), 4.50 (d, *J* = 13.4 Hz, 1H), 4.42 - 4.10 (m, 2H), 3.85 - 3.40 (m, 6H), 3.0 - 2.85 (m, 3H), 1.43 (s, 3H), 1.22 - 1.11 (m, 6H), 0.88 (d, *J* = 6.7 Hz, 3H). |
| **Example 76** | 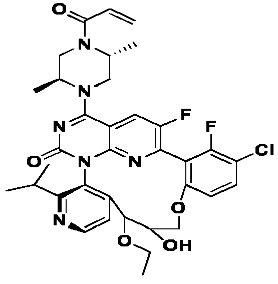 (R)-2⁴-((2S,5R)-4-acryloyl-2,5-dimethylpiperazin-1-yl)-3⁵-chloro-7-ethoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 695[M+1]⁺. |
| | | ¹H NMR (400 MHz, DMSO-*d₆*) δ 8.52 (d, *J* = 5.2 Hz, 1H), 8.30 - 8.21 (m, 1H), 8.00 (dd, *J =* 5.2, 1.9 Hz, 1H), 7.69 (t, *J* = 8.9 Hz, 1H), 7.28 (d, *J =* 9.0 Hz, 1H), 6.92 - 6.75 (m, 1H), 6.18 (d, *J =* 16.7 Hz, 1H), 5.75 (d, *J =* 10.4 Hz, 1H), 5.57 (t, *J =* 5.9 Hz, 1H), 4.90 - 4.75 (m, 2H), 4.55 - 4.35 (m, 2H), 4.32 - 4.09 (m, 1H), 4.00 (d, *J* = 3.8 Hz, 1H), 3.87-3.65 (m, 1H), 3.50 - 3.35 (m, 3H), 3.00 - 2.80 (m, 3H), 1.41 (d, *J* = 6.6 Hz, 3H), 1.35 - 1.09 (m, 6H), 0.93 (t, *J =* 6.9 Hz, 3H), 0.84 (t, *J =* 6.2 Hz, 3H). |
| **Example 77** | 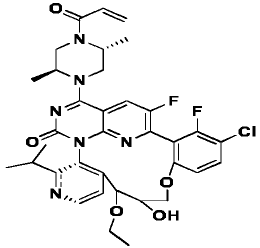 (S)-(S)-2⁴-((2S,5R)-4-acryloyl-2,5-dimethylpiperazin-1-yl)-3⁵-chloro-7-ethoxy-2⁶,3⁶-difluoro-6-hydroxy-1²-isopropyl-2¹,2²-dihydro-4-oxa-2(1,7)-pyrido[2,3-d]pyrimidina-1(3,4)-pyridina-3(1,2)-benzocycloheptane-2²-one | ESI-MS 695[M+1]⁺. |
| | | ¹H NMR (400 MHz, DMSO-*d₆*) δ 8.53 (d, *J =* 5.1 Hz, 1H), 8.47 (d, *J =* 9.1 Hz, 1H), 7.97 (d, *J =* 8.0 Hz, 1H), 7.69 (t, *J* = 8.8 Hz, 1H), 7.28 (d, *J =* 9.0 Hz, 1H), 6.89 - 6.77 (m, 1H), 6.19 (d, *J =* 16.6 Hz, 1H), 5.83 - 5.69 (m, 1H), 5.50 (t, *J* = 5.6 Hz, 1H), 5.00 - 4.88 (m, 1H), 4.50 - 4.37 (m, 1H), 4.33 - 3.75 (m, 5H), 3.65 - 3.40 (m, 3H), 3.00 - 2.75 (m, 3H), 1.38 - 1.09 (m, 9H), 0.97 (t, *J =* 6.6 Hz, 3H), 0.82 (d, *J =* 7.2 Hz, 3H). |
| **Example 78** | 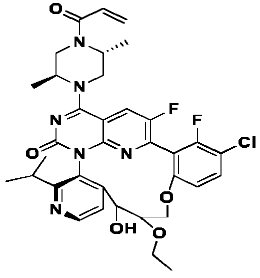 (R)-2⁴-((2S,5R)-4-acryloyl-2,5-dimethylpiperazin-1-yl)-3⁵-chloro-6-ethoxy-2⁶,3⁶-difluoro-7-hydroxy-1²-isopropyl-2¹,2²-dihydro-4-oxa-2(1,7)-pyrido[2,3-d]pyrimidina-1(3,4)-pyridina-3(1,2)-benzocycloheptane-22-one | ESI-MS 695[M+1]⁺. |
| | | ¹H NMR (400 MHz, DMSO-*d₆*) δ 8.52 (d, *J* = 5.0 Hz, 1H), 8.17 (dd, *J =* 14.3, 9.2 Hz, 1H), 7.90 (d, *J =* 5.0 Hz, 1H), 7.69 (t, *J =* 8.8 Hz, 1H), 7.29 (d, *J =* 9.0 Hz, 1H), 6.95 - 6.75 (m, 1H), 6.26 - 6.12 (m, 1H), 5.76 (d, *J =* 9.8 Hz, 1H), 4.96 (t, *J =* 6.9 Hz, 1H), 4.92 - 4.75 (m, 2H), 4.71 - 4.50 (m, 3H), 4.25 - 4.17 (m, 1H), 3.91 - 3.52 (m, 4H), 3.00 - 2.80 (m, 3H), 1.45 (t, *J =* 5.9 Hz, 3H), 1.37 - 1.10 (m, 9H), 0.91 (dd, *J =* 6.6, 4.5 Hz, 3H). |
| **Example 79** | 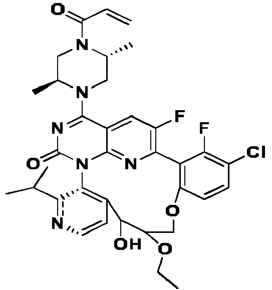 (S)-2⁴-(S)-((2S,5R)-4-acryloyl-2,5-dimethylpiperazin-1-yl)-3⁵-chloro-6-ethoxy-2⁶,3⁶-difluoro-7-hydroxy-1²-isopropyl-2¹,2²-dihydro-4-oxa-2(l,7)-pyrido[2,3-d]pyrimidina-1(3,4)-pyridina-3(1,2)-benzocycloheptane-2²-one | ESI-MS 695[M+1]⁺. |
| | | ¹H NMR (400 MHz, DMSO-*d₆*) δ 8.54 - 8.46 (m, 2H), 7.92 (d, *J =* 5.2 Hz, 1H), 7.70 (t, *J =* 8.9 Hz, 1H), 7.36 - 7.27 (m, 1H), 6.90 - 6.78 (m, 1H), 6.19 (dd, *J* = 16.6, 2.4 Hz, 1H), 5.82 - 5.67 (m, 1H), 5.09 - 4.88 (m, 2H), 4.80 - 4.38 (m, 2H), 4.26 - 3.60 (m, 7H), 2.99 - 2.77 (m, 3H), 1.31 - 1.07 (m, 12H), 0.85 (d, *J* = 6.8 Hz, 3H). |

### Biological Test Evaluation

### I. Anti-proliferation 2D CTG test

### 1. Experimental steps

### 1) Day 0: plating

When the cell confluency was 80%, the cells were isolated with 0.25% trypsin. The isolated cells were resuspended in 5 mL of fresh cell culture medium and centrifuged to collect the cells. The number of cells were counted at the same time. Then, the cells were suspended in a culture medium at a moderate concentration. The cells were placed into a 96-well plate with 1500 cells/well for H358 and 500 cells/well for MIAPACA-2. The 96-well plate was placed in an incubator at 37°C and incubated overnight.

### 2) Day 1: processing of compound

A 2 mM stock solution was serially diluted at 10 points at a ratio of 1:3. 5X culture medium containing the compound was transferred to the corresponding wells of 96 wells. The final peak concentration of the compound was 10 µM, and the final concentration of DMSO was 0.5%. The 96-well plate was placed in the incubator at 37°C and incubated for 5 days.

### 3) Baseline reading.

### 4) Day 6: reading of signals

A detection reagent (CTG) was added at 50 µL/well, and the signals were read in an Envision instrument.

### 2. Data processing

The percentage of inhibition (%) at each compound concentration was calculated based on the signals from the HPE and ZPE control wells contained in each assay plate and the fluorescence signals from individual compound wells. The inhibition rate in the ZPE control wells containing the enzyme and substrate was 0%, and the inhibition rate in the HPE control wells only containing the substrate was 100%. By testing the compound in terms of numeric concentration and inhibition percentage, the compound concentration required for 50% inhibition (IC₅₀) was determined using a four-parameter logarithmic dose-response equation. The endpoint value (IC₅₀) of the reference compound was evaluated in each experiment as a quality control measure. If the endpoint value was within three times the expected value, the experiment was considered acceptable.

**Table 1: Biological test results**

| **Example No.** | **2D-CTG (H358) IC₅₀(nM)** | **2D-CTG (MIA PaCa-2) IC₅₀(nM)** | **Example No.** | **2D-CTG (H358) IC₅₀(nM)** | **2D-CTG (MIA PaCa-2) IC₅₀(nM)** |
|---|---|---|---|---|---|
| **Example 1** | 13 | 10 | **Example 39** | 4 | 2 |
| **Example 1-R** | 9 | 12 | **Example 40** | 2000 | 2000 |
| **Example 1-S** | 931 | 100 | **Example 41** | 3 | 2 |
| **Example 2** | 2 | 1 | **Example 42** | 977 | 669 |
| **Example 3** | 1251 | 400 | **Example 43** | 5 | 6 |
| **Example 4** | 73 | 83 | **Example 44** | 250 | 125 |
| **Example 5** | 19 | 20 | **Example 45** | 2 | 2 |
| **Example 6** | 10 | 10 | **Example 46** | 261 | 148 |
| **Example 7** | 15 | 10 | **Example 47** | 9 | 5 |
| **Example 8** | 9 | 3 | **Example 48** | NT | NT |
| **Example 9** | 21 | 13 | **Example 49** | 7 | 6 |
| **Example 10** | 5 | 4 | **Example 50** | 2000 | 2000 |
| **Example 11** | 26 | 21 | **Example 51** | 10 | 7 |
| **Example 12** | 26 | 26 | **Example 52** | NT | NT |
| **Example 13** | 5 | 5 | **Example 53** | 17 | 16 |
| **Example 14** | 6 | 9 | **Example 54** | 830 | 708 |
| **Example 15** | 12 | 10 | **Example 55** | 8 | 7 |
| **Example 16** | 29 | 20 | **Example 56** | 221 | 218 |
| **Example 17** | 9 | 9 | **Example 57** | 35 | 18 |
| **Example 18** | 14 | 6 | **Example 58** | 461 | 314 |
| **Example 18-R** | 16 | 15 | **Example 59** | 23 | 9 |
| **Example 18-S** | 351 | 266 | **Example 60** | 45 | 38 |
| **Example 19** | 18 | 18 | **Example 61** | 38 | 16 |
| **Example 20** | 25 | 30 | **Example 62** | 20 | 16 |
| **Example 21** | 60 | 153 | **Example 63** | 4 | 4 |
| **Example 22** | 14 | 7 | **Example 64** | 3 | 1 |
| **Example 23** | 11 | 9 | **Example 65** | 1 | 1 |
| **Example 24** | 17 | 8 | **Example 66** | 5 | 3 |
| **Example 25** | 36 | 15 | **Example 67** | 1322 | 859 |
| **Example 26** | 2 | 1 | **Example 68** | 27 | 22 |
| **Example 27** | 9 | 4 | **Example 69** | 31 | 26 |
| **Example 28** | 28 | 27 | **Example 70** | 16 | 7 |
| **Example 29** | 6 | 6 | **Example 71** | 8 | 3 |
| **Example 30** | NT | NT | **Example 72** | 7 | 6 |
| **Example 31** | NT | NT | **Example 73** | 668 | 2000 |
| **Example 32** | 41 | 69 | **Example 74** | 2000 | 2000 |
| **Example 33** | 42 | 154 | **Example 75** | 3 | 6 |
| **Example 34** | 4 | 7 | **Example 76** | 4 | 4 |
| **Example 35** | 11 | 4 | **Example 77** | 523 | 2000 |
| **Example 36** | 1 | 1 | **Example 78** | 5 | 3 |
| **Example 37** | 14 | 5 | **Example 79** | 2000 | 2000 |
| **Example 38** | NT | NT | **Positive compound** | 8 | 11 |
| **Note** | "NT", i.e., "Not Tested", means that the compound was not tested yet. | | | | |
| | "Positive compound" refers to Example 13 in "CN113248521A", with a chemical structure as below: | | | | |
| | 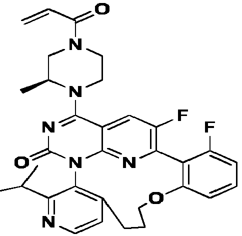 | | | | |

From the activity data of the specific compounds of the examples, it can be seen that the series of compounds of the present invention shows a strong inhibitory effect on the cellular activity of K-RAS, and under the same test conditions, the inhibitory effect of some of the compounds on the cellular activity of K-RAS is increased several times compared with the positive compounds.

All documents mentioned in the present invention are hereby incorporated by reference in their entirety, just as each document is cited separately as a reference. In addition, it should be understood that various modifications and changes may be made by those skilled in the art after reading the above disclosure of the present invention and these equivalent forms also fall within the scope defined by the claims appended hereto.

## Claims

1. A compound of formula (I), a stereoisomer or pharmaceutically acceptable salt thereof: 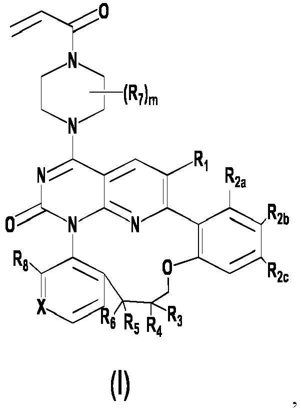
wherein, X is C(R₉) or N;
R₁ is selected from the group consisting of hydrogen, deuterium, halogen, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl, acetamido and -SF₅;
R₂ₐ is selected from the group consisting of hydrogen, deuterium, halogen, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl, acetamido and -SF₅;
R_{2b} is selected from the group consisting of hydrogen, deuterium, halogen, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl, acetamido and -SF₅;
R_{2c} is selected from the group consisting of hydrogen, deuterium, halogen, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl, acetamido and -SF₅;
R₃ and R₄ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, -SF₅, -S(O)ᵣR₁₀, -O-R₁₁, -C(O)OR₁₁, -C(O)R₁₂,-O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, or, R₃ and R₄, together with the carbon atom directly attached thereto, form C(O), 3-12 membered cycloalkyl or 3-12 membered heterocyclyl, above groups are optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, =O, -SF₅, -S(O)ᵣR₁₀, -O-R₁₁,-C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₅ and R₆ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, -SF₅, -S(O)ᵣR₁₀, -O-R₁₁, -C(O)OR₁₁, -C(O)R₁₂,-O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, or, R₅ and R₆, together with the carbon atom directly attached thereto, form C(O), 3-12 membered cycloalkyl or 3-12 membered heterocyclyl, above groups are optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, =O, -SF₅, -S(O)ᵣR₁₀, -O-R₁₁,-C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
provided that R₃, R₄, R₅ and R₆ are not all hydrogen at a time;
or, R₄ and R₅, together with the moiety directly attached thereto, form 3-12 membered cycloalkyl or 3-12 membered heterocyclyl, the 3-12 membered cycloalkyl or 3-12 membered heterocyclyl is optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, =O, -C₀₋₈ alkyl-SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂, R₃ and R₆ are defined as above;
m is 0, 1, 2, 3 or 4;
each R₇ is independently selected from the group consisting of hydrogen, deuterium, halogen, cyano, hydroxy, C₁₋₄ alkoxy, C₁₋₄ alkyl, C₃₋₆ cycloalkyl, 4-6 membered heterocyclyl, -SF₅, C₂₋₄ alkynyl, C₁₋₄ cyanoalkyl, C₁₋₄ hydroxyalkyl, C₁₋₄ haloalkyl and C₁₋₄ deuterioalkyl, or, when m≥2, two of R₇, together with the moiety directly attached thereto, form C₃₋₁₂ cycloalkyl or 3-12 membered heterocyclyl;
R₈ and R₉ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, -C₀₋₈ alkyl-SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂, above groups are optionally further substituted by one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, =O, -C₀₋₈ alkyl-SF₅, -C₀₋₈ alkyl-S(O)ᵣR₁₀, -C₀₋₈ alkyl-O-R₁₁, -C₀₋₈ alkyl-C(O)OR₁₁, -C₀₋₈ alkyl-C(O)R₁₂, -C₀₋₈ alkyl-O-C(O)R₁₂, -C₀₋₈ alkyl-NR₁₃R₁₄, -C₀₋₈ alkyl-C(=NR₁₃)R₁₂, -C₀₋₈ alkyl-N(R₁₃)-C(=NR₁₄)R₁₂, -C₀₋₈ alkyl-C(O)NR₁₃R₁₄ and -C₀₋₈ alkyl-N(R₁₃)-C(O)R₁₂;
each R₁₀ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₅₋₁₀ aryl, 5-10 membered heteroaryl and -NR₁₃R₁₄, above groups are optionally further substituted by one or more substituents selected from the group consisting of deuterium, halogen, hydroxy, oxo, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy and -NR₁₃R₁₄;
each R₁₁ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₅₋₁₀ aryl and 5-10 membered heteroaryl, above groups are optionally further substituted by one or more substituents selected from the group consisting of deuterium, halogen, hydroxy, oxo, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy and -NR₁₃R₁₄;
each R₁₂ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy and -NR₁₃R₁₄, above groups are optionally further substituted by one or more substituents selected from the group consisting of deuterium, halogen, hydroxy, cyano, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy and -NR₁₃R₁₄;
each R₁₃ and each R₁₄ are independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₅₋₁₀ aryl, 5-10 membered heteroaryl, sulfonyl, methanesulfonyl, isopropylsulfonyl, cyclopropylsulfonyl, p-toluenesulfonyl, amino, mono-C₁₋₈ alkyl amino, di-C₁₋₈ alkyl amino and C₁₋₁₀ alkanoyl, above groups are optionally further substituted by one or more substituents selected from the group consisting of deuterium, halogen, hydroxy, C₁₋₈ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy, amino, mono-C₁₋₈ alkyl amino, di-C₁₋₈ alkyl amino and C₁₋₁₀ alkanoyl;
or, R₁₃ and R₁₄, together with the nitrogen atom directly attached thereto, form 4-12 membered heterocyclyl, the 4-12 membered heterocyclyl is optionally further substituted by one or more substituents selected from the group consisting of deuterium, halogen, hydroxy, C₁₋₁₀ alkyl, C₁₋₁₀ alkoxy, C₃₋₁₂ cycloalkyl, C₃₋₁₂ cycloalkyloxy, 3-12 membered heterocyclyl, 3-12 membered heterocyclyloxy, C₅₋₁₀ aryl, C₅₋₁₀ aryloxy, 5-10 membered heteroaryl, 5-10 membered heteroaryloxy, amino, mono-C₁₋₈ alkyl amino, di-C₁₋₈ alkyl amino and C₁₋₁₀ alkanoyl;
each r is independently 0, 1 or 2.

2. The compound of formula (I), the stereoisomer or pharmaceutically acceptable salt thereof of claim 1, wherein, R₃ and R₄ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -SF₅, -S(O)ᵣR₁₀, -O-R₁₁, -C(O)OR₁₁, -C(O)R₁₂,-O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, or, R₃ and R₄, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, -SF₅, -S(O)ᵣR₁₀, -O-R₁₁, -C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₅ and R₆ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -SF₅, -S(O)ᵣR₁₀, -O-R₁₁, -C(O)OR₁₁, -C(O)R₁₂, -O-C(O)R₁₂, - NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, or, R₅ and R₆, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, -SF₅, -S(O)ᵣR₁₀, -O-R₁₁, -C(O)OR₁₁, -C(O)R₁₂, -O-C(O)R₁₂, -NR₁₃R₁₄, - C(=NR₁₃)R₁₂, -N(R₁₃)-C(=NR₁₄)R₁₂, -C(O)NR₁₃R₁₄ and -N(R₁₃)-C(O)R₁₂;
Provided that R₃, R₄, R₅ and R₆ are not all hydrogen at a time;
or, R₄ and R₅, together with the moiety directly attached thereto, form 3-6 membered cycloalkyl or 3-6 membered heterocyclyl, the 3-6 membered cycloalkyl or 3-6 membered heterocyclyl is optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, - C₀₋₄ alkyl-SF₅, -C₀₋₄ alkyl-S(O)ᵣR₁₀, -C₀₋₄ alkyl-O-R₁₁, -C₀₋₄ alkyl-C(O)OR₁₁, -C₀₋₄ alkyl-C(O)R₁₂, - C₀₋₄ alkyl-O-C(O)R₁₂, -C₀₋₄ alkyl-NR₁₃R₁₄, -C₀₋₄ alkyl-C(=NR₁₃)R₁₂, -C₀₋₄ alkyl-N(R₁₃)-C(=NR₁₄)R₁₂, -C₀₋₄ alkyl-C(O)NR₁₃R₁₄ and -C₀₋₄ alkyl-N(R₁₃)-C(O)R₁₂, R₃ and R₆ are defined as above;
wherein, R₁₀, R₁₁, R₁₂, R₁₃, R₁₄ and r are defined as in claim 1.

3. The compound of formula (I), the stereoisomer or pharmaceutically acceptable salt thereof of claim 1, wherein, R₁ is selected from the group consisting of hydrogen, deuterium, fluorine, chlorine, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl and -SF₅;
R₂ₐ is selected from the group consisting of hydrogen, deuterium, fluorine, chlorine, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl and -SF₅;
R_{2b} is selected from the group consisting of hydrogen, deuterium, fluorine, chlorine, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl and -SF₅;
R_{2c} is selected from the group consisting of hydrogen, deuterium, fluorine, chlorine, cyano, hydroxy, C₁₋₄ alkyl, C₁₋₄ haloalkyl, C₁₋₄ deuterioalkyl, C₁₋₄ alkoxy, C₁₋₄ haloalkoxy, C₁₋₄ deuterioalkoxy, C₃₋₆ cycloalkyl, 4-6 membered heterocyclyl and -SF₅.

4. The compound of formula (I), the stereoisomer or pharmaceutically acceptable salt thereof of claim 1, wherein, each R₇ is independently selected from the group consisting of hydrogen, deuterium, halogen, cyano, hydroxy, C₁₋₄ alkoxy, C₁₋₄ alkyl, C₂₋₄ alkynyl, C₃₋₆ cycloalkyl, 4-6 membered heterocyclyl, -SF₅ and C₁₋₄ cyanoalkyl, or, when m≥2, two of R₇, together with the moiety directly attached thereto, form C₃₋₆ cycloalkyl or 3-6 membered heterocyclyl;
**preferably,** each R₇ is independently selected from the group consisting of hydrogen, deuterium, fluorine, chlorine, cyano, hydroxy, methyl, ethyl, isopropoxy, cyclopropyl, cyclobutyl, cyclobutoxy, cyanomethyl and -SF₅.

5. The compound of formula (I), the stereoisomer or pharmaceutically acceptable salt thereof of claim 1, wherein, R₈ and R₉ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -C₀₋₄ alkyl-SF₅, -C₀₋₄ alkyl-S(O)ᵣR₁₀, - C₀₋₄ alkyl-O-R₁₁, -C₀₋₄ alkyl-C(O)OR₁₁, -C₀₋₄ alkyl-C(O)R₁₂, -C₀₋₄ alkyl-O-C(O)R₁₂, -C₀₋₄ alkyl-NR₁₃R₁₄, -C₀₋₄ alkyl-C(=NR₁₃)R₁₂, -C₀₋₄ alkyl-N(R₁₃)-C(=NR₁₄)R₁₂, -C₀₋₄ alkyl-C(O)NR₁₃R₁₄ and - C₀₋₄ alkyl-N(R₁₃)-C(O)R₁₂, above groups are optionally further substituted by one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, -C₀₋₄ alkyl-SF₅, -C₀₋₄ alkyl-S(O)ᵣR₁₀, -C₀₋₄ alkyl-O-R₁₁, -C₀₋₄ alkyl-C(O)OR₁₁, -C₀₋₄ alkyl-C(O)R₁₂, -C₀₋₄ alkyl-O-C(O)R₁₂, -C₀₋₄ alkyl-NR₁₃R₁₄, -C₀₋₄ alkyl-C(=NR₁₃)R₁₂, -C₀₋₄ alkyl-N(R₁₃)-C(=NR₁₄)R₁₂, -C₀₋₄ alkyl-C(O)NR₁₃R₁₄ and -C₀₋₄ alkyl-N(R₁₃)-C(O)R₁₂;
**preferably,** R₈ and R₉ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, -SF₅, -S(O)ᵣR₁₀, -O-R₁₁, -C(O)OR₁₁, -C(O)R₁₂, - O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₅₋₈ aryl, 5-8 membered heteroaryl, =O, - SF₅, -S(O)ᵣR₁₀, -O-R₁₁, -C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
wherein, R₁₀, R₁₁, R₁₂, R₁₃, R₁₄ and r are defined as in claim 1.

6. The compound of formula (I), the stereoisomer or pharmaceutically acceptable salt thereof of claim 1, wherein, the compound of formula (I) is a compound having formula (II): 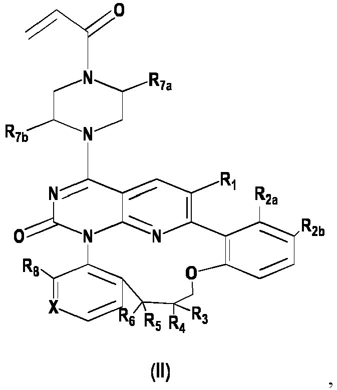
wherein, X is CH or N;
R₁ is selected from the group consisting of hydrogen, deuterium, fluorine, chlorine, cyano, hydroxy, methyl, ethyl, isopropyl, trifluoromethyl, difluoromethyl, trideuteriomethyl, dideuteriomethyl, methoxy, ethoxy, isopropoxy, trifluoromethoxy, difluoromethoxy, trideuteriomethoxy, dideuteriomethoxy, trifluoroisopropoxy, trideuterioisopropoxy, cyclopropyl, cyclobutyl and cyclobutoxy;
R₂ₐ is selected from the group consisting of hydrogen, deuterium, fluorine, chlorine, cyano, hydroxy, methyl, ethyl, isopropyl, trifluoromethyl, difluoromethyl, trideuteriomethyl, dideuteriomethyl, methoxy, ethoxy, isopropoxy, trifluoromethoxy, difluoromethoxy, trideuteriomethoxy, dideuteriomethoxy, trifluoroisopropoxy, trideuterioisopropoxy, cyclopropyl, cyclobutyl and cyclobutoxy;
R_{2b} is selected from the group consisting of hydrogen, deuterium, fluorine, chlorine, cyano, hydroxy, methyl, ethyl, isopropyl, trifluoromethyl, difluoromethyl, trideuteriomethyl, dideuteriomethyl, methoxy, ethoxy, isopropoxy, trifluoromethoxy, difluoromethoxy, trideuteriomethoxy, dideuteriomethoxy, trifluoroisopropoxy, trideuterioisopropoxy, cyclopropyl, cyclobutyl and cyclobutoxy;
R₃ and R₄ are each independently selected from hydrogen, deuterium, -O-R₁₁ and -O-C(O)R₁₂, or, R₃ and R₄, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, - S(O)ᵣR₁₀, -O-R₁₁, -C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
R₅ and R₆ are each independently selected from hydrogen, deuterium, -O-R₁₁ and -O-C(O)R₁₂, or, R₅ and R₆, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, - S(O)ᵣR₁₀, -O-R₁₁, -C(O)OR₁₁, -C(O)R₁₂, -O-C(O)R₁₂, -NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂;
provided that R₃, R₄, R₅ and R₆ are not all hydrogen at a time;
or, R₄ and R₅, together with the moiety directly attached thereto, form 3-6 membered cycloalkyl or 3-6 membered heterocyclyl, the 3-6 membered cycloalkyl or 3-6 membered heterocyclyl is optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, -S(O)ᵣR₁₀, -O-R₁₁, -C(O)OR₁₁, -C(O)R₁₂, -O-C(O)R₁₂, -C₀₋₂ alkyl-NR₁₃R₁₄, -C(=NR₁₃)R₁₂, -N(R₁₃)-C(=NR₁₄)R₁₂, -C(O)NR₁₃R₁₄ and -N(R₁₃)-C(O)R₁₂, R₃ and R₆ are defined as above;
R₇ₐ is selected from the group consisting of hydrogen, deuterium, fluorine, chlorine, cyano, hydroxy, methyl, ethyl, isopropoxy, cyclopropyl, cyclobutyl and cyclobutoxy;
R_{7b} is selected from the group consisting of hydrogen, deuterium, fluorine, chlorine, cyano, hydroxy, methyl, ethyl, isopropoxy, cyclopropyl, cyclobutyl and cyclobutoxy;
R₈ is selected from the group consisting of hydrogen, deuterium, methyl, ethyl, propyl, isopropyl, trifluoromethyl, difluoromethyl, trideuteriomethyl, dideuteriomethyl, cyclopropyl, cyclobutyl, azacyclobutyl, oxacyclobutyl, methoxy, ethoxy and isopropoxy;
wherein, R₁₀, R₁₁, R₁₂, R₁₃, R₁₄ and r are defined as in claim 1.

7. The compound of formula (I), the stereoisomer or pharmaceutically acceptable salt thereof of claim 6, wherein, the compound of formula (I) is a compound having formula (III): 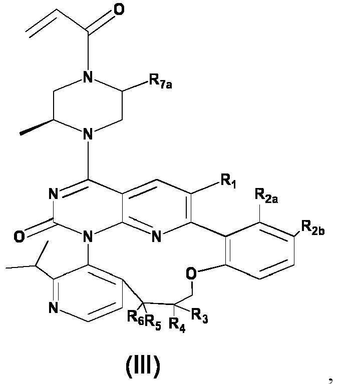
wherein, R₁ is hydrogen, deuterium, fluorine or chlorine;
R₂ₐ is hydrogen, deuterium, fluorine or chlorine;
R_{2b} is selected from the group consisting of hydrogen, deuterium, fluorine, chlorine, cyano, hydroxy, methyl, trifluoromethyl, methoxy, trifluoromethoxy and cyclopropyl;
R₇ₐ is selected from the group consisting of hydrogen, deuterium, fluorine, chlorine, methyl, ethyl, isopropoxy, cyclopropyl, cyclobutyl and cyclobutoxy;
R₃, R₄, R₅ and R₆ are defined as in claim 6.

8. The compound of formula (I), the stereoisomer or pharmaceutically acceptable salt thereof of claim 7, wherein, R₅ is -O-R₁₁ or -O-C(O)R₁₂, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, - SF₅, -S(O)ᵣR₁₀, -O-R₁₁, -C(O)OR₁₁, -C(O)R₁₂, -O-C(O)R₁₂, -NR₁₃R₁₄, -C(O)NR₁₃R₁₄ and -N(R₁₃)-C(O)R₁₂; R₆ is hydrogen or deuterium;
R₃ is hydrogen, deuterium, -O-R₁₁ or -O-C(O)R₁₂, R₄ is hydrogen or deuterium, or, R₃ and R₄, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, -S(O)ᵣR₁₀, -O-R₁₁, -C(O)OR₁₁, -C(O)R₁₂, -O-C(O)R₁₂, -NR₁₃R₁₄, -C(O)NR₁₃R₁₄ and -N(R₁₃)-C(O)R₁₂;
wherein, R₁₀, R₁₁, R₁₂, R₁₃, R₁₄ and r are defined as in claim 7.

9. The compound of formula (I), the stereoisomer or pharmaceutically acceptable salt thereof of claim 8, wherein, R₅ is selected from the group consisting of hydroxy, 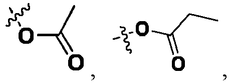 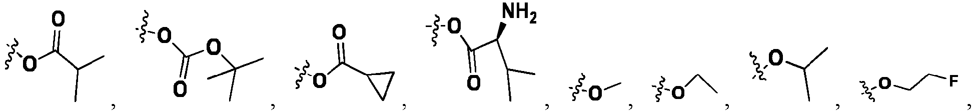 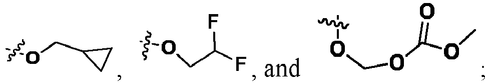 R₆ is hydrogen or deuterium;
R₃ is selected from the group consisting of hydrogen, deuterium, hydroxy, 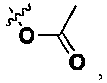 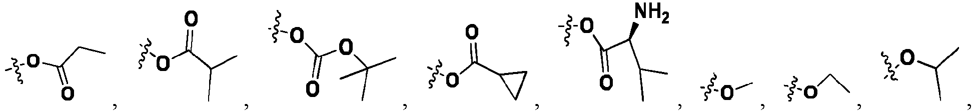 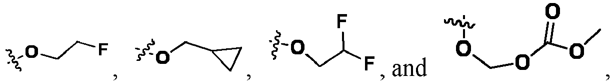 R₄ is hydrogen or deuterium, or, R₃ and R₄, together with the carbon atom directly attached thereto, form C(O), cyclopropyl, cyclobutyl, azacyclobutyl or oxacyclobutyl.

10. The compound of formula (I), the stereoisomer or pharmaceutically acceptable salt thereof of claim 7, wherein, R₅ and R₆, together with the carbon atom directly attached thereto, form C(O);
R₃ is hydrogen, deuterium, -O-R₁₁ or -O-C(O)R₁₂, R₄ is hydrogen or deuterium, or, R₃ and R₄, together with the carbon atom directly attached thereto, form C(O), 3-6 membered cycloalkyl or 3-6 membered heterocyclyl, above groups are optionally further substituted by one or more substituents selected from the group consisting of deuterium, halogen, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, -S(O)ᵣR₁₀, -O-R₁₁, -C(O)OR₁₁, -C(O)R₁₂, -O-C(O)R₁₂, -NR₁₃R₁₄, -C(O)NR₁₃R₁₄ and -N(R₁₃)-C(O)R₁₂;
wherein, R₁₀, R₁₁, R₁₂, R₁₃, R₁₄ and r are defined as in claim 7.

11. The compound of formula (I), the stereoisomer or pharmaceutically acceptable salt thereof of claim 10, wherein, R₅ and R₆, together with the carbon atom directly attached thereto, form C(O);
R₃ is selected from the group consisting of hydrogen, deuterium, hydroxy, 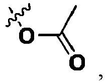 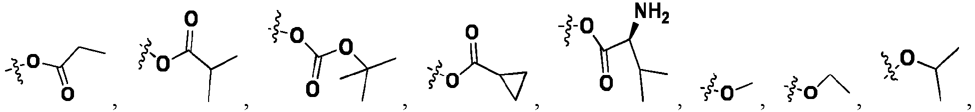 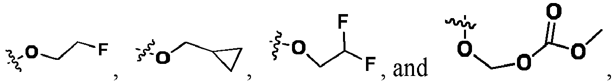 R₄ is hydrogen or deuterium, or, R₃ and R₄, together with the carbon atom directly attached thereto, form C(O), cyclopropyl, cyclobutyl, azacyclobutyl or oxacyclobutyl.

12. The compound of formula (I), the stereoisomer or pharmaceutically acceptable salt thereof of claim 7, wherein, R₄ and R₅, together with the moiety directly attached thereto, form 3-6 membered cycloalkyl or 3-6 membered heterocyclyl, the 3-6 membered cycloalkyl or 3-6 membered heterocyclyl is optionally further substituted by one or more substituents selected from the group consisting of deuterium, fluorine, chlorine, cyano, C₁₋₄ alkyl, C₁₋₄ haloalkyl, C₁₋₄ deuterioalkyl, C₂₋₄ alkynyl, C₃₋₆ cycloalkyl, 3-6 membered heterocyclyl, =O, -SF₅, -S(O)ᵣR₁₀, -O-R₁₁, -C(O)OR₁₁, - C(O)R₁₂, -O-C(O)R₁₂, -CH₂-NR₁₃R₁₄, -C(O)NR₁₃R₁₄ and -N(R₁₃)-C(O)R₁₂; R₃ is hydrogen or deuterium; R₆ is hydrogen or deuterium;
wherein, R₁₀, R₁₁, R₁₂, R₁₃, R₁₄ and r are defined as in claim 7.

13. The compound of formula (I), the stereoisomer or pharmaceutically acceptable salt thereof of claim 12, wherein, R₄ and R₅, together with the moiety directly attached thereto, form 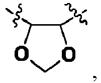 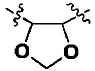 is optionally further substituted by one or more substituents selected from the group consisting of deuterium, fluorine, chlorine, cyano, methyl, ethyl, propyl, isopropyl, trifluoromethyl, difluoromethyl, trideuteriomethyl, dideuteriomethyl, cyclopropyl, cyclobutyl, azacyclobutyl, oxacyclobutyl, =O, methoxy, ethoxy, isopropoxy and 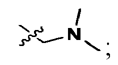 R₃ is hydrogen or deuterium; R₆ is hydrogen or deuterium.

14. The compound of formula (I), the stereoisomer or pharmaceutically acceptable salt thereof of claim 1, wherein, each R₁₀ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₅₋₈ aryl, 5-8 membered heteroaryl and -NR₁₃R₁₄, above groups are optionally further substituted by one or more substituents selected from the group consisting of deuterium, halogen, hydroxy, oxo, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy and -NR₁₃R₁₄;
each R₁₁ is independently selected from the group consisting of hydrogen, deuterium, C₁₋₄ alkyl, C₂₋₄ alkenyl, C₃₋₆ cycloalkyl, 3-6 membered heterocyclyl, C₅₋₈ aryl and 5-8 membered heteroaryl, above groups are optionally further substituted by one or more substituents selected from the group consisting of deuterium, halogen, hydroxy, oxo, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy and -NR₁₃R₁₄;
each R₁₂ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy and -NR₁₃R₁₄, above groups are optionally further substituted by one or more substituents selected from the group consisting of deuterium, halogen, hydroxy, cyano, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy and -NR₁₃R₁₄;
each R₁₃ and each R₁₄ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₅₋₈ aryl, 5-8 membered heteroaryl, sulfonyl, methanesulfonyl, isopropylsulfonyl, cyclopropylsulfonyl, p-toluenesulfonyl, amino, mono-C₁₋₄ alkyl amino, di-C₁₋₄ alkyl amino and C₁₋₄ alkanoyl, above groups are optionally further substituted by one or more substituents selected from the group consisting of deuterium, halogen, hydroxy, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy, amino, mono-C₁₋₄ alkyl amino, di-C₁₋₄ alkyl amino and C₁₋₄ alkanoyl;
or, R₁₃ and R₁₄, together with the nitrogen atom directly attached thereto, form 4-6 membered heterocyclyl, the 4-6 membered heterocyclyl is optionally further substituted by one or more substituents selected from the group consisting of deuterium, halogen, hydroxy, C₁₋₄ alkyl, C₁₋₄ alkoxy, C₃₋₆ cycloalkyl, C₃₋₆ cycloalkyloxy, 3-6 membered heterocyclyl, 3-6 membered heterocyclyloxy, C₅₋₈ aryl, C₅₋₈ aryloxy, 5-8 membered heteroaryl, 5-8 membered heteroaryloxy, amino, mono-C₁₋₄ alkyl amino, di-C₁₋₄ alkyl amino and C₁₋₄ alkanoyl.

15. The compound of formula (I), the stereoisomer or pharmaceutically acceptable salt thereof of claim 1, wherein, the compound is selected from the group consisting of:

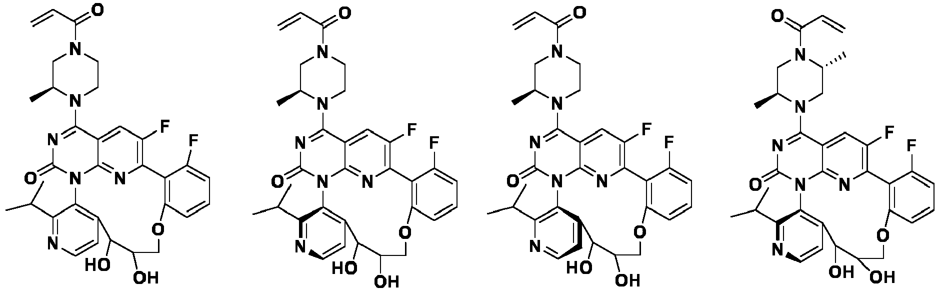

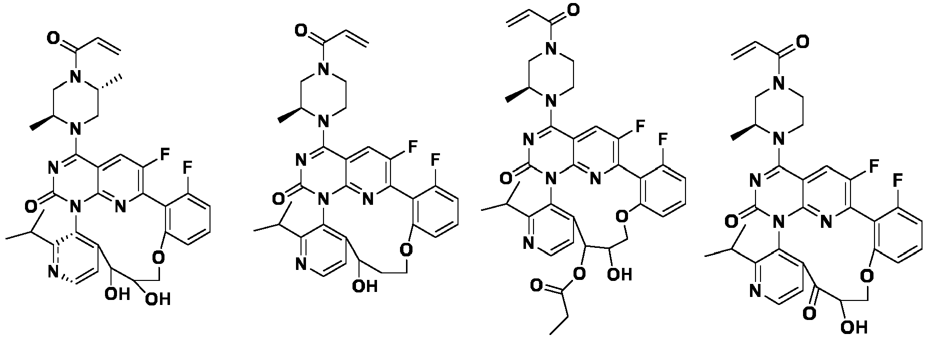

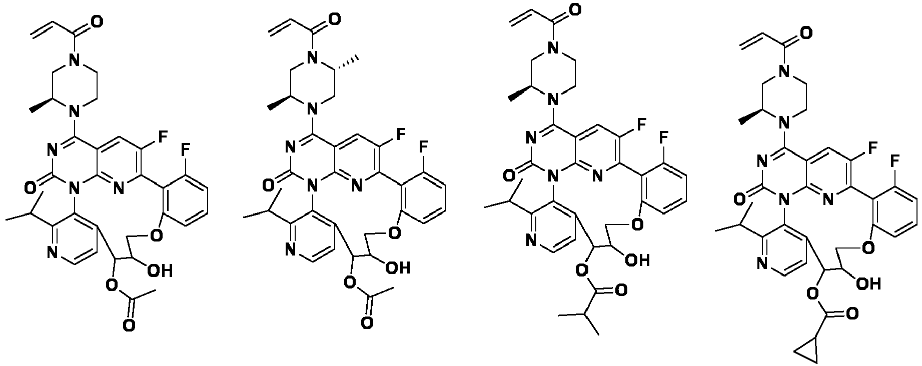

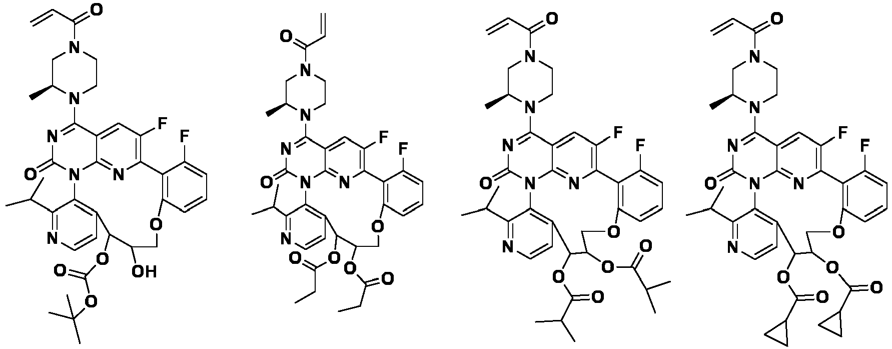

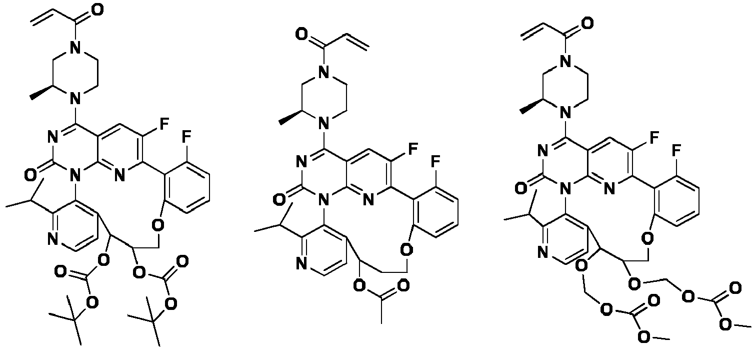

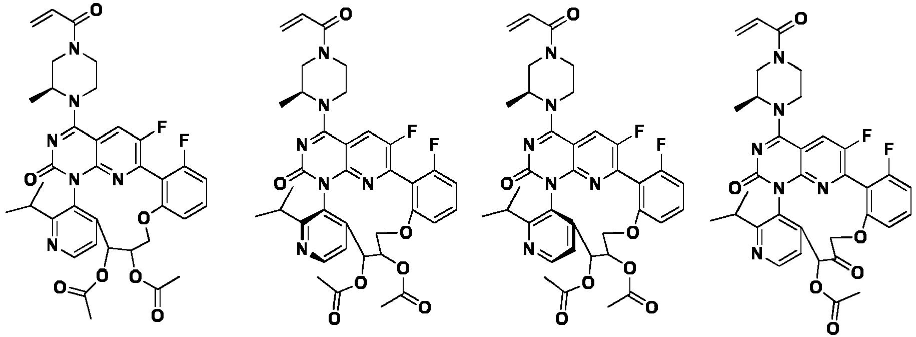

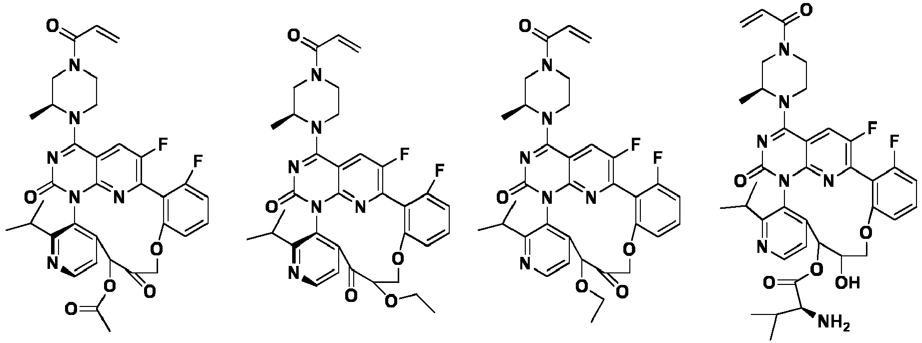

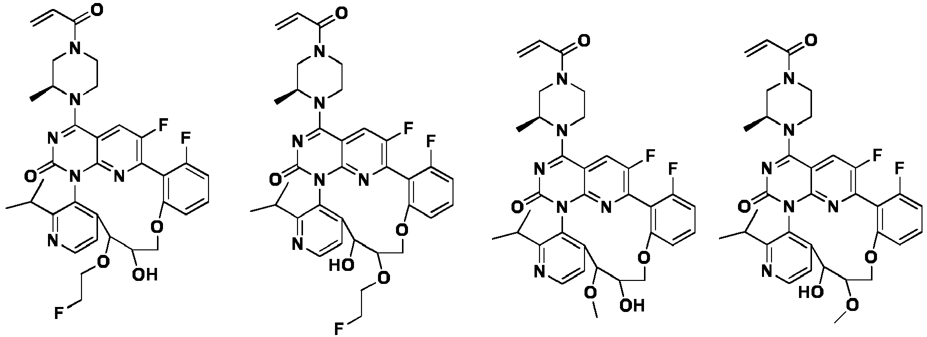

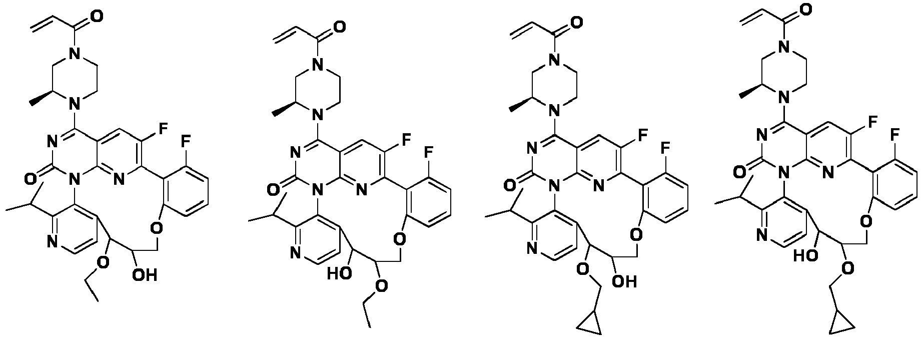

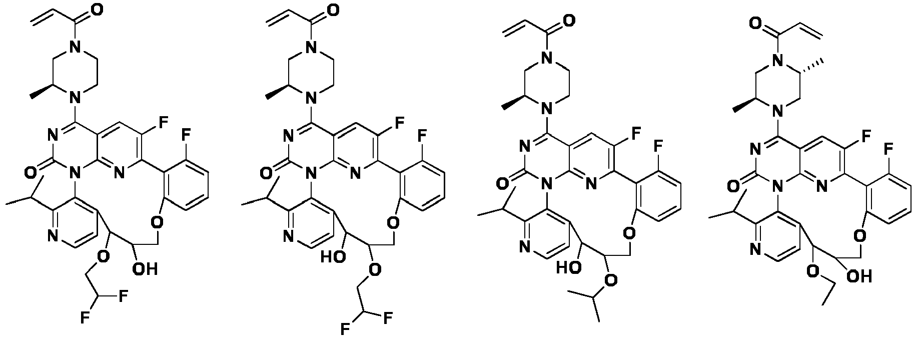

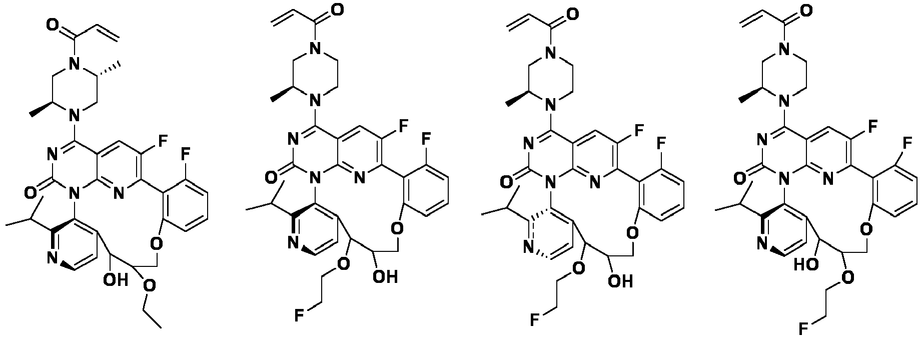

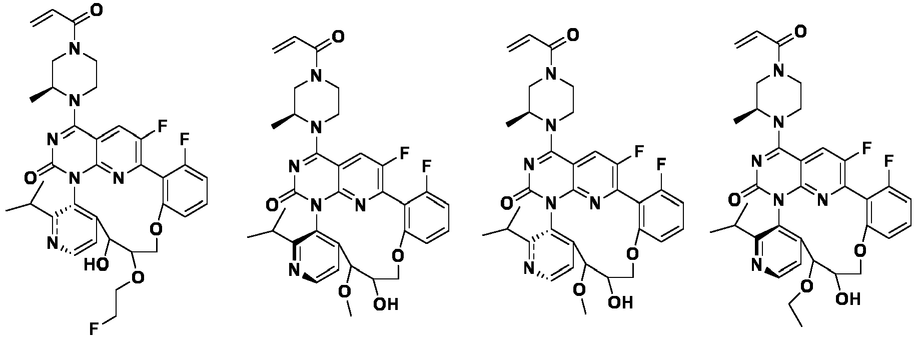

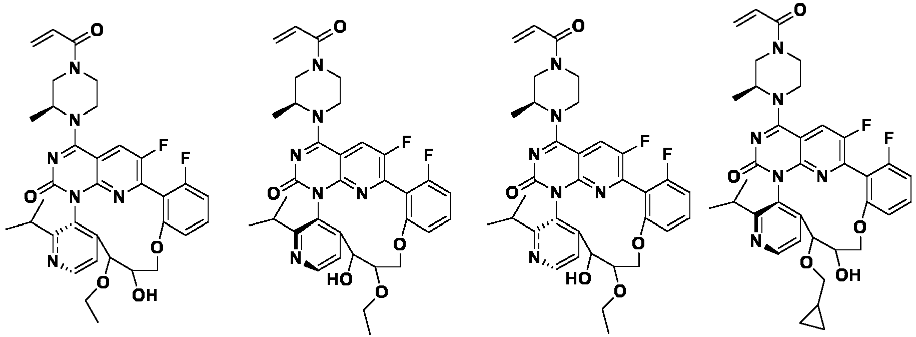

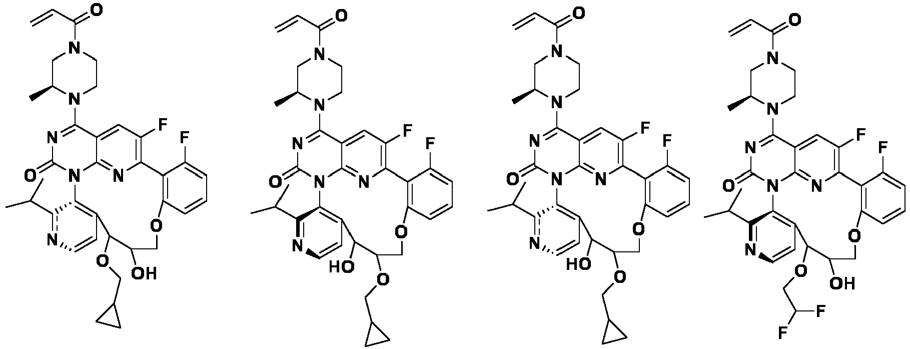

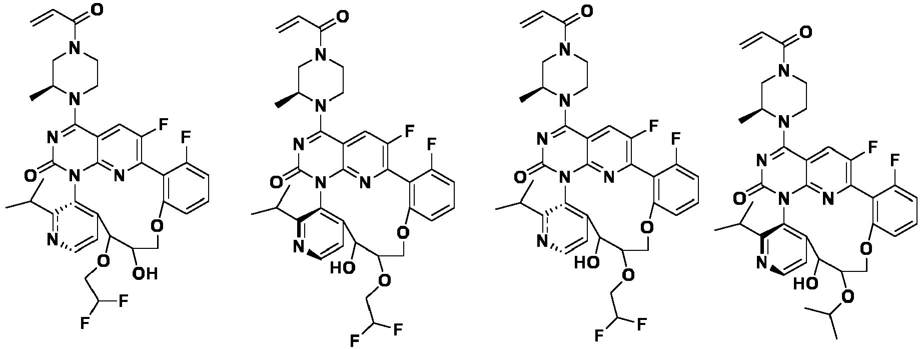

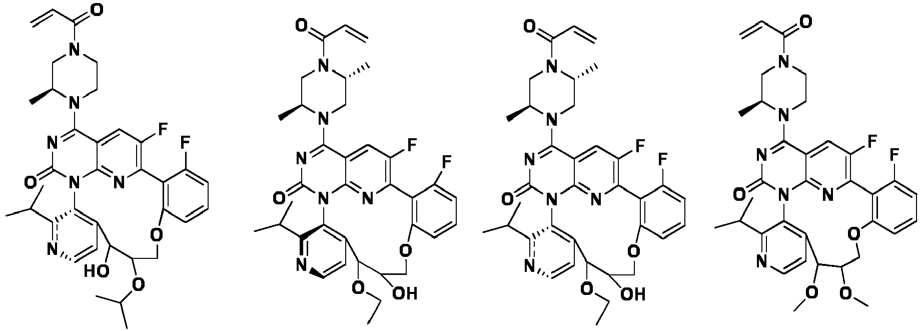

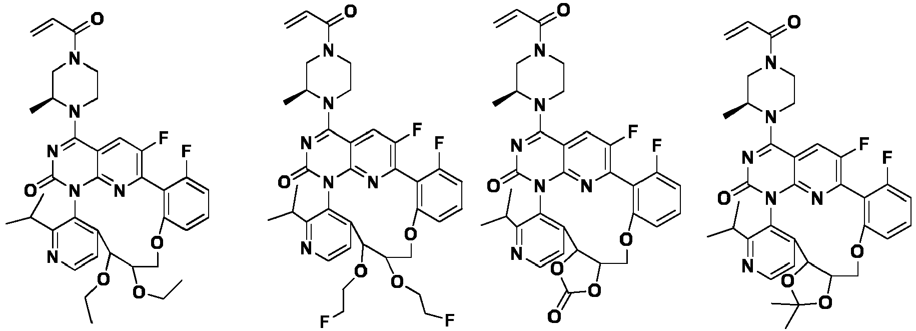

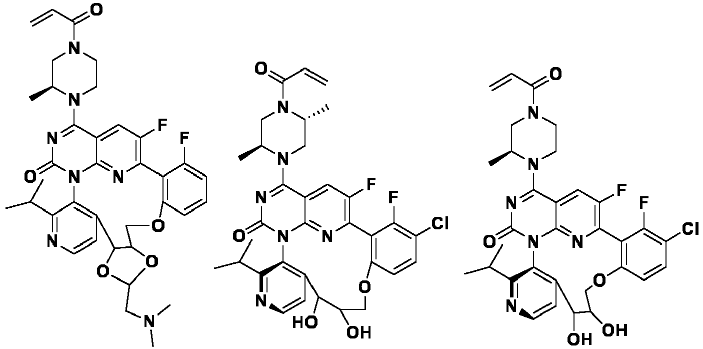

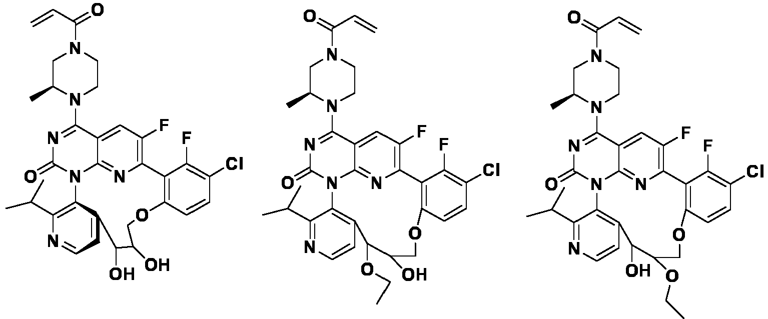

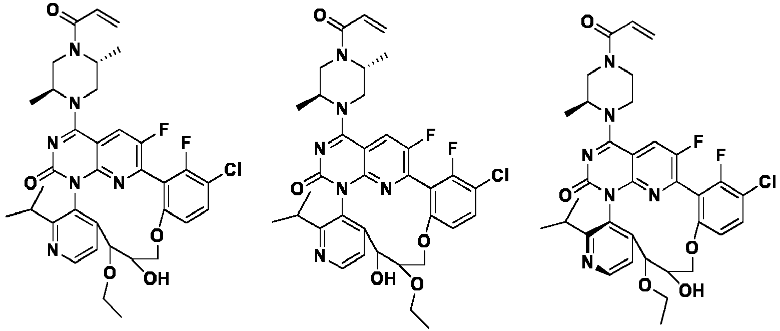

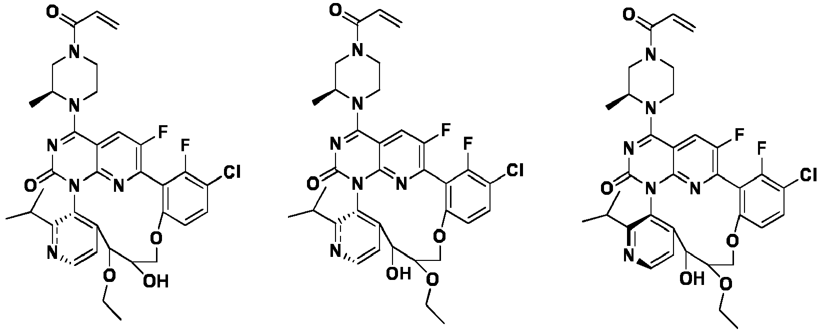

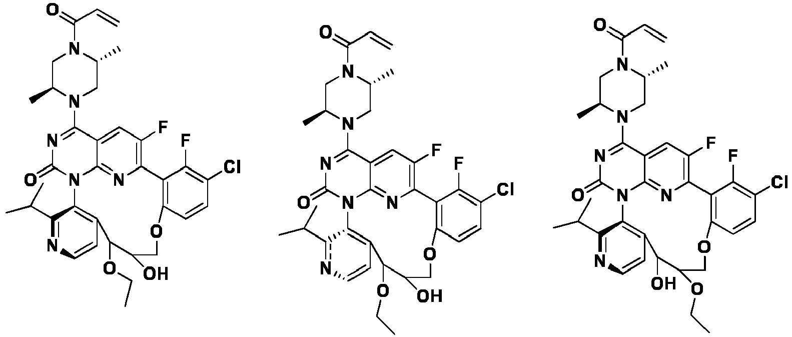

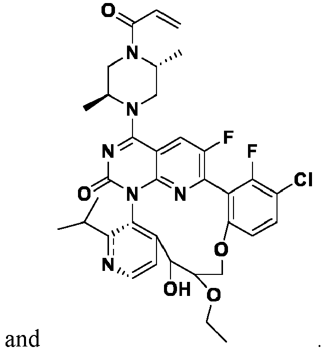

16. A method for preparing the compound of formula (I), the stereoisomer or pharmaceutically acceptable salt thereof of any one of claims 1-15, comprising the following steps: 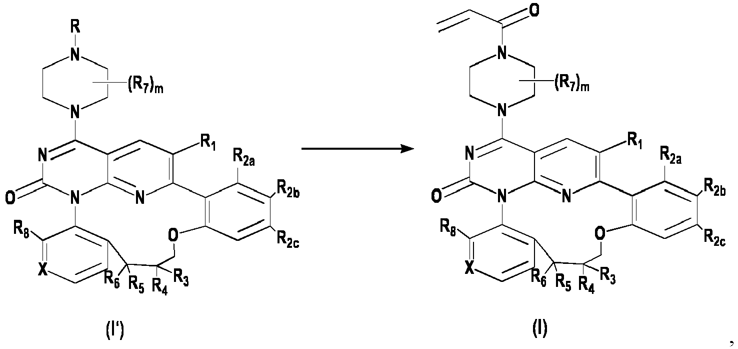 wherein, R is H or an amino-protecting group, preferably, the amino-protecting group is tert-butyloxycarbonyl; R₁, R₂ₐ, R_{2b}, R_{2c}, R₃, R₄, R₅, R₆, R₇, R₈, X and m are defined as in claim 1.

17. A pharmaceutical composition comprising the compound of formula (I), the stereoisomer or pharmaceutically acceptable salt thereof of any one of claims 1-15, and a pharmaceutically acceptable carrier.

18. Use of the compound of formula (I), the stereoisomer or pharmaceutically acceptable salt thereof of any one of claims 1-15 in the preparation of a medicament for treating tumor or cancer at least partially mediated by K-RAS G12C mutation.

19. The use of claim 18, wherein, the cancer or tumor is selected from the group consisting of a malignant tumor or cancer of the lung, a malignant tumor or cancer of the liver and gallbladder, a malignant tumor or cancer of the gastrointestinal tract, a malignant tumor or cancer of the blood system, a sarcoma, a malignant tumor or cancer of the skin, a malignant tumor or cancer of the bone, a malignant tumor or cancer of the genitourinary tract, a malignant tumor or cancer of the nervous system, a gynecological malignant tumor or cancer, and a malignant tumor or cancer of the adrenal gland.

20. The use of claim 19, wherein, **the malignant tumor or cancer of the lung is selected from** the group consisting of bronchial carcinoma (squamous cell carcinoma, undifferentiated small cell, undifferentiated large cell or adenocarcinoma), non-small cell lung cancer, bronchial adenoma, sarcoma, lymphoma, cartilagenous hamartoma, and mesothelioma;
**the malignant tumor or cancer of the liver and gallbladder is selected from** the group consisting of liver cancer, hepatoblastoma, hemangiosarcoma, hepatocellular adenoma, hemangioma, gallbladder cancer, ampullary cancer and biliary duct cancer;
**the malignant tumor or cancer of the gastrointestinal tract is selected from** the group consisting of a malignant tumor or cancer of the esophagus (squamous cell carcinoma, adenocarcinoma, leiomyosarcoma or lymphoma), a malignant tumor or cancer of the stomach (cancer, lymphoma or leiomyosarcoma), a malignant tumor or cancer of the pancreas (ductal adenocarcinoma, insulinoma, glucagonoma, gastrinoma, carcinoid tumor, uveal tumor), a malignant tumor or cancer of the small intestine (adenocarcinoma, lymphoma, carcinoid tumor, Kaposi's sarcoma, leiomyoma, hemangioma, lipoma, neurofibroma, or fibroma), a malignant tumor or cancer of the large intestine (adenocarcinoma, adenoma, or tubular adenoma) and leiomyoma;
**the malignant tumor or cancer of the blood system is selected from** the group consisting of acute or chronic myeloid leukemia, acute lymphocytic leukemia, chronic lymphocytic leukemia, myeloproliferative disease, multiple myeloma, myelodysplastic syndrome, Hodgkin's disease and non-Hodgkin lymphoma;
**the sarcoma** is selected from the group consisting of angiosarcoma, fibrosarcoma, rhabdomyosarcoma, liposarcoma, myxoma, rhabdomyoma, fibroma, lipoma and teratoma;
**the malignant tumor or cancer of the skin is selected from** the group consisting of malignant melanoma, basal cell carcinoma, squamous cell carcinoma, Kaposi sarcoma, nevocarcinoma, hyperplastic nevus, lipoma, hemangioma, dermatofibroma, keloma, or psoriasis;
**the malignant tumor or cancer of the bone is selected from** the group consisting of osteosarcoma, fibrosarcoma, malignant fibrous histiotoma, chondrosarcoma, Ewing's sarcoma, malignant lymphoma, multiple myeloma, malignant giant cell tumor chordoma, osteochondroma, benign chondroma, chondroblastoma, cartilage mucosal fibroma, osteoid osteoma, and giant cell tumor;
**the malignant tumor or cancer of the genitourinary tract is selected from** the group consisting of a malignant tumor or cancer of the kidney (adenocarcinoma, Wilm's tumor, or nephroblastoma), lymphoma, leukemia, a malignant tumor or cancer of the bladder or urethra (squamous cell carcinoma, transitional cell carcinoma, or adenocarcinoma), a malignant tumor or cancer of the prostate gland (adenocarcinoma or sarcoma), a malignant tumor or cancer of the testicle (leukemia, teratoma, embryonal carcinoma, or teratoma), choriocarcinoma, sarcoma, mesenchymal cell cancer, fibroma, fibroadenoma, adenomatoid tumor and lipoma;
**the malignant tumor or cancer of the nervous system is selected from** the group consisting of osteoma, hemangioma, granuloma, xanthoma, scleromalacia, meningioma, meningosarcoma, glioma, astrocytoma, medulloblastoma, neuroglioma, ependymoma, genital tumor, glioblastoma multiforme, oligodendroglioma, schwannoma, retinoblastoma, congenital tumor, spinal neurofibroma, meningioma and sarcoma;
**the gynecological malignant tumor or cancer is selected from** the group consisting of endometrial cancer (serous cystadenocarcinoma, mucinous cystadenocarcinoma or unclassified cancer), granulosa-thecoma, leydig cell tumor, abnormal myometrial tumor, malignant teratoma, squamous epithelial carcinoma, Brenner's tumor, adenoepithelial carcinoma, melanoma, clear cell carcinoma, squamous cell carcinoma, botryoid sarcoma and fallopian tube carcinoma; and
**the malignant tumor or cancer of the adrenal gland is** neuroblastoma.

21. Use of the compound of formula (I), the stereoisomer or pharmaceutically acceptable salt thereof of any one of claims 1-15 as a medicament for treating tumor or cancer at least partially mediated by K-RAS G12C mutation.
